# EUROPÄISCHE PATENTSCHRIFT

(11) **EP 2 039 728 B1**
(45) Veröffentlichungstag und Bekanntmachung des Hinweises auf die Patenterteilung: **02.05.2012**
(21) Anmeldenummer: 08163756.3
(22) Anmeldetag: 05.09.2008
(51) Int. Cl.: C09B 67/22, C09B 1/54, C09B 1/467, C08J 3/22, C08K 5/00, C09B 1/00

(54) **Rote Farbstoffmischung**
Red paint mixture
Mélange de couleur rouge

(30) Priorität: 18.09.2007 DE 102007044743; 09.05.2008 DE 102008023114
(43) Veröffentlichungstag der Anmeldung: 25.03.2009
(73) Patentinhaber: LANXESS Deutschland GmbH, 51369 Leverkusen (DE)
(72) Erfinder: Michaelis, Stephan, 51519 Odenthal (DE); Berneth, Horst, 51373 Leverkusen (DE); Kuckert, Eberhard, 51375 Leverkusen (DE)

(56) Entgegenhaltungen:
- EP-A1- 0 621 320
- EP-A2- 0 546 856
- EP-A2- 0 564 237
- CN-A- 1 597 788
- CS-B5- 123 304
- DE-A1- 4 241 116
- DE-A1- 19 533 026
- DE-A1- 19 623 411
- US-A- 5 332 404
- US-A- 5 578 419

## Beschreibung

Die Erfindung betrifft Mischungen, enthaltend spezielle Rotfarbstoffe und spezielle Orangefarbstoffe, darauf basierende Masterbatches sowie ihre Verwendung zum Massefärben von Kunststoffen, die gefärbten Kunststoffe an sich sowie die Verwendung und Masterbatches spezieller Rotfarbstoffe.

In den letzten Jahren ist der Bedarf an braungefärbten Kunststoffflaschen, insbesondere PET-Flaschen stark gestiegen. Bislang verwendete man zur Einfärbung eine braune Farbstoffmischung, die als Rotfarbstoff das C.I Solvent Red 135, das Perinon der Formel 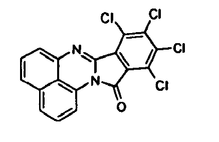 sowie eine weiteren Gelb- und Grünfarbstoff enthält. Diese Farbmischung hat einen sehr verbreiteten Braunton etabliert. Der coloristischen Vorgabe dieses Brauntons auf der einen Seite stehen gestiegene anwendungstechnische Anforderungen sowie die Notwendigkeit nach chlorfreien Ersatzstoffen auf der anderen Seite entgegen. Neben hoher Lichtechtheit, Thermostabilität und Farbstärke sowie guter Löslichkeit im Kunststoff werden auch Sublimations- und Migrierechtheiten verlangt.
Aufgabe der vorliegenden Erfindung war es daher, einen coloristischen Ersatz für die Rotkomponente der Braunmischung zu finden, die gleichzeitig die geforderten anwendungstechnischen Eigenschaften übertrifft.

Gefunden wurde nun eine Mischung, enthaltend wenigstens einen Rotfarbstoff der Formel (I) oder (C) 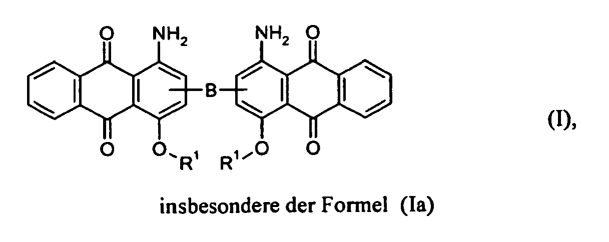 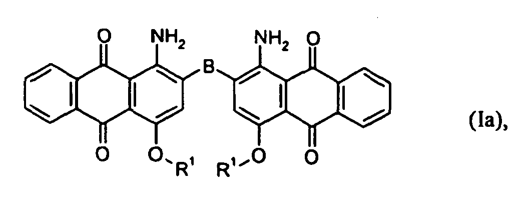 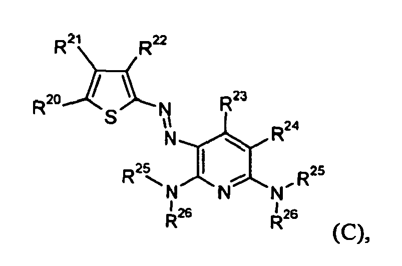 worin
- R¹: für Wasserstoff, gegebenenfalls substituiertes C₁-C₈-Alkyl oder gegebenenfalls substituiertes C₃-C₈-Cycloalkyl steht,
- B: für eine Brücke der Formeln -O-B¹-O- oder -CH₂-B²-CH₂- steht,
- B¹: für eine Brücke der Formel 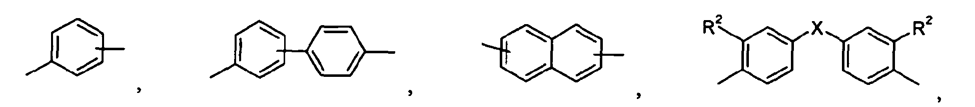 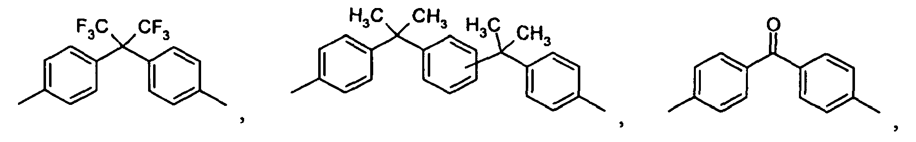 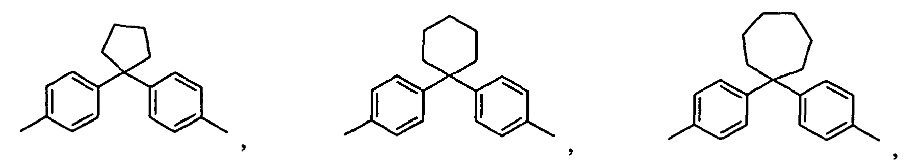 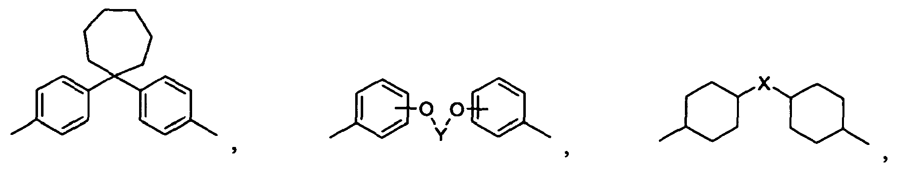 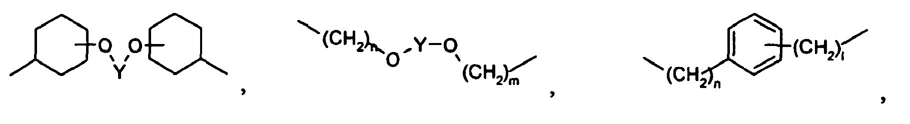 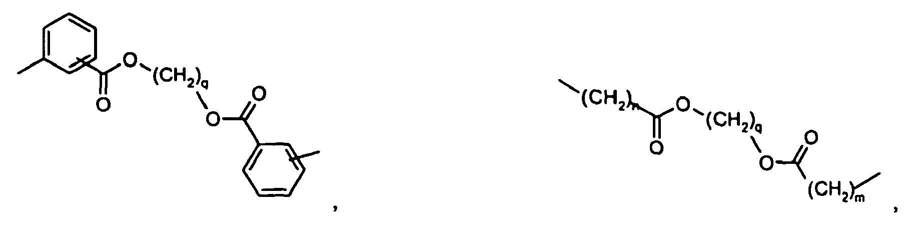 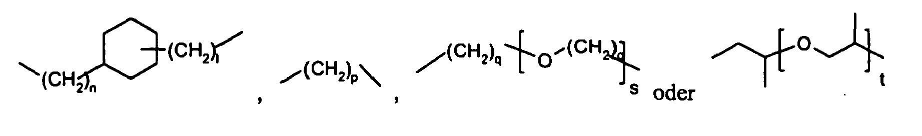
- B²: für eine Brücke der Formel 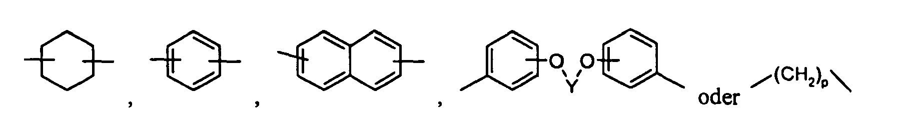 steht,
- R²: für Wasserstoff, Methyl, Ethyl, Methoxy oder Fluor steht,
- X: für eine Brücke der Formel 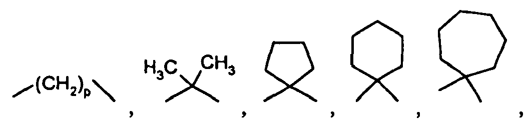 O, S, SO₂ oder CO
steht,
- Y: für eine Brücke der Formel 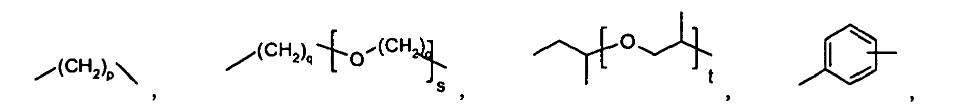 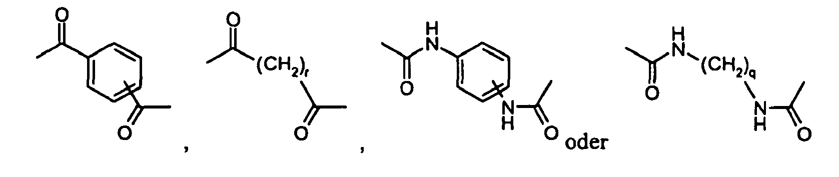 steht,
- l: für eine ganze Zahl von 0 bis 8 steht,
- m, n, p und r: unabhängig voneinander für eine ganze Zahl von 1 bis 8 stehen,
- q: für eine ganze Zahl von 2 bis 8 steht und
- s und t: unabhängig voneinander für eine ganze Zahl von 1 bis 4 stehen, und
- R²⁰ und R²²: unabhängig voneinander für Cyano oder gegebenenfalls substituiertes C₁-C₈- Alkoxycarbonyl stehen,
- R²¹: für Wasserstoff oder gegebenenfalls substituiertes C₁-C₃-Alkyl steht,
- R²³: für Wasserstoff, gegebenenfalls substituiertes C₁-C₈-Alkyl oder gegebenenfalls substituiertes C₁-C₈-Alkoxycarbonyl steht,
- R²⁴: für Cyano, gegebenenfalls substituiertes C₁-C₈-Alkylsulfonyl oder gegebenenfalls substituiertes C₁-C₈-Alkoxycarbonyl steht,
- R²⁵: für gegebenenfalls substituiertes C₁-C₈-Alkyl, gegebenenfalls substituiertes C₃-C₇- Cycloalkyl, gegebenenfalls substituiertes C₆-C₁₀-Aryl steht,
- R²⁶: für Wasserstoff oder unabhängig von R²⁵ die Bedeutung von R²⁵ hat oder
- NR²⁵R²⁶: für Pyrrolidino, Piperidino oder Morpholino steht,
und wenigstens einen Orangefarbstoff der Formel
(L), (LI), (LII), (LIII), (LIV) oder (LV) 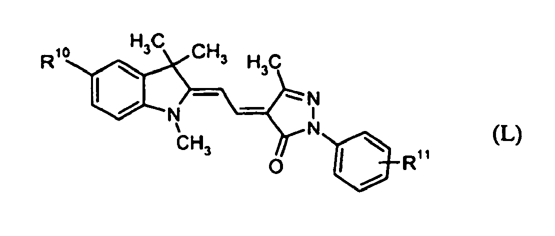 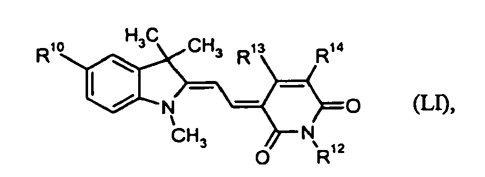 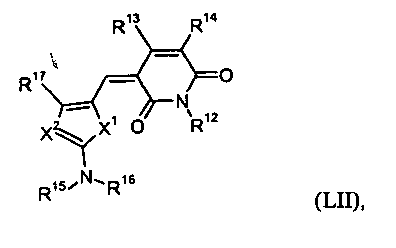 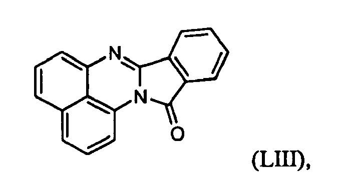 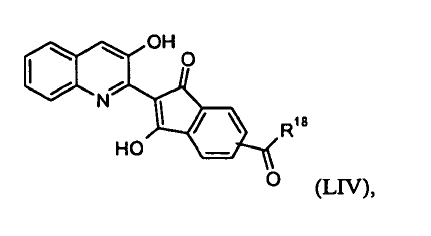 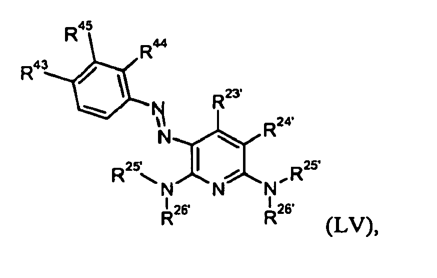 worin
- R¹⁰: für Wasserstoff, gegebenenfalls substituiertes C₁-C₈-Alkyl, gegebenenfalls substituiertes C₁-C₈-Alkoxy, gegebenenfalls substituiertes C₁-C₈-Alkylsulfonyl, gegebenenfalls substituiertes C₁-C₈-Alkoxycarbonyl, Nitro, Cyano oder Fluor steht,
- R¹¹: für Wasserstoff, gegebenenfalls substituiertes C₁-C₈-Alkyl, gegebenenfalls substituiertes C₁-C₈-Alkoxy, gegebenenfalls substituiertes C₁-C₈-Alkoxycarbonyl, Nitro, Cyano oder Fluor steht,
- R¹²: für Wasserstoff, gegebenenfalls substituiertes C₁-C₈-Alkyl oder gegebenenfalls substituiertes C₃-C₈-Cycloalkyl steht,
- R¹³: für Wasserstoff, gegebenenfalls substituiertes C₁-C₈-Alkyl oder gegebenenfalls substituiertes C₁-C₈-Alkoxycarbonyl steht,
- R¹⁴: für Cyano, gegebenenfalls substituiertes C₁-C₈-Alkylsulfonyl oder gegebenenfalls substituiertes C₁-C₈-Alkoxycarbonyl steht,
- X¹: für O oder S steht,
- X²: für N oder C-CN steht,
- R¹⁵ und R¹⁶: unabhängig voneinander für gegebenenfalls substituiertes C₁-C₈- Alkyl, gegebenenfalls substituiertes C₃-C₈-Cycloalkyl oder gegebenenfalls substituiertes C₆-C₁₀-Aryl stehen oder
- NR¹⁵R¹⁶: für Pyrrolidino, Piperidino oder Morpholino steht,
- R¹⁷: für Wasserstoff oder C₁-C₈-Alkyl steht,
- R¹⁸: für gegebenenfalls substituiertes C₆-C₁₀-Aryl steht,
- R^{23'}: für Wasserstoff, gegebenenfalls substituiertes C₁-C₈-Alkyl oder gegebenenfalls substituiertes C₁-C₈-Alkoxycarbonyl steht,
- R^{24'}: für Cyano, gegebenenfalls substituiertes C₁-C₈-Alkylsulfonyl oder gegebenenfalls substituiertes C₁-C₈-Alkoxycarbonyl steht,
- R^{25'}: für gegebenenfalls substituiertes C₁-C₈-Alkyl, gegebenenfalls substituiertes C₃-C₇- Cycloalkyl, gegebenenfalls substituiertes C₆-C₁₀-Aryl steht,
- R^{26'}: für Wasserstoff oder unabhängig von R^{25'} die Bedeutung von R^{25'} hat oder
- NR^{25'}R^{26'}: für Pyrrolidino, Piperidino oder Morpholino steht,
- R⁴³ und R⁴⁴: unabhängig voneinander für Wasserstoff, gegebenenfalls substituiertes C₁-C₄- Alkyl, insbesondere durch ein oder mehrere Fluor substituiertes C₁-C₄-Alkyl, Nitro, Cyano, C₁-C₄-Alkoxycarbonyl stehen und
- R⁴⁵: für Wasserstoff, Methyl, Cyano, Fluor oder Chlor steht.

Bevorzugt entspricht die Verbindung der Formel I den Formeln (Ia) bis (Ic) 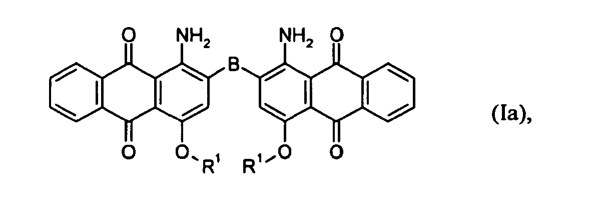 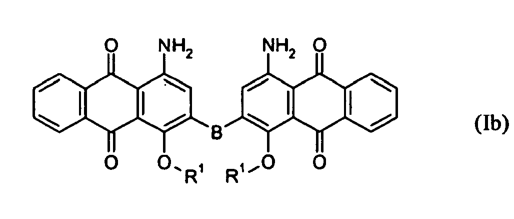 und 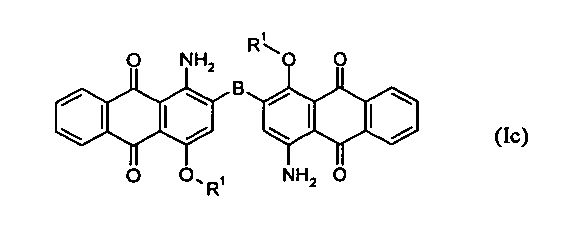 insbesondere der Formel Ia.

Im Falle der Naphthalinstruktur greifen die unbestimmten Bindungen vorzugsweise in den Positionen 1,5-, 1,8-, 2,6- oder 2,7- an. Im Falle der Benzol- und Cyclohexanstrukturen greifen die unbestimmten Bindungen in den Positionen 1,2-, 1,3- oder 1,4- an.

Unter C₁-C₈-Alkyl wird hier und im Folgenden geradkettiges bzw. verzweigtes C₁-C₈-Alkyl, z. B. Methyl, Ethyl, n-Propyl, 2-Propyl, n-Butyl, 2-Butyl, iso-Butyl, tert.-Butyl, Pentyl, Hexyl, Heptyl, Octyl oder 2-Ethylhexyl verstanden.

Unter substituiertem C₁-C₈-Alkyl wird geradkettiges bzw. verzweigtes C₁-C₈-Alkyl, wie oben beschrieben verstanden, das durch mindestens einen der Reste Fluor, Cyano, Hydroxy, C₁-C₄-Alkoxy, Acetoxy, Propoxy, Butoxy, C₁-C₄-Alkoxycarbonyl, Cyclopentyl, Cyclohexyl, Phenyl, Naphthyl substituiert ist, z. B. Trifluormethyl, Perfluorbutyl, 2-Chlorethyl, Cyanmethyl, 2-Cyanethyl, 2-Methoxyethyl, 2-Ethoxyethyl, 2-Acetoxyethyl, 2-Methoxycarbonylethyl, Cyclohexylmethyl, Benzyl, Phenethyl oder Phenylpropyl.

Hier und im Folgenden ist beispielsweise mit Butyl stets n-Butyl, 2-Butyl, iso-Butyl oder tert.-Butyl gemeint, wenn nicht ausdrücklich etwas anderes gesagt wird.

Unter C₃-C₈-Cycloalkyl wird hier und im Folgenden Cyclopropyl, Cyclobutyl, Cyclopentyl, Cyclohexyl, Cycloheptyl oder Cyclooctyl verstanden. Unter substituiertem C₃-C₈-Cycloalkyl wird beispielsweise Methylcyclohexyl verstanden.
Vorzugsweise steht R¹ für Wasserstoff. Ebenfalls vorzugsweise steht R¹ für Methyl.

Bevorzugte Verbindungen der Formel I sind solche, worin
- B¹: für eine Brücke der Formel 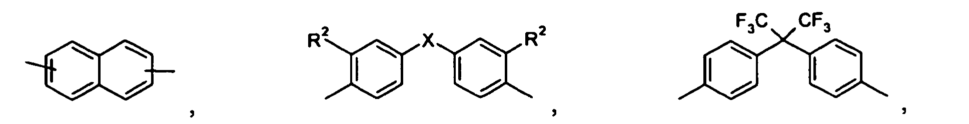 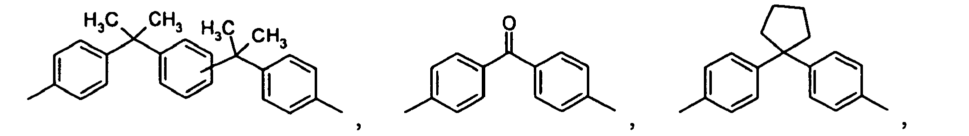 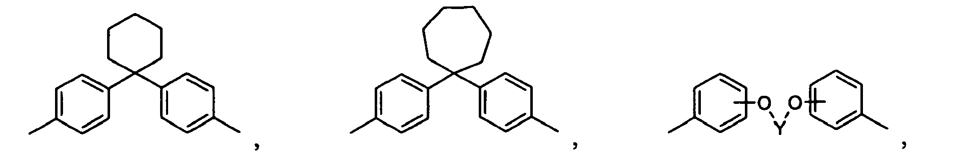 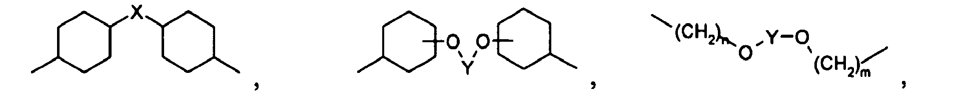 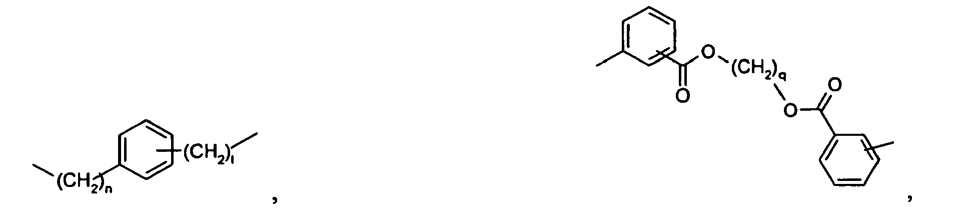 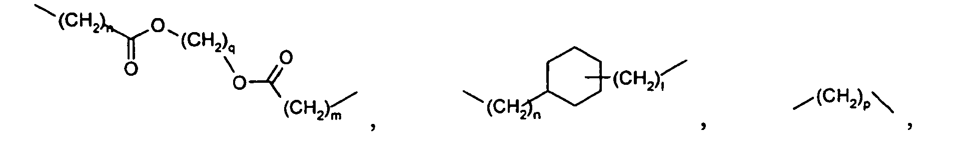 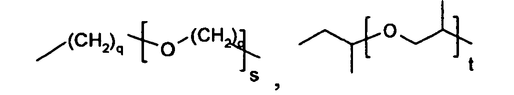 steht,
- R²: für Methyl, Ethyl, Methoxy oder Fluor steht,
- X: für eine Brücke der Formeln 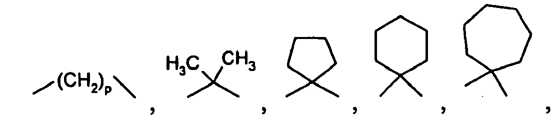 O, S, SO₂, CO
steht,
- Y: für eine Brücke der Formeln 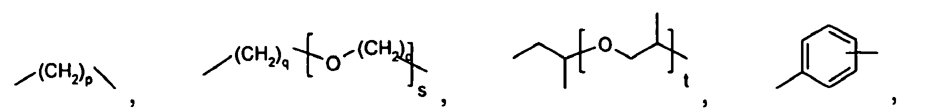 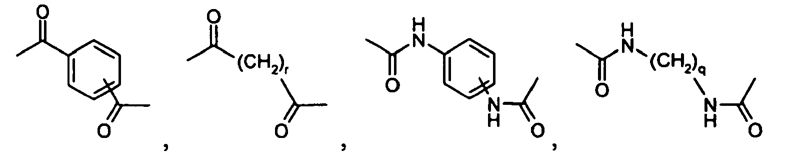 steht,
- l: für eine ganze Zahl von 0 bis 8 steht,
- m, n, p und r: unabhängig voneinander für eine ganze Zahl von 1 bis 8 stehen,
- q: für eine ganze Zahl von 2 bis 8 steht und
- s und t: unabhängig voneinander für eine ganze Zahl von 1 bis 4 stehen.

Besonders bevorzugte Brücken der Formel B¹ sind solche 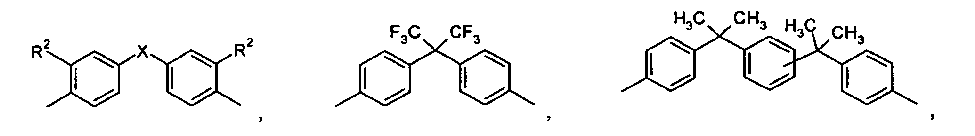 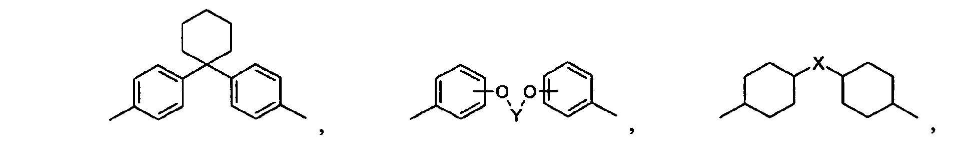 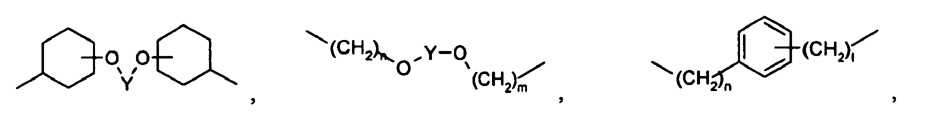 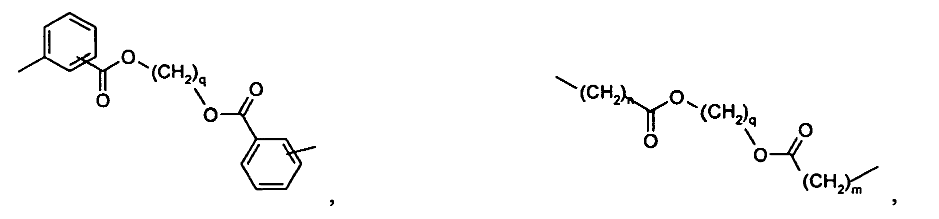 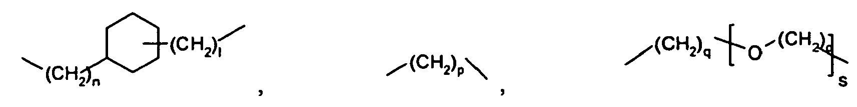 oder 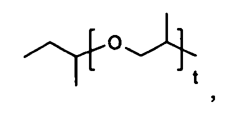 worin
- R²: für Methyl steht,
- X: für eine Brücke der Formel 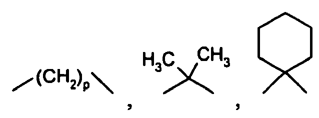 oder O
steht,
- Y: für eine Brücke der Formel 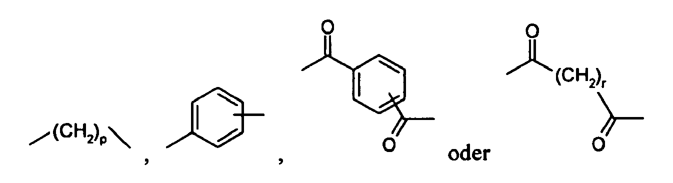 steht,
- l: für eine ganze Zahl von 0 bis 4 steht,
- m, n und p: unabhängig voneinander für eine ganze Zahl von 1 bis 4 stehen,
- q und r: unabhängig voneinander für eine ganze Zahl von 2 bis 6 steht,
- s und t: unabhängig voneinander für eine ganze Zahl von 1 bis 2 stehen und
die unbestimmten Ringpositionen 1,2-, 1,3- oder 1,4- bedeuten.

Ganz besonders bevorzugte Brücken B¹ sind: 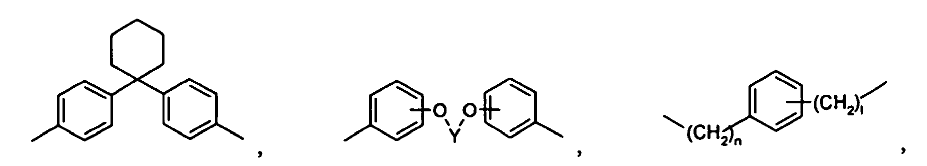 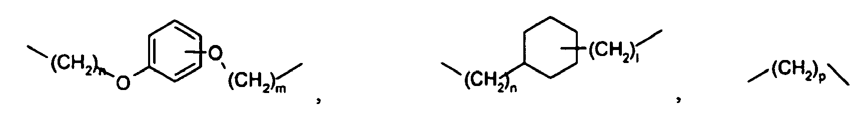 oder 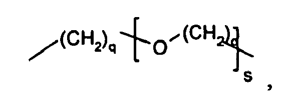 worin
- Y: für eine Brücke der Formel 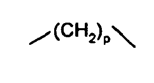 steht,
- l: für eine ganze Zahl von 1 bis 2 steht,
- m, n und p: unabhängig voneinander für eine ganze Zahl von 1 bis 2 stehen,
- q: für eine ganze Zahl von 2 bis 4 steht,
- s und t: unabhängig voneinander für 1 stehen und
die unbestimmten Ringpositionen 1,4- bedeuten.

Bevorzugte Brücken B² sind: 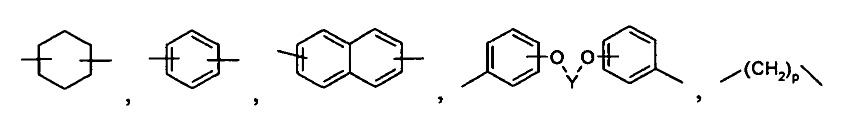 worin
- Y: für eine Brücke der Formeln 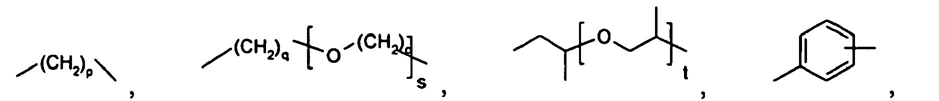 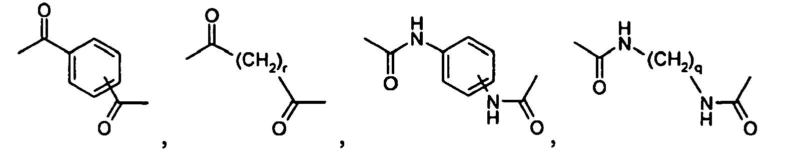 steht,
- p und r: unabhängig voneinander für eine ganze Zahl von 1 bis 8 stehen,
- q: für eine ganze Zahl von 2 bis 8 steht und
- s und t: unabhängig voneinander für eine ganze Zahl von 1 bis 4 stehen.

Besonders bevorzugte Brücken B² sind 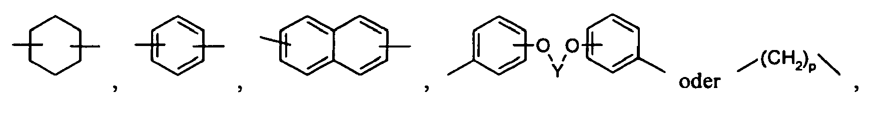 worin
- Y: für eine Brücke der Formel 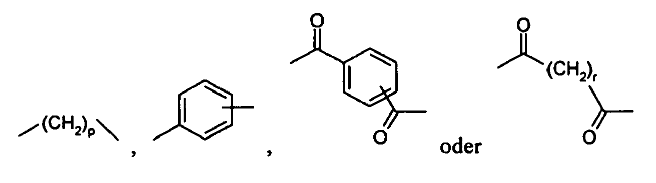 steht,
- p: für eine ganze Zahl von 1 bis 6 steht und
- r: für eine ganze Zahl von 2 bis 6 steht
und die unbestimmten Ringpositionen bei Benzol- oder Cyclohexanringen 1,2-, 1,3- oder 1,4-und beim Napththalinring 2,6- oder 2,7- bedeuten.

Ganz besonders bevorzugte Brücken B² sind: 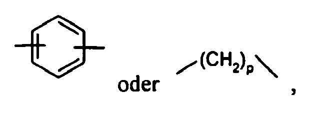 worin
- p: für eine ganze Zahl von 1 bis 4 steht
und die unbestimmten Ringpositionen 1,4- bedeuten.

Bevorzugte Rotfarbstoffe der Formel (C) sind solche, worin
- R²⁰: für Cyano, Methoxycarbonyl oder Ethoxycarbonyl, besonders bevorzugt für Cyano oder Methoxycarbonyl, ganz besonders bevorzugt für Methoxycarbonyl,
- R²²: für Cyano,
- R²¹: für Wasserstoff oder Methyl, besonders bevorzugt für Methyl,
- R²³: für Methyl, Trifluormethyl oder Methoxycarbonyl, besonders bevorzugt für Methyl,
- R²⁴: für Cyano, Methansulfonyl, Methoxycarbonyl oder Ethoxycarbonyl, besonders bevorzugt für Cyano,
- R²⁵: für Methyl, Ethyl, Propyl, Butyl, Methoxyethyl, Methoxypropyl, Cyanethyl, Benzyl, Cyclohexyl, Phenyl oder Tolyl, besonders bevorzugt für Ethyl, Propyl, Butyl, Methoxyethyl, Methoxypropyl, Benzyl, Cyclohexyl oder Phenyl, ganz besonders bevorzugt für Propyl, Butyl oder Methoxypropyl,
- R²⁶: für Wasserstoff oder unabhängig von R²⁵ die Bedeutung von R²⁵, oder
- NR²⁵R²⁶: für Pyrrolidino, Piperidino oder Morpholino.

Die Farbstoffe der Formel (I) und wenigstens ein Orangefarbstoff der Formel (L) bis (LV) werden vorzugsweise im Verhältnis von 1:99 bis 99:1, vorzugsweise von 20:80 bis 97:3, besonders bevorzugt von 40:60 bis 95:5, ganz besonders bevorzugt 50:50 bis 90:10 gemischt.

Die Rotfarbstoffe der Formel (C) und wenigstens ein Orangefarbstoff der Formel (L) bis (LV) werden bevorzugt im Verhältnis zu 1:99 bis 99:1, vorzugsweise 20:80 bis 97:3, besonders bevorzugt 30:70 bis 95:5, ganz besonders bevorzugt von 40:60 bis 90:10 gemischt. Ganz besonders bevorzugt ist eine Mischung der Farbstoffe der Formeln (C) und (L) im Verhältnis 40:60 bis 60:40.

Die Mischung kann beispielsweise erfolgen durch Vermischen der Farbstoffe in fester Form beispielsweise als Pulver, Granulate oder Pasten. Im Falle beispielsweise feuchter Pasten kann anschließend getrocknet werden. Sie kann auch erfolgen durch Vermischen von Suspensionen der Farbstoffe, die beispielsweise anschließend sprühgetrocknet oder sprühgranuliert werden. Die Mischung kann schließlich auch erfolgen bei der Herstellung von Masterbatches oder bei der Färbung von polymeren Materialien.

Geeignete Farborte für erfindungsgemäße rote Mischungen sind nach den Lab-Werten L von 35-60, a von 65-85 und b von 20-75, bevorzugt L von 40-50, a von 65-75 und b von 40-75.

Für die Orangefarbstoffe (L) bis (LV) stehen bevorzugt
- R¹⁰: für Wasserstoff, Methyl, Ethyl, Methoxy, Methansulfonyl, Methoxycarbonyl, Ethoxycarbonyl, Nitro, Cyano oder Fluor, besonders bevorzugt für Wasserstoff, Methansulfonyl oder Methoxycarbonyl, ganz besonders bevorzugt für Methoxycarbonyl,
- R¹¹: für Wasserstoff, Methyl, Methoxy, Methoxycarbonyl, Nitro, Cyano oder Fluor, besonders bevorzugt für Wasserstoff,
- R¹²: für Methyl, Ethyl, Propyl, Butyl, 2-Methoxyethyl, 2-Cyanoethyl, Benzyl oder Cyclohexyl, besonders bevorzugt für Methyl, Ethyl, Propyl, Butyl oder Benzyl, ganz besonders bevorzugt für Methyl oder Ethyl,
- R¹³: für Methyl oder Methoxycarbonyl, besonders bevorzugt für Methyl,
- R¹⁴: für Cyano, Methansulfonyl, Methoxycarbonyl oder Ethoxycarbonyl, besonders bevorzugt für Cyano,
- X¹: für O oder S, besonders bevorzugt für S,
- X²: für N oder C-CN steht,
- R¹⁵ und R¹⁶: unabhängig voneinander für Methyl, Ethyl, Propyl, Butyl, Cyanoethyl, Benzyl, Cyclopentyl, Cyclohexyl, Phenyl oder Tolyl, besonders bevorzugt für Methyl, Ethyl, Cyanoethyl, Benzyl, Cyclohexyl oder Phenyl, ganz besonders bevorzugt für Methyl oder Ethyl, oder
- NR¹⁵R¹⁶: für Pyrrolidino, Piperidino oder Morpholino,
- R¹⁷: für Wasserstoff oder Methyl, besonders bevorzugt für Wasserstoff,
- R¹⁸: für Phenyl, Tolyl, Dimethylphenyl, Trimethylphenyl, besonders bevorzugt für Tolyl oder Dimethylphenyl,
- R^{23'}: für Methyl, Trifluormethyl oder Methoxycarbonyl,
- R^{24'}: für Cyano, Methylsulfonyl, Trifluormethylsulfonyl, Methoxy- oder Ethoxycarbonyl,
- R^{25'}: für Methyl, Ethyl, Propyl, Butyl, Chlorethyl, Methoxyethyl, Methoxypropyl, Dimethylaminopropyl, Phenyl, Tolyl, Methoxyphenyl, Fluorphenyl, Chlorphenyl oder Cyanophenyl,
- R^{26'}: für Wasserstoff oder NR^{25'}R^{26'} für Pyrrolidino, Piperidino oder Morpholino,
- R⁴³ und R⁴⁴: unabhängig voneinander für Trifluormethyl, Nitro, Cyano, Methoxy- oder Ethoxycarbonyl, wobei einer von beiden zusätzlich für Wasserstoff stehen kann,
- R⁴⁵: für Wasserstoff.

Eine bevorzugte erfindungsgemäße Mischung enthält einen Rotfarbstoff der Formel (I) und wenigstens einen Orangefarbstoff der Formel (L), (LI), (LII), (LIII), (LIV) oder (LV), insbesondere der Formel (L).

Eine ebenfalls bevorzugte erfindungsgemäße Mischung enthält einen Rotfarbstoff der Formel (C) und wenigstens einen Orangefarbstoff der Formel (L), (LI), (LII), (LIII), (LIV) oder (LV), insbesondere der Formel (L).
Besonders bevorzugt ist eine erfindungsgemäße Mischung enthaltend einen Rotfarbstoff der Formel (C), worin
- R²⁰: für Cyano, Methoxycarbonyl oder Ethoxycarbonyl, besonders bevorzugt für Cyano oder Methoxycarbonyl, ganz besonders bevorzugt für Methoxycarbonyl,
- R²²: für Cyano,
- R²¹: für Wasserstoff oder Methyl, besonders bevorzugt für Methyl,
- R²³: für Methyl oder Methoxycarbonyl, besonders bevorzugt für Methyl,
- R²⁴: für Cyano, Methansulfonyl, Methoxycarbonyl oder Ethoxycarbonyl, besonders bevorzugt für Cyano,
- R²⁵: für Methyl, Ethyl, Propyl, Butyl, Methoxyethyl, Methoxypropyl, Cyanethyl, Benzyl, Cyclohexyl, Phenyl oder Tolyl, besonders bevorzugt für Ethyl, Propyl, Butyl, Methoxyethyl, Methoxypropyl, Benzyl, Cyclohexyl oder Phenyl, ganz besonders bevorzugt für Propyl, Butyl oder Methoxypropyl,
- R²⁶: für Wasserstoff oder unabhängig von R²⁵ die Bedeutung von R²⁵, oder
- NR²⁵R²⁶: für Pyrrolidino, Piperidino oder Morpholino,
steht, insbesondere wenn
- R²⁰: für COOCH₃, R²¹ und R²³ für CH₃, R²² und R²⁴ für CN, R²⁵ für -CH₂CH₂CH₂OCH₃ und R²⁶ für H steht
und einen Orangefarbstoff der Formel (L), insbesondere worin
- R¹⁰: für COOCH₃ und R¹¹ für H steht.

Beispiele für solche Mischungen sind: 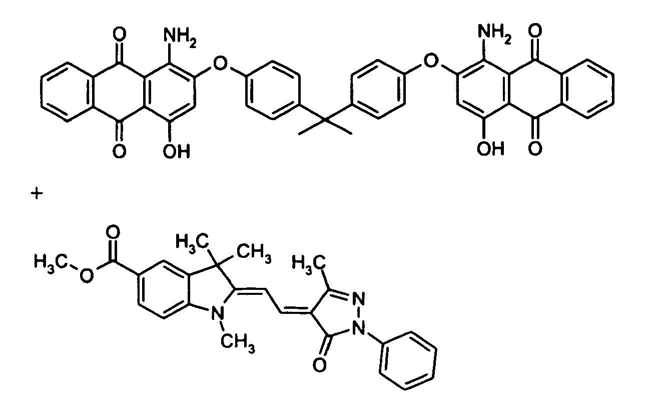 vorzugsweise im Verhältnis 50:50, 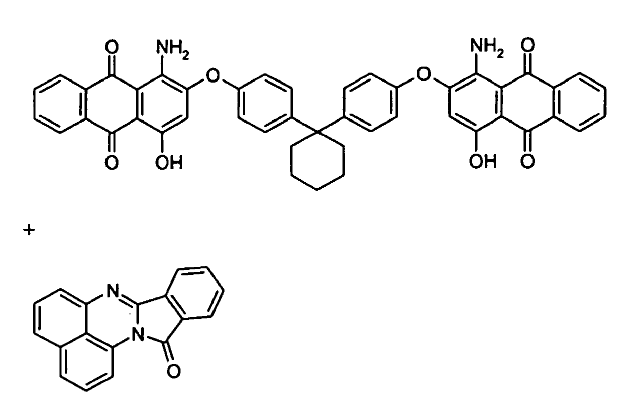 vorzugsweise im Verhältnis 45:55, 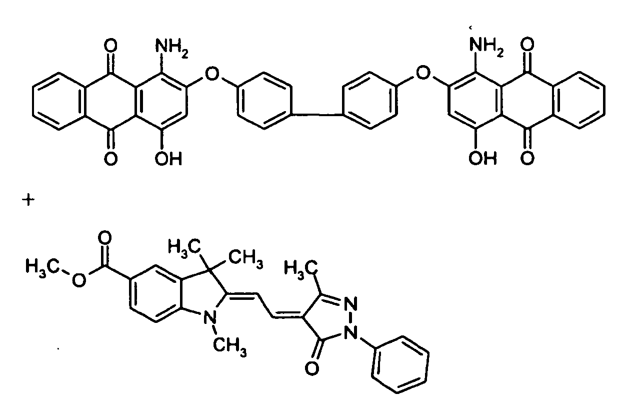 vorzugsweise im Verhältnis 83:17, 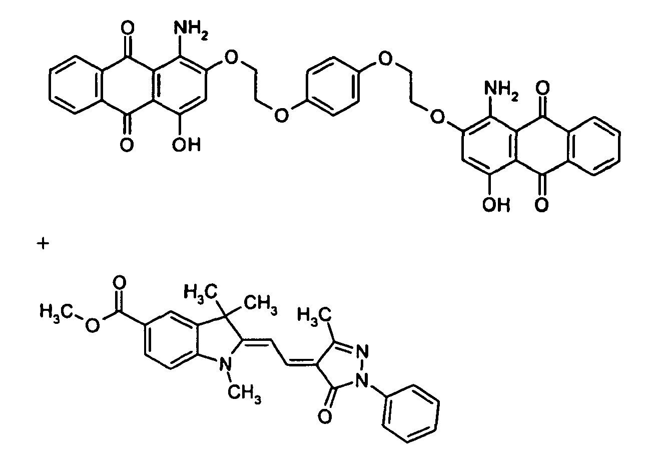 vorzugsweise im Verhältnis 83:17, 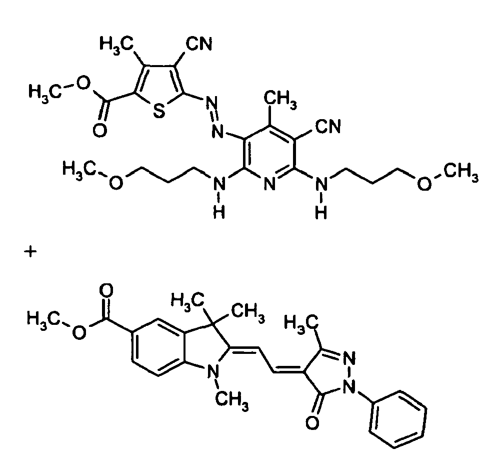 vorzugsweise im Verhältnis 50,8:48,2, 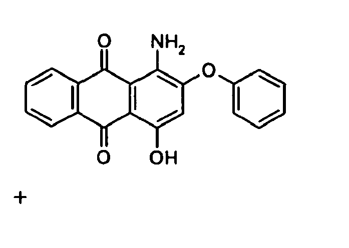 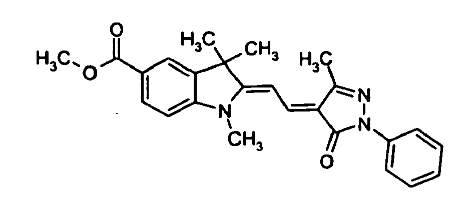 vorzugsweise im Verhältnis 48:52, 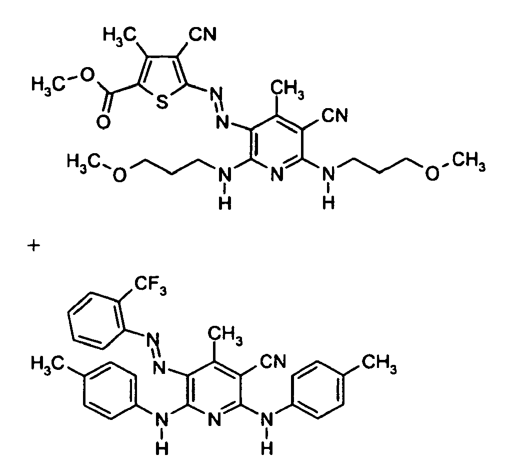 vorzugsweise im Verhältnis 50:50.

Die erfindungsgemäßen Mischungen können, sofern sie als farbgebende Komponenten lediglich Rot-und Orangefarbstoffe enthalten, zum Massefärben von Kunststoffen noch mit weiteren Farbstoffen, insbesondere solchen zur Erzielung eines braunen Farbtons eingesetzt werden, die weiteren Farbstoffen können aber auch in der Mischung bereits enthalten sein.
Die erfindungsgemäßen Mischungen enthalten daher vorzugsweise
a) wenigstens einen Gelbfarbstoff, vorzugsweise einen mit einem λₘₐₓ-Wert von 420 bis 460 nm und wenigstens einen Grünfarbstoff, vorzugsweise einen mit einem maximalen Wert von 610 bis 700 nm
   oder
b) wenigstens einen Gelbfarbstoff, vorzugsweise einen mit einem λₘₐₓ-Wert von 420 bis 460 nm und wenigstens einen Blaufarbstoff, vorzugsweise einen mit einem λₘₐₓ-Wert von 570 bis 640 nm.

Als besonders bevorzugte Gelbfarbstoffe eignen sich beispielsweise solche der Formeln 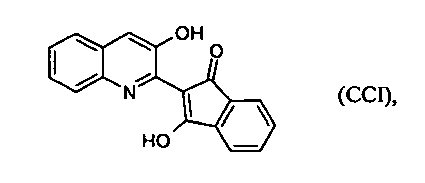 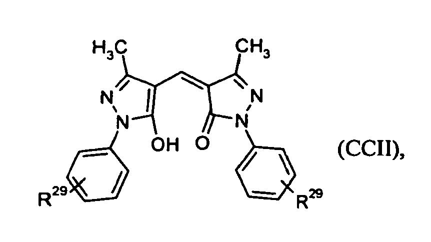 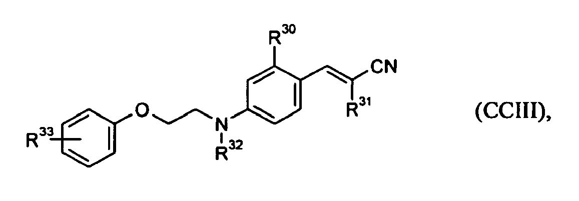 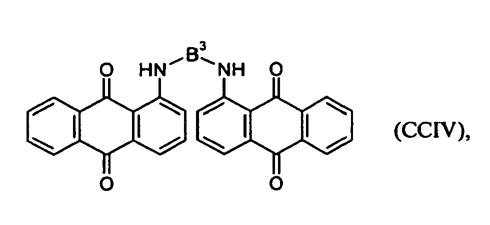 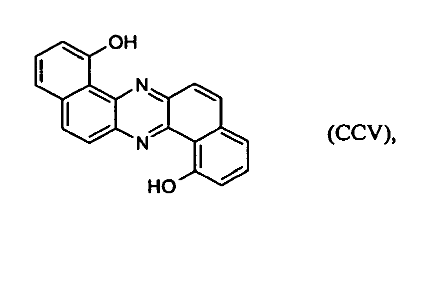 worin
- R²⁹: für einen oder mehrere Reste mit der Bedeutung Wasserstoff, gegebenenfalls substituiertes C₁- C₈-Alkyl, gegebenenfalls substituiertes C₁-C₈-Alkoxy, Fluor, Cyano, gegebenenfalls substituiertes C₃-C₇-Cycloalkyl oder gegebenenfalls substituiertes C₆-C₁₀-Aryl steht,
- R³⁰: für Wasserstoff, Methyl, Ethyl oder Methoxy steht,
- R³¹: für Cyano, C₁-C₈-Alkoxycarbonyl oder C₁-C₈-Alkansulfonyl steht,
- R³²: für Wasserstoff, gegebenenfalls substituiertes C₁-C₈-Alkyl oder gegebenenfalls substituiertes C₃-C₇-Cycloalkyl steht,
- R³³: für Wasserstoff, gegebenenfalls substituiertes C₁-C₈-Alkyl, gegebenenfalls substituiertes C₁- C₈-Alkoxy, gegebenenfalls substituiertes C₃-C₇-Cycloalkyl oder gegebenenfalls substituiertes C₆-C₁₀-Aryl steht,
- B³: für eine Brücke der Formel 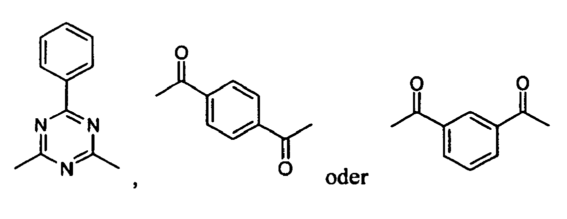
steht.

Bevorzugt stehen
- R²⁹: für Wasserstoff, Methyl, Ethyl, Propyl, Butyl, Benzyl, Cyano, Cyclohexyl, Phenyl oder Tolyl, besonders bevorzugt für p-ständiges Methyl, Ethyl, Propyl, Butyl, Cyclohexyl oder Phenyl, ganz besonders bevorzugt für p-ständiges Butyl oder Cyclohexyl,
- R³⁰: für Wasserstoff oder Methyl,
- R³¹: für Cyano, Methoxycarbonyl, Ethoxycarbonyl oder Methansulfonyl, besonders bevorzugt für Cyano oder Methoxycarbonyl, ganz besonders bevorzugt für Cyano,
- R³²: für Methyl, Ethyl, Propyl oder Cyclohexyl, besonders bevorzugt für Methyl oder Ethyl,
- R³³: für Methyl, Ethyl, Propyl, Butyl, Pentyl, Hexyl, Heptyl, Octyl, Methoxy, Ethoxy, Cyclopentyl, Cyclohexyl, Phenyl oder Tolyl, besonders bevorzugt für p-ständiges Butyl, Pentyl, Hexyl, Heptyl, Octyl, Cyclohexyl oder Phenyl, ganz besonders bevorzugt für p-ständiges n-Butyl, tert.-Butyl, 1,1,3,3-Tetramethylbutyl, 2-Ethylhexyl, Cyclohexyl oder Phenyl, und
- B³: für eine Brücke der Formel

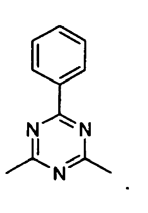

Als besonders bevorzugte Grünfarbstoffe eignen sich beispielsweise solche der Formel (CCLI) 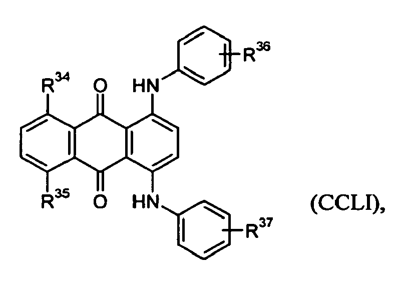 worin
- R³⁴ und R³⁵: unabhängig voneinander für Wasserstoff, Hydroxy oder C₁-C₄-Alkoxy stehen,
- R³⁶ und R³⁷: unabhängig voneinander für Wasserstoff, gegebenenfalls substituiertes C₁-C₈-Alkyl, gegebenenfalls substituiertes C₁-C₈-Alkoxy, Fluor, gegebenenfalls substituiertes C₃-C₇- Cycloalkyl oder gegebenenfalls substituiertes C₆-C₁₀-Aryl stehen.

Bevorzugt stehen
- R³⁴ und R³⁵: für Wasserstoff oder Hydroxy, besonders bevorzugt für Wasserstoff, und sind gleich,
- R³⁶ und R³⁷: unabhängig voneinander für Wasserstoff, Methyl, Ethyl, Propyl, Butyl, Methoxy, Brom, Cyclohexyl oder Phenyl, besonders bevorzugt für p-ständiges Methyl, Ethyl, Butyl, ganz besonders bevorzugt beide gleichermaßen für p-ständiges Methyl oder tert.-Butyl.

Als besonders bevorzugte blaue Farbstoffe eignen sich solche der Formel (CCCI) 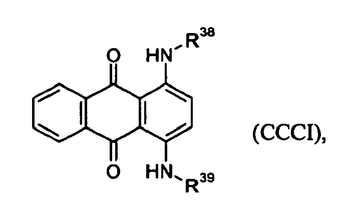 worin
- R³⁸ und R³⁹: unabhängig voneinander für gegebenenfalls substituiertes C₁-C₈-Alkyl, gegebenenfalls substituiertes C₃-C₇-Cycloalkyl oder gegebenenfalls substituiertes C₆-C₁₀-Aryl stehen.

Bevorzugt stehen
- R³⁸ und R³⁹: unabhängig voneinander für Methyl, Ethyl, Propyl, Butyl, Pentyl, Hexyl, Cyclopentyl, Cyclohexyl oder für einen Phenylrest der Formel 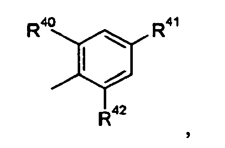
- R⁴⁰: Methyl, Ethyl oder Propyl,
- R⁴¹: für Wasserstoff, Methyl, Ethyl, Propyl, Butyl oder Cyclohexyl,
- R⁴²: Wasserstoff, Methyl, Ethyl oder Propyl.

Besonders bevorzugt stehen
- R³⁸ und R³⁹: für Methyl, Ethyl, Propyl, Butyl, Cyclohexyl oder für einen Phenylrest der Formel 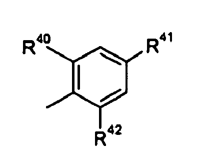 und sind gleich,
- R⁴⁰: Methyl oder Ethyl,
- R⁴¹: für Wasserstoff, Methyl oder Ethyl,
- R⁴²: Methyl oder Ethyl.

Die erfindungsgemäßen Mischungen, enthaltend wenigstens einen Rotfarbstoff der Formel (I) und wenigstens einen Orangefarbstoff der Formel (L) bis (LV) werden weiterhin vorzugsweise mit einem gelben und einem grünen oder blauen Farbstoff im Verhältnis von [(I) + (L) bis (LV)]: gelb: [grün bzw. blau] von 30-70:20-50:1-30, vorzugsweise 45-65:25-40:15-25 gemischt.

Die erfindungsgemäßen Mischungen, enthaltend wenigstens einen Rotfarbstoff der Formel (C) und wenigstens einen Orangefarbstoff der Formel (L) bis (LV) werden mit einem gelben und einem grünen oder blauen Farbstoff im Verhältnis von [(C) + (L) bis (LV)]: gelb: [grün bzw. blau] von 15-40:20-70:20-50, vorzugsweise 20-35:25-65:25-40 gemischt.

Geeignete Farborte für solche braunen Mischungen sind im Lab-System beispielsweise für L von 30-80, a von 10-30 und b von 40-80, vorzugsweise L von 50-75, a von 10-25 und b von 42-70.

Beispiele sind: 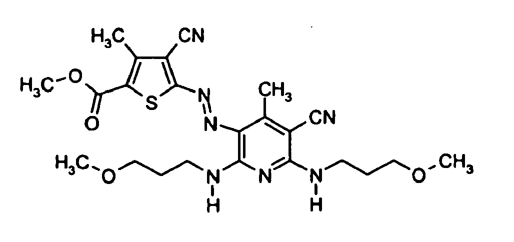 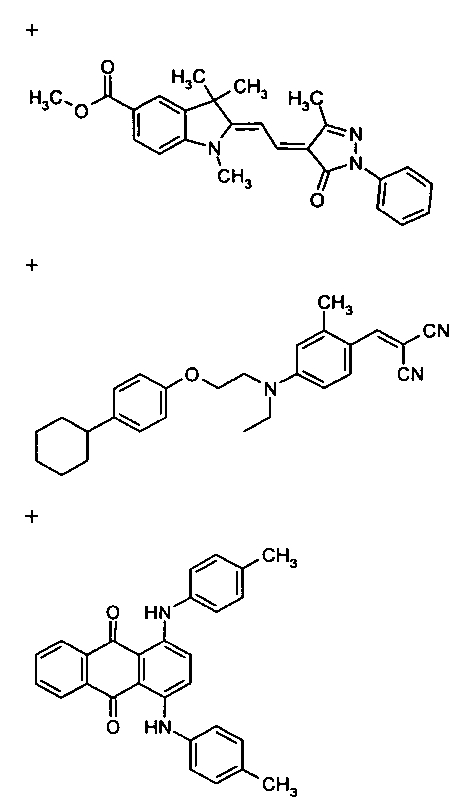 vorzugsweise im Verhältnis 16,3:15,8:40,9:27,0, 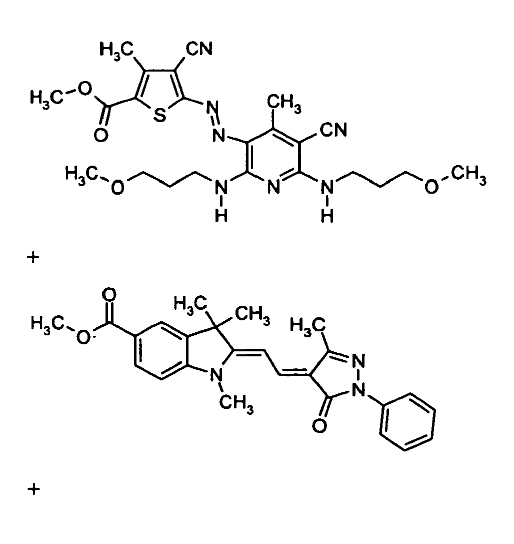 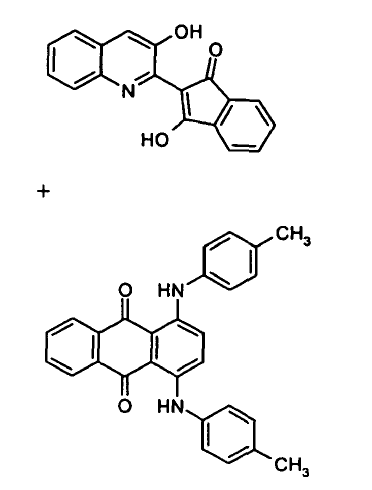 vorzugsweise im Verhältnis 16,0:15,5:41,8:26,7, 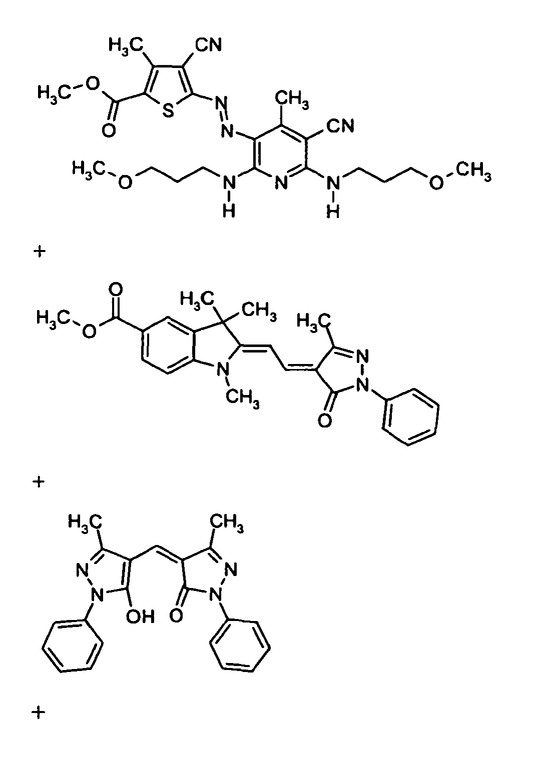 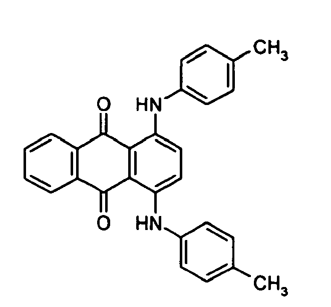 im Verhältnis 9,0:8,7:66,7:15,6, 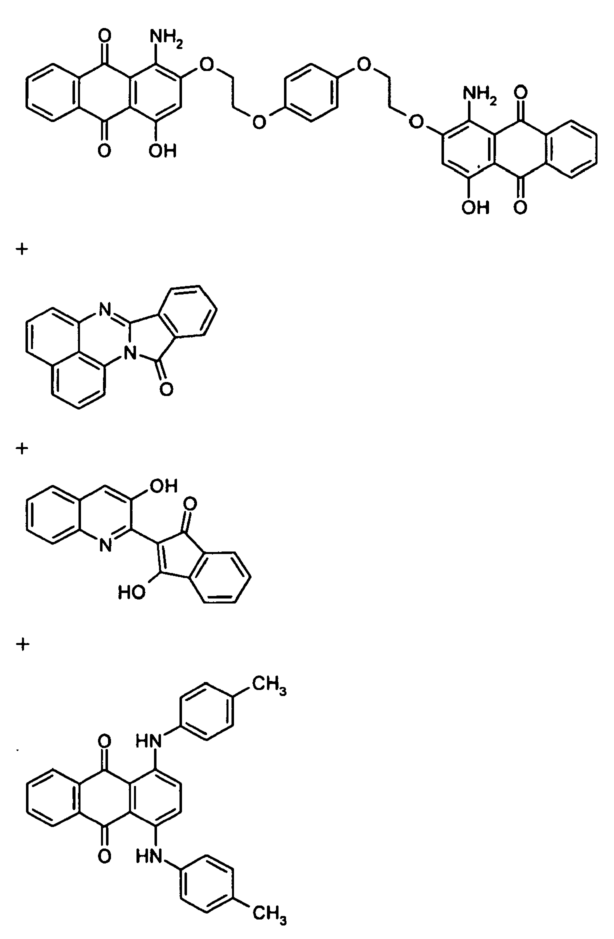 vorzugsweise im Verhältnis 33,8:20,1:27,1:18,0, 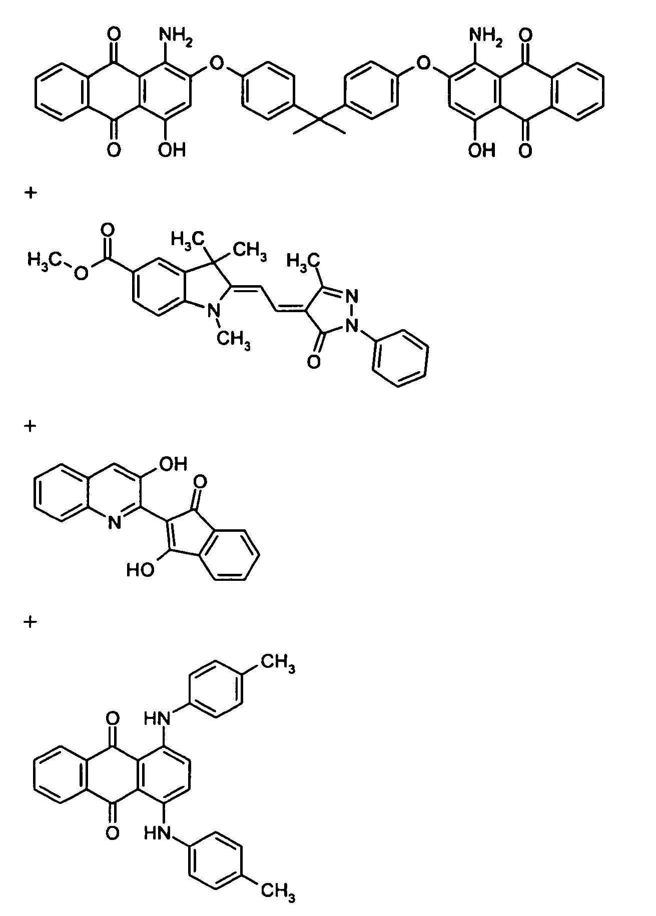 vorzugsweise im Verhältnis 32,1:19,4:29,1:19,4, 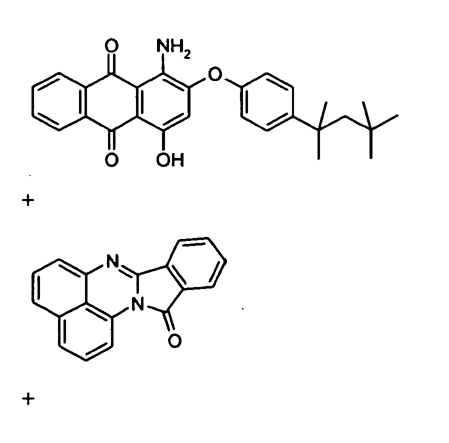 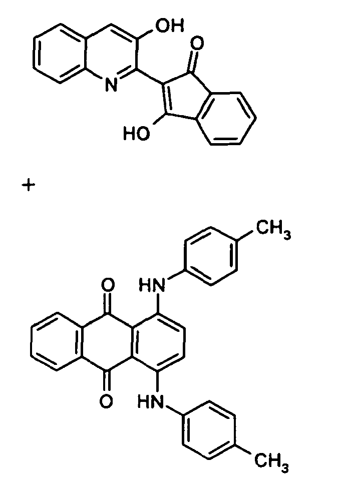 vorzugsweise im Verhältnis 38,1:17,3:26,8:17,8, 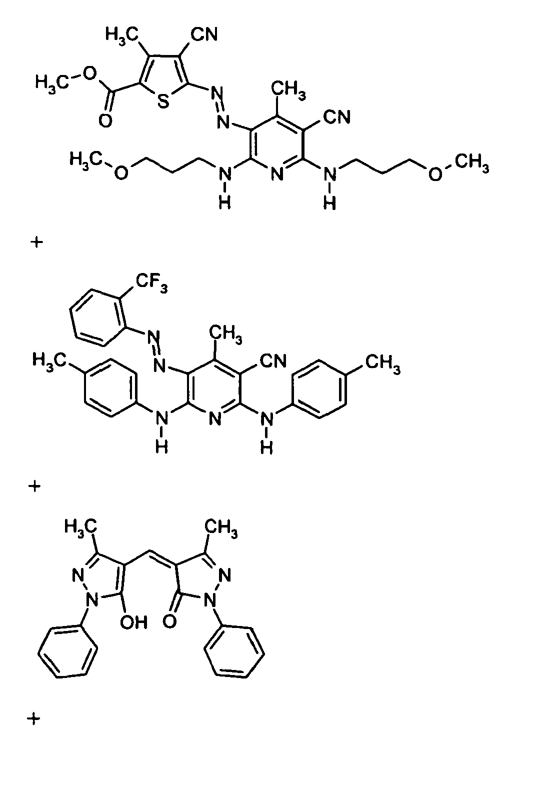 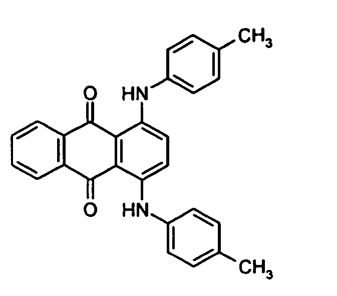 vorzugsweise im Verhältnis 9,0:8,5:67,0:15,5.

Zur Vermeidung von Dosierproblemen und zum Erreichen einer homogenen Verteilung der erfindungsgemäßen Mischung im Kunststoff, bevorzugen viele Kunststoffverarbeiter ein Konzentrat des jeweiligen Additiv, hier also der Farbstoffmischung in den Basispolymeren oder anderen Carriern.

Diese Konzentrate werden im Rahmen dieser Anmeldung Masterbatches genannt.

Die Erfindung betrifft daher auch Masterbatches, enthaltend 15 - 70 Gew.%, vorzugsweise 40 bis 70 Gew.% der erfindungsgemäßen Mischung und einem Carrier.

Vorzugsweise ergibt sich die Menge des Carriers als Rest zu 95 Gew.%, vorzugsweise zu 98 Gew.%, insbesondere zu 99 Gew.% des Masterbatches.

Als Carrier eignen sich vorzugsweise die Basis polymere des zu färbenden Kunststoffes selbst. Derartige Masterbatches sind in der Regel fest. Als besonders bevorzugte Basispolymere sind Thermoplaste, beispielsweise Vinylpolymere, Polyester, Polyamide sowie Polyolefine, insbesondere Polyethylen und Polypropylen, oder Polycarbonate.

Geeignete Vinylpolymere sind Polystyrol, Styrol-Acrylnitril-Copolymere, Styrol-Butadien-Copolymere, Styrol-Butadien-Acrylnitril-Terpolymere, Polymethacrylat, Polyvinylchlorid u. a.

Weiterhin geeignet sind Polyester wie beispielsweise Polyethylenterephthalate, Polycarbonate und Celluloseester.

Bevorzugt sind Polystyrol, Styrol-Mischpolymere, Polyethylenterephthalate, Polycarbonate, Polymethacrylate und Polyamide. Besonders bevorzugt sind Polystyrol, Polyethylenterephthalate, Polycarbonate und Polymethacrylate zu nennen. Ganz besonders geeignet sind Polyethylenterephthalate.

Vorzugsweise wird zur Herstellung solcher Masterbatches jeweils ein Basispolymer bzw. eine Mischung mehrerer Basispolymere mit der erfindungsgemäßen Farbstoffmischung erwärmt, aufgeschmolzen, gemischt und in eine schüttfähige Form überführt, beispielsweise in Granulatform, z. B. als erstarrte Tröpfchen, Kugeln, Hohlkugeln, Schuppen und dergleichen.

Sollten die Farbstoffmischungen erst nach der Polymerisation der Kunststoffe eingesetzt werden, so werden sie beispielsweise mit dem Kunststoffgranulat trocken vermischt oder vermahlen und dieses Gemisch z. B. auf Mischwalzen oder in Schnecken plastifiziert und homogenisiert. Man kann die Mischungen aber auch der schmelzflüssigen Masse zugeben und durch Rühren homogen verteilen. Das derart vorgefärbte Material wird dann wie üblich z. B. durch Verspinnen zu Borsten, Fäden usw. oder durch Extrusion oder im Spritzguss-Verfahren zu Formteilen weiterverarbeitet.

Als Carrier sind ebenfalls Dispersions Carrier bevorzugt. Die Verwendung solcher Carrier führt vorzugsweise zu flüssigen, pumpbaren Masterbatches.

Die Erfindung betrifft weiterhin die Verwendung der erfindungsgemäßen Mischung zum Massefärben von Kunststoffen.

Unter Massefärben werden hierbei insbesondere Verfahren verstanden, bei denen die Farbstoffmischung in die geschmolzene Kunststoffmasse eingearbeitet werden, z. B. unter Zuhilfenahme eines Extruders, oder bei denen der Farbstoff bereits den Ausgangskomponenten zur Herstellung des Kunststoffs, z. B. Monomeren vor der Polymerisation, zugesetzt wird.

Besonders bevorzugte Kunststoffe sind Thermoplaste, beispielsweise Vinylpolymere, Polyester, Polyamide sowie Polyolefine, insbesondere Polyethylen und Polypropylen, oder Polycarbonate.

Geeignete Vinylpolymere sind Polystyrol, Styyrol-Acrylnitril-Copolymere, Styrol-Butadien-Copolymere, Styrol-Butadien-Acrylnitril-Terpolymere, Polymethacrylat, Polyvinylchlorid u. a.

Weiterhin geeignet sind Polyester wie beispielsweise Polyethylenterephthalate, Polycarbonate und Celluloseester.

Bevorzugt sind Polystyrol, Styrol-Mischpolymere, Polyethylenterephthalate, Polycarbonate, Polymethacrylate und Polyamide. Besonders bevorzugt sind Polystyrol, Polyethylenterephthalate, Polycarbonate und Polymethacrylate.

Die erwähnten hochmolekularen Verbindungen können einzeln oder in Gemischen, als plastische Massen oder Schmelzen vorliegen. Besonders bevorzugt ist Polyethylenterephthalat (PET).

Die erfindungsgemäßen Mischungen werden beispielsweise in feinverteilter Form zur Anwendung gebracht, wobei Dispergiermittel mitverwendet werden können, aber nicht müssen. Sie können jedoch beispielsweise auch in Masterbatches, beispielsweise granulierter Form, z. B. als erstarrte Tröpfchen, Kugeln, Hohlkugeln, Schuppen und dergleichen zur Anwendung gebracht werden.

Da die Farbstoffmischungen gegen Polymerisationskatalysatoren, insbesondere Peroxiden beständig sind, ist es auch möglich, sie den monomeren Ausgangsmaterialien für die Kunststoffe zuzusetzen und dann in Gegenwart von Polymerisationskatalysatoren zu polymerisieren. Dazu werden die Farbstoffmischungen vorzugsweise in den monomeren Komponenten gelöst oder mit ihnen innig vermischt.

Die erfindungsgemäßen Farbstoffmischungen werden vorzugsweise in Mengen von 0,0001 bis 1 Gew.-%, insbesondere 0,01 bis 0,5 Gew.-%, bezogen auf den Kunststoff oder die Synthesefasern eingesetzt.

Bei Einsatz von Masterbatches erfolgt die Menge so, dass sich vorzugsweise ebenfalls diese Farbstoffmischungsanteile, bezogen auf den Kunststoff, ergeben.

Durch Zusatz von in den Polymeren unlöslichen Pigmenten wie z. B. Titandioxid, können entsprechende wertvolle gedeckte Färbungen erhalten werden.

Titandioxid kann in einer Menge von 0,01 bis 10 Gew.-%, vorzugsweise 0,1 bis 5 Gew.-% bezogen auf die Polymermenge verwendet werden.

Man erhält transparente bzw. gedeckte blaustichig rote Färbungen mit guter Hitzebeständigkeit sowie guter Licht-, Wetter-, Sublimier- und Migrierechtheit. Bei Mitverwendung von gelben und grünen, bzw. blauen Farbstoffen erhält man braune Färbungen mit den gleichen Echtheiten.

Die Erfindung betrifft daher auch ein Verfahren zum Massefärben von Kunststoffen, dadurch gekennzeichnet, dass man die Mischung gemäß Anspruch 1, vorzugsweise in Form ihres Masterbatches in die geschmolzene Kunststoffmasse einarbeitet oder bereits den Ausgangskomponenten zur Herstellung des Kunststoffs vor der Polymerisation zusetzt.

Die Erfindung betrifft weiterhin Kunststoffe, enthaltend die erfindungsgemäße Farbmischung. Solche Kunststoffe sind insbesondere Hohlkörper wie Flaschen, insbesondere Getränkeflaschen.

Man erhält transparente bzw. gedeckte rote bzw. braune Färbungen mit guter Hitzebeständigkeit sowie guter Licht-, Wetter-, Sublimier- und Migrierechtheit.

Insbesondere die Sublimations- und Migrationsechtheiten der erfindungsgemäßen Farbstoffe der Formel (I) sind verglichen mit beispielsweise dem Farbstoff C. I. Solvent Red 60 deutlich verbessert.

Die erfindungsgemäßen roten und braunen Mischungen zeigen ebenfalls gute Echtheiten, insbesondere gute Sublimations- und Migrationsechtheiten. Diese Mischungen zeichnen sich auch durch hohe Löslichkeit in den Kunststoffen, insbesondere in Masterbatches aus. Verglichen mit Braunmischungen auf Basis von C. I. Solvent Red 135 ist die Löslichkeit in Masterbatches deutlich erhöht, was wegen der relativ hohen Molmasse der erfindungsgemäßen verbrückten Farbstoffe der Formel (I) unerwartet ist.

Die Erfindung betrifft weiterhin Masterbatche 5, enthaltend 15 bis 90 Gew.%, vorzugsweise 15 bis 70 Gew.% Verbindungen der Formel I und einen Carrier 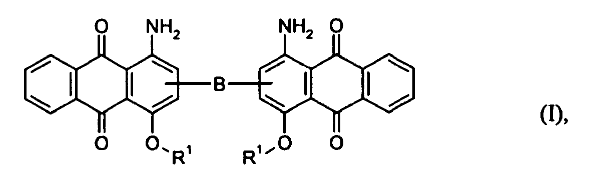 worin
- -B-: für -O-B¹-O- oder -CH₂-B²-CH₂- steht,
- R¹: für Wasserstoff, gegebenenfalls substituiertes C₁-C₈-Alkyl oder gegebenenfalls substituiertes C₃-C₈-Cycloalkyl steht,
- B¹: für eine Brücke der Formel 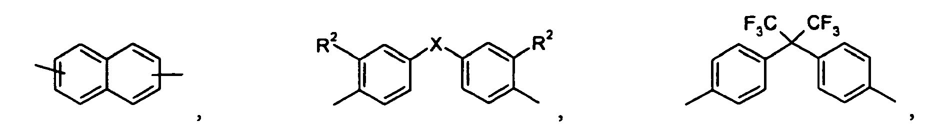 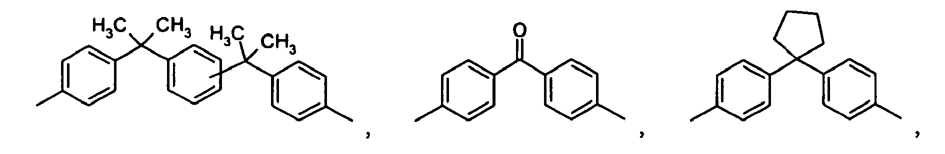 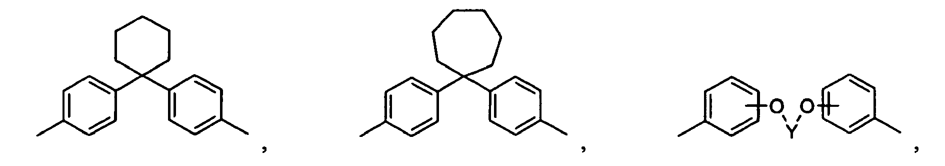 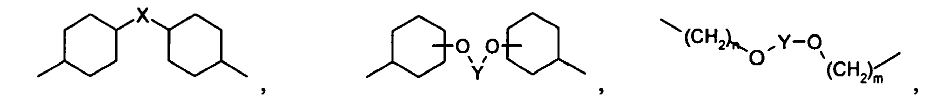 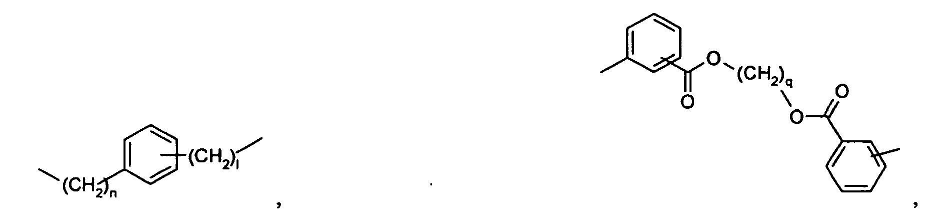 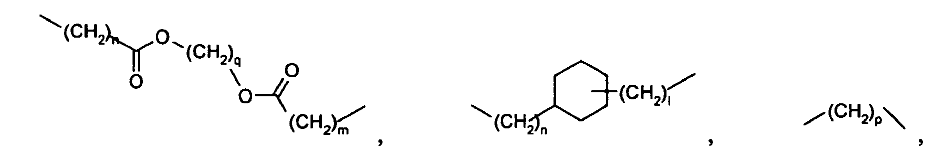 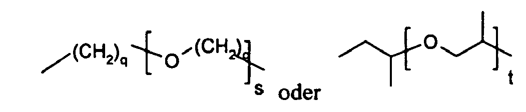 steht,
- B²: für eine Brücke der Formel 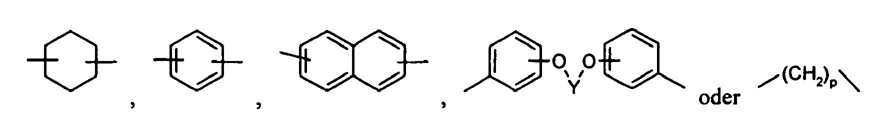 steht,
- R²: für Methyl, Ethyl, Methoxy oder Fluor steht,
- X: für eine Brücke der Formel 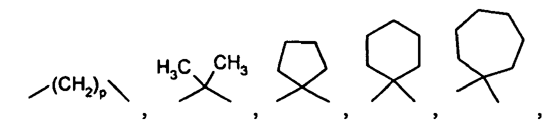 O, S, SO₂ oder CO
steht,
- Y: für eine Brücke der Formel 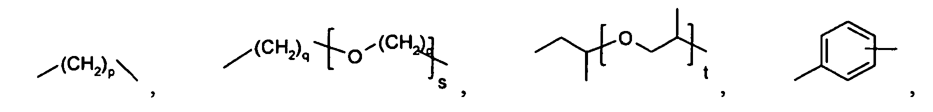 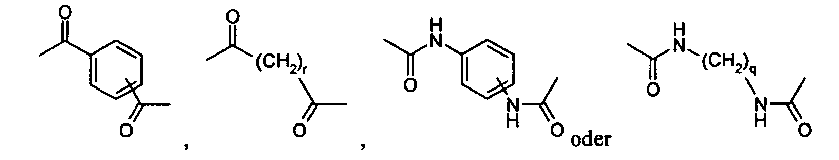 steht,
- l: für eine ganze Zahl von 0 bis 8 steht,
- m, n, p und r: unabhängig voneinander für eine ganze Zahl von 1 bis 8 stehen,
- q: für eine ganze Zahl von 2 bis 8 steht und
- s und t: unabhängig voneinander für eine ganze Zahl von 1 bis 4 stehen.

Bevorzugt entspricht die Verbindung der Formel (I) den Formeln (Ia) bis (Ic) 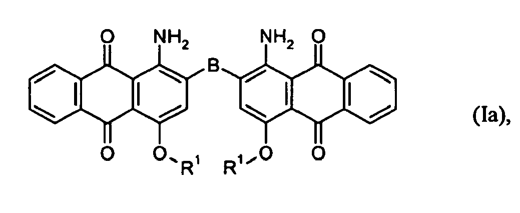 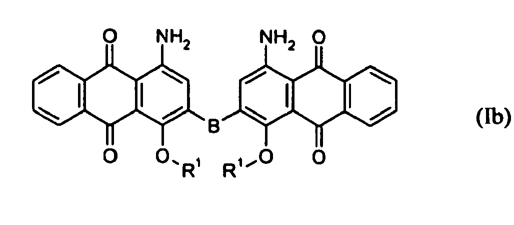 und 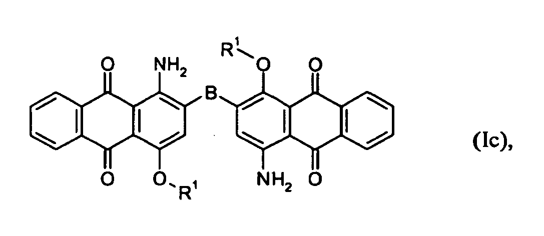 insbesondere Formel (Ia).

Im Falle der Naphthalinstruktur greifen die unbestimmten Bindungen vorzugsweise in den Positionen 1,5-, 1,8-, 2,6- oder 2,7- an. Im Falle der Benzol- und Cyclohexanstrukturen greifen die unbestimmten Bindungen in den Positionen 1,2-, 1,3- oder 1,4- an.

Unter C₁-C₈-Alkyl wird hier und im Folgenden geradkettiges bzw. verzweigtes C₁-C₈-Alkyl, z. B. Methyl, Ethyl, n-Propyl, 2-Propyl, n-Butyl, 2-Butyl, iso-Butyl, tert.-Butyl, Pentyl, Hexyl, Heptyl, Octyl, 2-Ethylhexyl verstanden.

Unter substituiertem C₁-C₈-Alkyl wird geradkettiges bzw. verzweigtes C₁-C₈-Alkyl, wie oben beschrieben, verstanden, das durch mindestens einen der Reste Fluor, Cyano, Hydroxy, C₁- bis C₄-Alkoxy, Acetoxy, Propoxy, Butoxy, C₁-C₄-Alkoxycarbonyl, Cyclopentyl, Cyclohexyl, Phenyl, Naphthyl substituiert ist, z. B. Trifluormethyl, Perfluorbutyl, Cyanmethyl, 2-Cyanethyl, 2-Methoxyethyl, 2-Ethoxyethyl, 2-Acetoxyethyl, 2-Methoxycarbonylethyl, Cyclohexylmethyl, Benzyl, Phenethyl oder Phenylpropyl.

Hier und im Folgenden ist beispielsweise mit Butyl stets n-Butyl, 2-Butyl, iso-Butyl oder tert.-Butyl gemeint, wenn nicht ausdrücklich etwas Anderes gesagt wird.

Unter C₃-C₈-Cycloalkyl wird hier und im Folgenden Cyclopropyl, Cyclobutyl, Cyclopentyl, Cyclohexyl, Cycloheptyl oder Cyclooctyl verstanden. Unter substituiertem C₃-C₈-Cycloalkyl wird beispielsweise Methylcyclohexyl verstanden.

Vorzugsweise steht R¹ für Wasserstoff. Ebenfalls vorzugsweise steht R¹ für Methyl.

Bevorzugte Brücken B¹ sind: 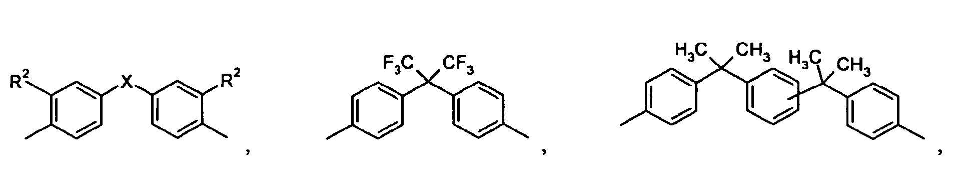 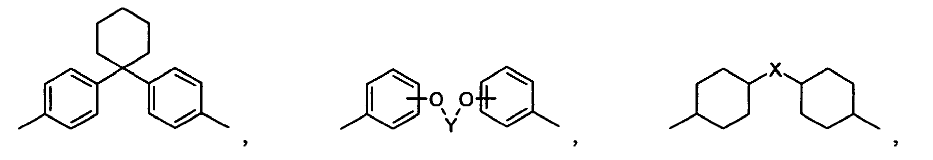 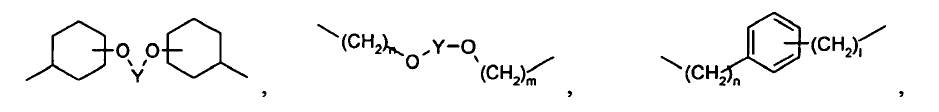 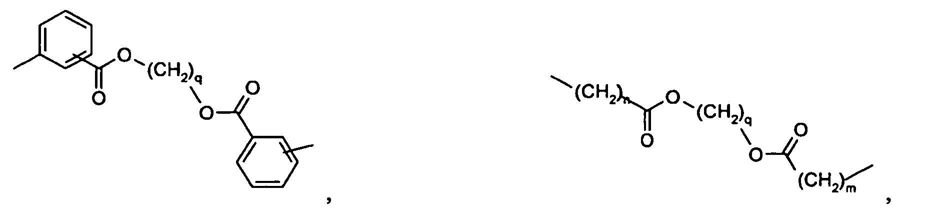 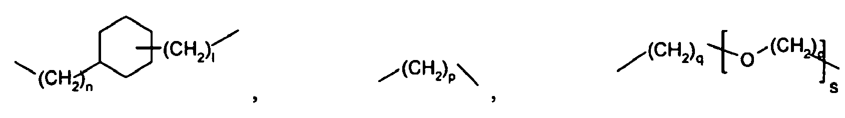 oder 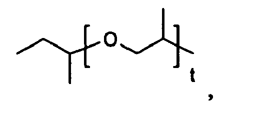 worin
- R²: für Methyl steht,
- X: für eine Brücke der Formeln 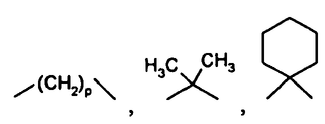 oder O
steht,
- Y: für eine Brücke der Formel 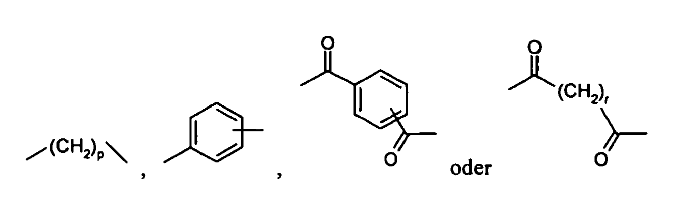 steht,
- l: für eine ganze Zahl von 0 bis 4 steht,
- m, n und p: unabhängig voneinander für eine ganze Zahl von 1 bis 4 stehen,
- q und r: unabhängig voneinander für eine ganze Zahl von 2 bis 6 steht,
- s und t: unabhängig voneinander für eine ganze Zahl von 1 bis 2 stehen und
die unbestimmten Ringpositionen 1,2-, 1,3- oder 1,4- bedeuten.

Besonders bevorzugte Brücken B¹ sind: 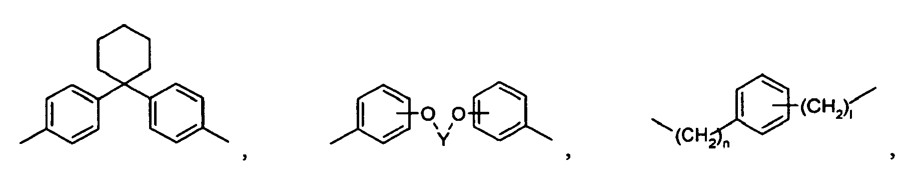 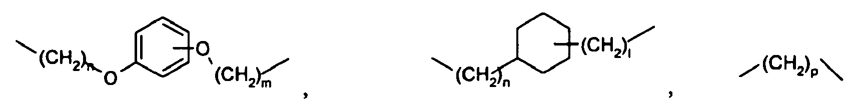 oder 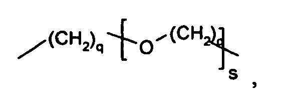 worin
- Y: für eine Brücke der Formel 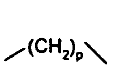 steht,
- l: für eine ganze Zahl von 1 bis 2 steht,
- m, n und p: unabhängig voneinander für eine ganze Zahl von 1 bis 2 stehen,
- q: für eine ganze Zahl von 2 bis 4 steht,
- s und t: unabhängig voneinander für 1 stehen und
die unbestimmten Ringpositionen 1,4- bedeuten.

Bevorzugte Brücken B² sind: 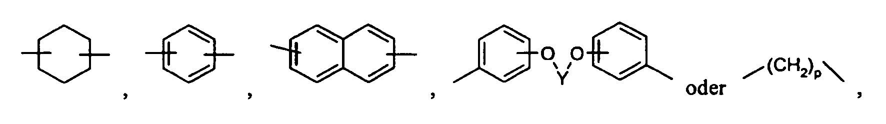 worin
- Y: für eine Brücke der Formeln 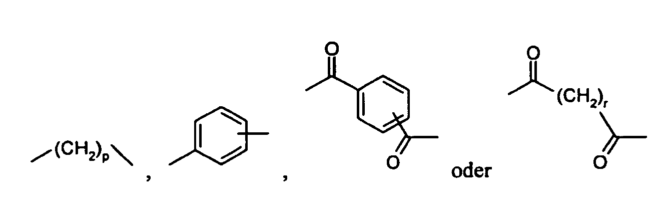 steht,
- p: für eine ganze Zahl von 1 bis 6 steht und
- r: für eine ganze Zahl von 2 bis 6 steht.

Besonders bevorzugte Brücken B² sind: 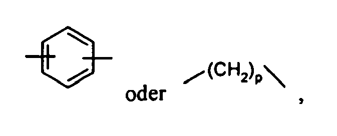 worin
- p: für eine ganze Zahl von 1 bis 4 steht.

Die Erfindung betrifft weiterhin die Verwendung von der Verbindung der Formel zum Massefärben von Kunstsoffen.

Die Erfindung betrifft weiterhin die Verwendung der Verbindungen der Formel I oder der erfindungsgemäßen Mischungen zum Färben von Synthesefasern, vorzugsweise in dispergierter Form.

Ein Verfahren zur Herstellung der Farbstoffe der Formel (I),
worin
- B: für eine Brücke -O-B¹-O- steht,
erfolgt beispielsweise so, dass man eine Anthrachinonverbindung der Formel (II) 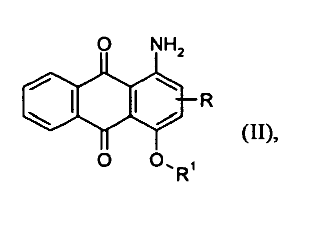 worin
- R¹: für Wasserstoff, gegebenenfalls substituiertes C₁-C₈-Alkyl oder gegebenenfalls substituiertes C₃-C₈-Cycloalkyl steht und
- R: für Chlor, Brom oder Phenoxy steht,
mit einem bifunktionellen Alkohol der Formel (III) umsetzt

HO-B¹-OH (III),

worin
- B¹: für eine Brücke der Formel 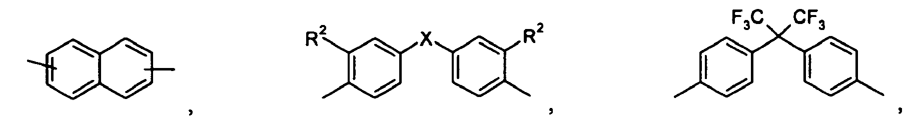 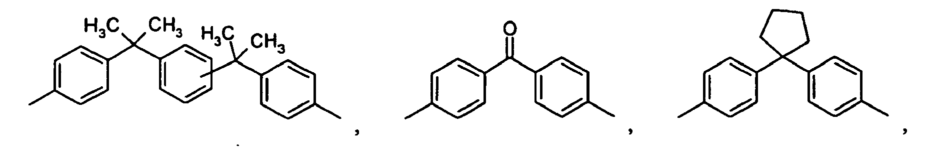 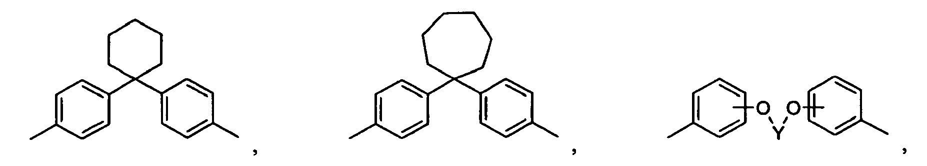 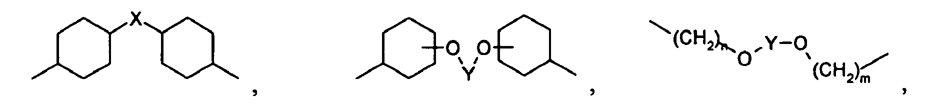 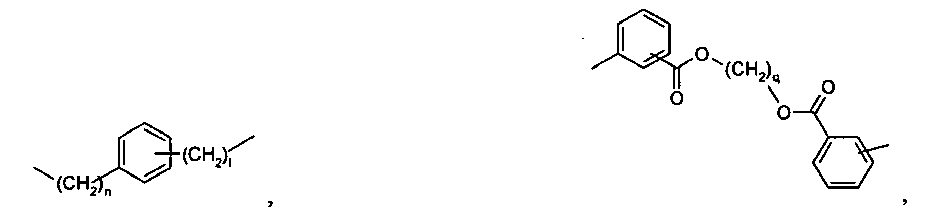 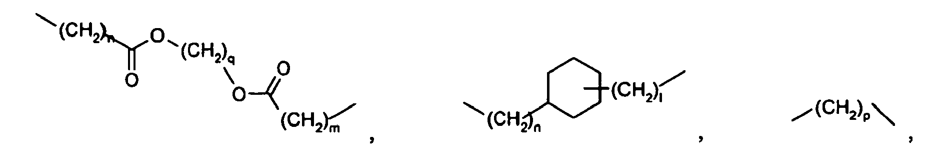 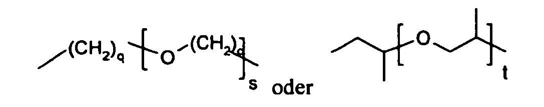 steht,
- R²: für Methyl, Ethyl, Methoxy oder Fluor steht,
- X: für eine Brücke der Formel 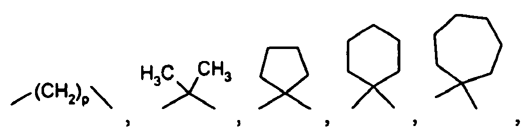 O, S, SO₂ oder CO
steht,
- Y: für eine Brücke der Formel 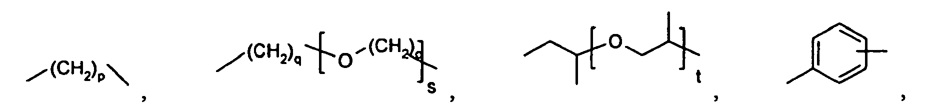 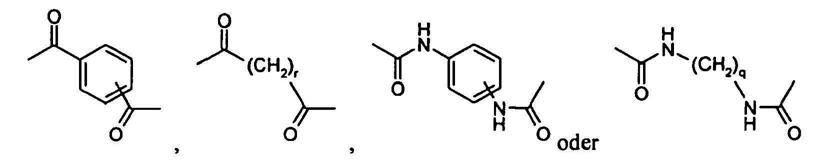 steht,
- l: für eine ganze Zahl von 0 bis 8 steht,
- m, n, p und r: unabhängig voneinander für eine ganze Zahl von 1 bis 8 stehen,
- q: für eine ganze Zahl von 2 bis 8 steht und
- s und t: unabhängig voneinander für eine ganze Zahl von 1 bis 4 stehen.

Farbstaffe dieses Typs (dimere Aminoanthrachinone) sind bekannt; siche CN1597788, US 5578419, EP 0564237 sowie EP 0546856.

Ein Verfahren zur Herstellung der Farbstoffe der Formel (I),
worin
- B: für eine Brücke -O-B¹-O- steht und
- B¹: für eine Brücke der Formel 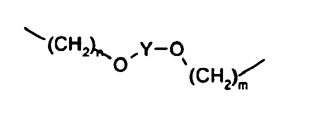 steht,
worin
- Y: für eine bifunktionelle Gruppe der Formel 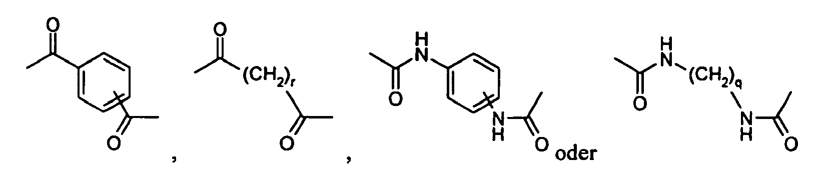 steht,
- m, n und r: unabhängig voneinander für eine ganze Zahl von 1 bis 8 stehen und n und m gleich sind und
- q: für eine ganze Zahl von 2 bis 8 steht,
erfolgt beispielsweise so, dass man eine Anthrachinonverbindung der Formel (IV) 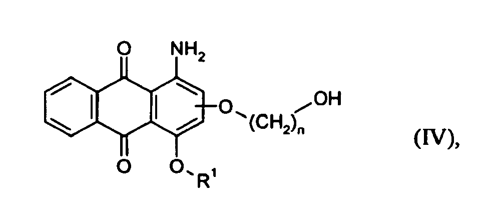 worin
- R¹: für Wasserstoff, gegebenenfalls substituiertes C₁-C₈-Alkyl oder gegebenenfalls substituiertes C₃-C₈-Cycloalkyl steht und
- n: für eine ganze Zahl von 1 bis 8 steht,
mit einem bifunktionellen Säurechlorid oder Ester der Formeln (V) 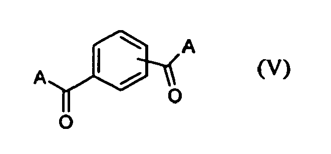 oder (VI)

A-CO-(CH₂)ᵣ-CO-A (VI)

oder einem bifunktionellen Isocyanat der Formeln (VII) 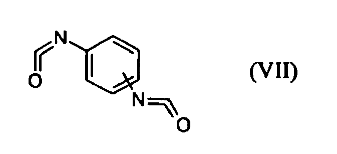 oder (VIII)

O=C=N-(CH₂)_{q}-N=C=O (VIII)

worin
- A: für Cl oder Methoxy steht,
- r: für eine ganze Zahl von 1 bis 8 steht und
- q: für eine ganze Zahl von 2 bis 8 steht,
umsetzt.

Ein Verfahren zur Herstellung der Farbstoffe der Formel (I),
worin
- B: für eine Brücke -O-B¹-O- steht und
- B¹: für eine Brücke der Formel 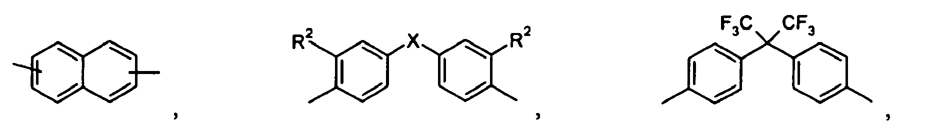 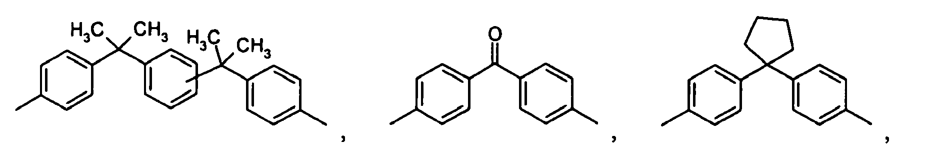 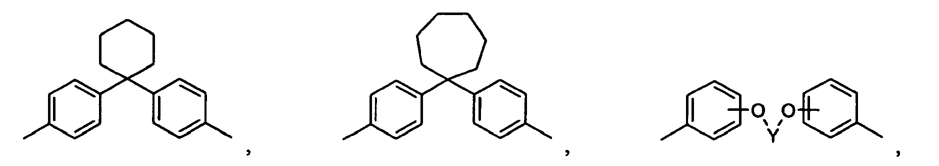 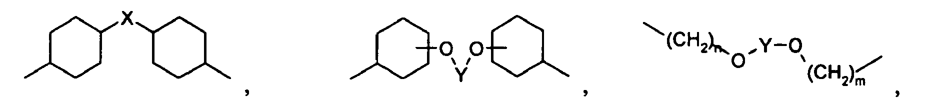 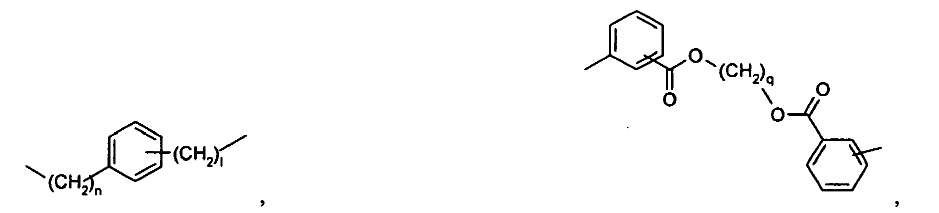 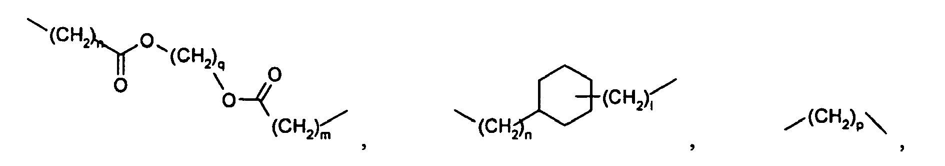 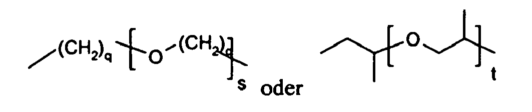 steht,
- R²: für Methyl, Ethyl, Methoxy oder Fluor steht,
- X: für eine Brücke der Formel 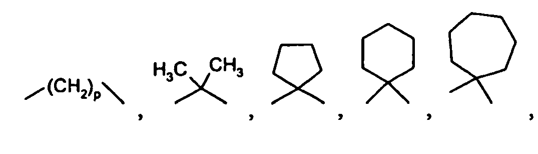 O, S, SO₂ oder CO
steht,
- Y: für eine Brücke der Formel 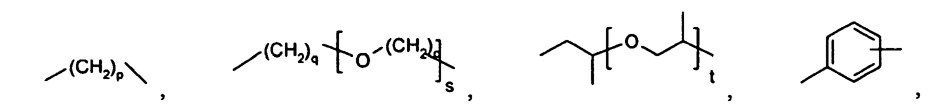 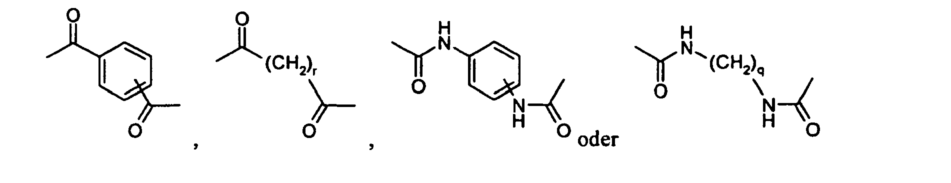 steht,
- l: für eine ganze Zahl von 0 bis 8 steht,
- m, n, p und r: unabhängig voneinander für eine ganze Zahl von 1 bis 8 stehen,
- q: für eine ganze Zahl von 2 bis 8 steht und
- s und t: unabhängig voneinander für eine ganze Zahl von 1 bis 4 stehen,
erfolgt beispielsweise so, dass man eine Anthrachinonverbindung der Formel (II) 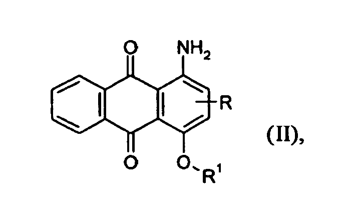 worin
- R¹: für Wasserstoff, gegebenenfalls substituiertes C₁-C₈-Alkyl oder gegebenenfalls substituiertes C₃-C₈-Cycloalkyl steht und
- R: für Chlor, Brom oder Phenoxy steht,
mit einer Anthrachinonverbindung der Formel (IX) 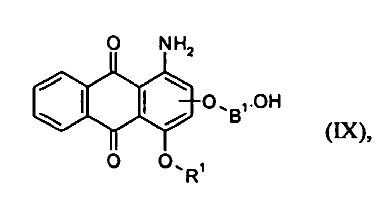 worin
- R¹: für Wasserstoff, gegebenenfalls substituiertes C₁-C₈-Alkyl oder gegebenenfalls substituiertes C₃-C₈-Cycloalkyl steht
umsetzt.

Bei den Formeln (II), (IV) und (IX) sind analog zu Formel (I) die jeweiligen Isomeren gemeint, z. B. 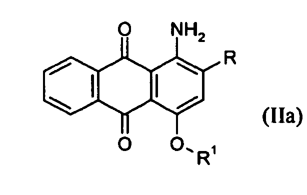 und

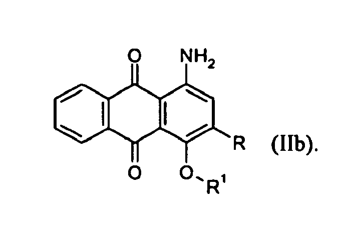

Die Umsetzung erfolgt beispielsweise im Molverhältnis (II)/(III) bzw. (IV)/(V) bzw. (IV)/(VI) bzw. (IV)/(VII) bzw. (IV)/(VIII) von 2/1 bzw. im Molverhältnis (II)/(IX) von 1/1. Es kann jedoch auch vorteilhaft sein, eine der beiden Komponenten relativ zu diesem Verhältnis im Überschuss einzusetzen.

Die Umsetzung erfolgt vorteilhaft in einem Lösungsmittel. Beispiele hierfür sind dipolar aprotische Lösungsmittel wie N-Methyl- oder -ethylpyrrolidon, Dimethylformamid und aromatische Lösungsmittel wie Toluol, Xylol, Chlorbenzol, 1,2-Dichlorbenzol, Benzoesäuremethylester sowie Heterocylen wie Pyridin, Chinolin.

Die Umsetzung erfolgt vorteilhaft in Gegenwart einer Base. Geeignete Basen sind beispielsweise Hydroxide, Carbonate, Amine, z. B. Alkalihydroxide wie Natrium- oder Kaliumhydroxid, Alkalicarbonate wie Natrium- oder Kaliumcarbonat, Amine wie Pyridin, Chinolin, Triethylamin.

Phasentransferkatalysatoren wie quartäre Ammoniumsalze oder Kronenether können ebenfalls zugesetzt werden. Beispiele sind Tetrabutylammoniumbromid, Trimethylbenzylammoniumhydroxid, Tricaprylmethylammoniumchlorid, 18-Krone-6.

Die Umsetzung erfolgt beispielsweise bei Temperaturen im Bereich 80 bis 220 °C, vorzugsweise im Bereich 80 bis 180 °C, besonders vorzugsweise im Bereich 100 bis 160 °C.

Besonders bevorzugt ist bei der Umsetzung der Verbindungen der Formel (II) mit solchen der Formel (III) wasserfreies Natrium- oder Kaliumcarbonat als Base und N-Methyl- oder -ethylpyrrolidon als Lösungsmittel oder wasserfreies Natrium- oder Kaliumcarbonat als Base, 1,2-Dichlorbenzol als Lösungsmittel und 18-Krone-6 oder Tetrabutylammoniumbromid oder Tricaprylmethylammoniumchlorid als Phasentransferkatalysator.
Nach beendeter Umsetzung können beispielsweise die Reaktionsprodukte durch Zusatz von Alkoholen wie Methanol oder Ethanol oder von Wasser oder Mischungen davon ausgefällt und durch Filtrieren isoliert und gegebenenfalls weiter gereinigt werden.

Ein weiteres Verfahren zur Herstellung der Farbstoffe der Formel (I),
worin
- B: für eine Brücke -CH₂-B²-CH₂- steht,
erfolgt beispielsweise so, dass man eine Dihydroanthrachinonverbindung, die in einer tautomeren Form der Formel (X) 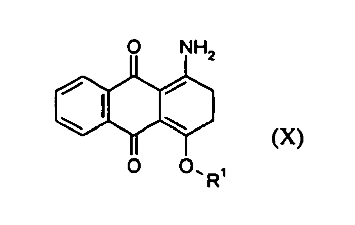 entspricht,
worin
- R¹: für Wasserstoff, gegebenenfalls substituiertes C₁-C₈-Alkyl oder gegebenenfalls substituiertes C₃-C₈-Cycloalkyl steht,
mit einem bifunktionellen Aldehyd der Formel (XI)

OCH-B²-CHO (XI),

worin
- B²: für eine Brücke der Formel 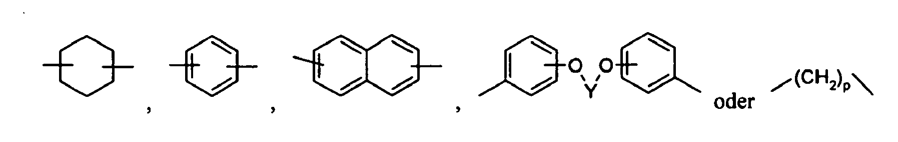 steht,
- Y: für eine Brücke der Formel 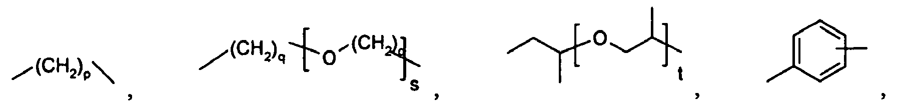 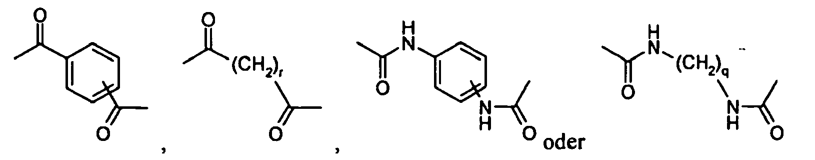 steht,
- p und r: unabhängig voneinander für eine ganze Zahl von 1 bis 8 stehen,
- q: für eine ganze Zahl von 2 bis 8 steht und
- s und t: unabhängig voneinander für eine ganze Zahl von 1 bis 4 stehen,
umsetzt.

Die Umsetzung erfolgt beispielsweise im Molverhältnis (X)/(XI) von 2/1. Es kann jedoch auch vorteilhaft sein, eine der beiden Komponenten relativ zu diesem Verhältnis im Überschuss einzusetzen.

Die Umsetzung erfolgt vorteilhaft in einem Lösungsmittel. Beispiele hierfür sind C₁-C₁₀-Alkohole wie Methanol, Ethanol, Propanol, 2-Propanol, Butanol, Pentanol, Hexanol, Benzylalkohol, dipolar aprotische Lösungsmittel wie N-Methyl- oder -ethylpyrrolidon, Dimethylformamid und aromatische Lösungsmittel wie Toluol, Xylol, Chlorbenzol, 1,2-Dichlorbenzol, Benzoesäuremethylester sowie Heterocylen wie Pyridin, Chinolin.

Die Umsetzung erfolgt vorteilhaft in Gegenwart von Säuren und/oder Basen. Geeignete Säuren sind beispielsweise Salzsäure, Benzolsulfonsäure, Toluolsulfonsäure, Essigsäure, Methansulfonsäure. Geeignete Basen sind beispielsweise Amine wie Piperidin, Morpholin, Piperazin, Pyridin, Chinolin, Triethylamin. Ebenfalls vorteilhaft ist die Kombination von Säuren und Basen, beispielsweise Essigsäure und Piperidin.

Die Umsetzung erfolgt beispielsweise bei Temperaturen im Bereich 40 bis 180 °C, vorzugsweise im Bereich 70 bis 120 °C.

Nach beendeter Umsetzung können beispielsweise die Reaktionsprodukte gegebenenfalls durch Zusatz von Alkoholen wie Methanol oder Ethanol oder von Wasser oder Mischungen davon ausgefällt und durch Filtrieren isoliert und gegebenenfalls weiter gereinigt werden.

Masterbatche, enthaltend 15 bis 70 Gew.%, vorzugsweise 40 bis 70 Gew.%.

Die Erfindung betrifft weiterhin die Verwendung von der Verbindungen der Formel I zum Massefärben von Kunststoffen.

Die Erfindung betrifft weiterhin die Verwendung der erfindungsgemäßen Verbindung der Formel I oder der erfindungsgemäßen Mischungen zum Färben von Synthesefasern, vorzugsweise in dispergierter Form.

### Beispiele

### Beispiel 1

2,70 g Cyclohexyliden-bisphenol wurden unter Stickstoffatmosphäre in 20 ml N-Methylpyrrolidon mit 4 ml 50-proz. Kalilauge 3 h bei 100 °C verrührt. Anschließend wurden 6,40 g 1-Amino-2-brom-4-hydroxyanthrachinon eingetragen. 2 h wurde bei 120 °C verrührt, auf 80 °C abgekühlt, mit 15 ml Methanol verdünnt und nach Abkühlen auf Raumtemperatur abgesaugt. Nach Waschen mit 10 ml Methanol und 100 ml heißem Wasser wurde bei 80 °C im Vakuum getrocknet. Man erhielt 3,90 g (52 % d. Th.) eines roten Pulvers der Formel 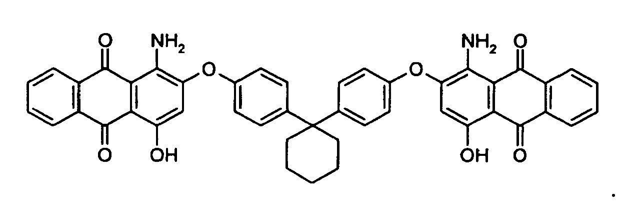 UV/VIS (NMP): λₘₐₓ = 523, 561 nm, ε = 24880 1 mol⁻¹ cm⁻¹ (bei 523 nm).

### Beispiel 2

Analog zu Beispiel 1, aber unter Verwendung von 3,5 g 4,4-(1,3-phenylendiisopropyliden)-bisphenol erhielt man 4,80 g (57,6 % d. Th.) eines roten Pulvers der Formel 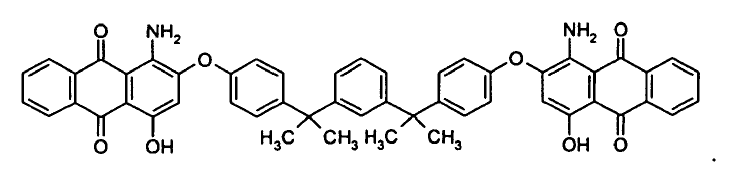 UV/VIS (NMP): λₘₐₓ = 523, 560 nm, ε = 20850 1 mol⁻¹ cm⁻¹ (bei 523 nm).

### Beispiel 3

2,30 g 2,2-Bis(4-hydroxy-3-methylphenyl)propan wurden unter Stickstoffatmosphäre in 15 ml N-Methylpyrrolidon mit 4 ml 50-proz. Kalilauge 3 h bei 100 °C verrührt. Anschließend wurden 5,71 g 1-Amino-2-brom-4-hydroxyanthrachinon eingetragen. 7 h wurde bei 120 °C verrührt, auf 80 °C abgekühlt, mit 15 ml Methanol verdünnt und nach Abkühlen auf Raumtemperatur abgesaugt. Nach Waschen mit 10 ml Methanol und 100 ml heißem Wasser wurde bei 80 °C im Vakuum getrocknet. Man erhielt 1,07 g (16 % d. Th.) eines roten Pulvers der Formel 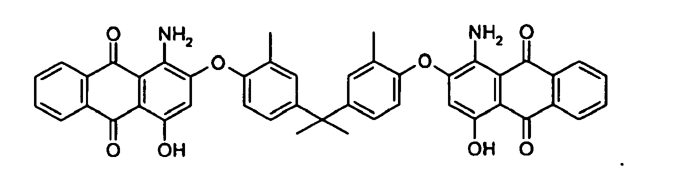 UV/VIS (NMP): λₘₐₓ = 522, 558 nm, ε = 23970 1 mol⁻¹ cm⁻¹ (bei 522 nm).

### Beispiel 4

Unter Stickstoffatmosphäre wurden 2,50 g 1,4-Bis-(2-hydroxyethoxy)benzol, 1,42 g Kaliumhydroxid und 8,36 g 1-Amino-2-phenoxy-4-hydroxyanthrachinon in 20 ml N-Ethylpyrrolidon eingetragen und 11 h bei 130 °C gerührt. Nach Abkühlen auf 80 °C wurde mit 60 ml Methanol verdünnt und nach Abkühlen auf Raumtemperatur abgesaugt. Nach Waschen mit 10 ml Methanol und 100 ml heißem Wasser wurde bei 80 °C im Vakuum getrocknet. Man erhielt 6,80 g (80 % d. Th.) eines roten Pulvers der Formel 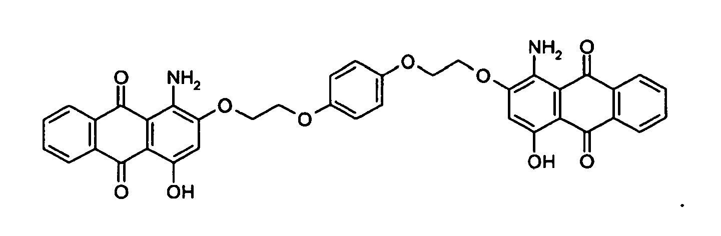 UV/VIS (NMP): λₘₐₓ = 518, 555 nm, ε = 24420 l mol⁻¹ cm⁻¹ (bei 518 nm).

### Beispiel 5

Unter Stickstoffatmosphäre wurden 2,50 g 2,7-Dihydroxynaphthalin, 3,31 g wasserfreies Natriumcarbonat, 0,83 g 18-Krone-6 und 9,93 g 1-Amino-2-brom-4-hydroxyanthrachinon in 25 ml 1,2-Dichlorbenzol eingetragen und 4 h bei 165 °C gerührt. Nach Abkühlen auf 80 °C wurde mit 60 ml Methanol verdünnt, 1 h bei 60 °C gerührt und nach Abkühlen auf Raumtemperatur abgesaugt. Nach Waschen mit 10 ml Methanol und 100 ml heißem Wasser wurde bei 80 °C im Vakuum getrocknet. Man erhielt 7,51 g (76 % d. Th.) eines roten Pulvers der Formel 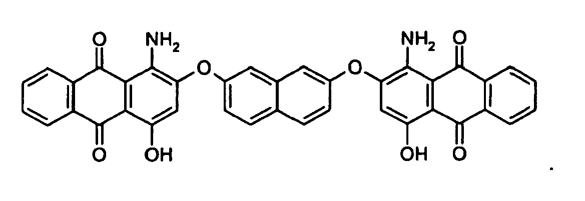 UV/VIS (NMP): λₘₐₓ = 525, 560 nm, ε = 26710 l mol⁻¹ cm⁻¹ (bei 525 nm).

### Beispiel 6

Analog zu Beispiel 5 wurden unter Verwendung von 2,50 g 2,6-Dihydroxynaphthalin 8,00 g (80,8 % d. Th.) eines roten Pulvers der Formel 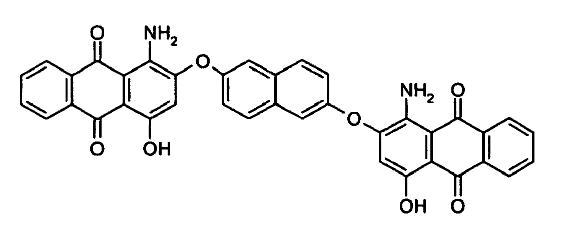 erhalten.
UV/VIS (NMP): λₘₐₓ = 525, 561 nm, ε = 23870 l mol⁻¹ cm⁻¹ (bei 525 nm).

### Beispiel 7

Unter Stickstoffatmosphäre wurden 2,50 g 1,4-Butandiol und 5,88 g Natriumcarbonat in 35 ml N-Ethylpyrrolidon 3 h bei 150 °C gerührt. Dann wurden 18,4 g 1-Amino-2-phenoxy-4-hydroxyanthrachinon eingetragen und es wurde 24 h bei 160 °C gerührt. Nach Abkühlen auf 80 °C wurde mit 60 ml Methanol verdünnt, 1 h bei 60 °C gerührt und nach Abkühlen auf Raumtemperatur abgesaugt. Nach Waschen mit 10 ml Methanol und 100 ml heißem Wasser wurde bei 80 °C im Vakuum getrocknet. Man erhielt 8,53 g eines roten Pulvers. Dieses wurde zur Reinigung zweimal in 100 ml Toluol 2 h gekocht, heiß abgesaugt und mit 250 ml heißem Toluol und anschließend 100 ml heißem Methanol gewaschen. Schließlich wurde mit 100 ml heißem Wasser gewaschen und bei 80 °C im Vakuum getrocknet. Man erhielt 5,42 g (35 % d. Th.) eines roten Pulvers der Formel 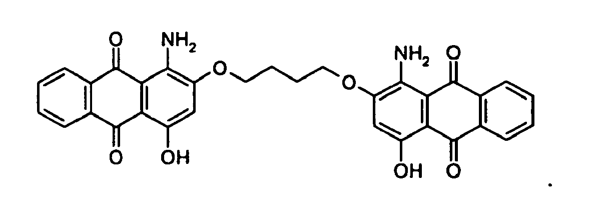 UV/VIS (NMP): λₘₐₓ = 518, 556 nm, ε = 14740 l mol⁻¹ cm⁻¹ (bei 518 nm).

### Beispiel 8

a) 2,24 g Kaliumhydroxid wurden in 62 g Ethylenglycol gelöst. 9,9 g 1-Amino-2-phenoxy-4-hydroxyanthrachinon wurden eingetragen und es wurde 6 h bei 120 °C unter Stickstoffatmosphäre gerührt. Nach Abkühlen auf Raumtemperatur wurde abgesaugt, mit 20 ml Ethylenglycol und 100 ml Wasser gewaschen und bei 80 °C im Vakuum getrocknet. Man erhielt 7,7 g (86 % d. Th.) eines roten Pulvers der Formel 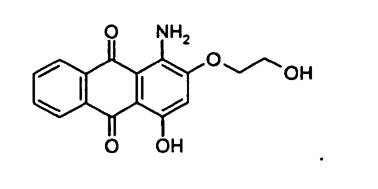
b) 2,0 g des Produkts aus a) wurden unter Stickstoffatmosphäre in 25 ml Pyridin vorgelegt. 0,68 g Terephthalsäuredichlorid wurden zugesetzt und es wurde 7 h bei Rückfluss gerührt. Nach Abkühlen auf Raumtemperatur wurde abgesaugt, mit 10 ml Methanol und 100 ml heißem Wasser gewaschen und bei 80 °C im Vakuum getrocknet. Man erhielt 1,85 g (76 % d. Th.) eines roten Pulvers der Formel 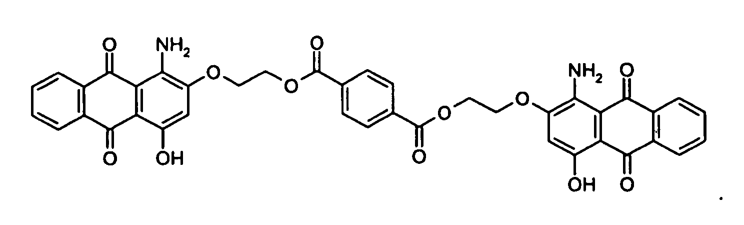
UV/VIS (NMP): λₘₐₓ = 518, 555 nm.

### Beispiel 9

2,53 g 1,4-Cyclohexandimethanol und 3,71 g wasserfreies Natriumcarbonat wurden in 30 ml N-Ethylpyrrolidon unter Stickstoffatmosphäre 3 h bei 120 °C gerührt. 11,49 g 1-Amino-2-phenoxy-4-hydroxyanthrachinon wurden eingetragen und anschließend wurde 15 h bei 160 °C gerührt. Nach dem Abkühlen auf 100 °C wurde mit 60 ml Methanol verdünnt und auf Raumtemperatur abgekühlt. Es wurde abgesaugt, mit 10 ml Methanol und 100 ml heißem Wasser gewaschen und bei 80 °C im Vakuum getrocknet. Das Rohprodukt wurde in 100 ml Toluol 3 h unter Rühren gekocht, heiß abgesaugt, mit 10 ml heißem Toluol und 30 ml Methanol gewaschen und bei 80 °C im Vakuum getrocknet. Man erhielt 2,71 g (25 % d. Th.) eines roten Pulvers der Formel 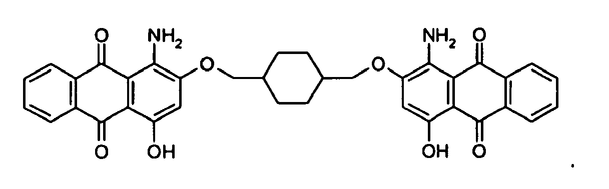 UV/VIS (NMP): λₘₐₓ = 519, 555 nm, ε = 21960 1 mol⁻¹ cm⁻¹ (bei 519 nm).

### Beispiel 10

Analog zu Beispiel 1 wurden unter Verwendung von 2,3 g 2,2-Bis(4-hydroxy-phenyl)propan und 6,69 g 1-Amino-2-brom-4-methoxyanthrachinon 4,73 g (64,3 % d. Th.) des roten Farbstoffs der Formel 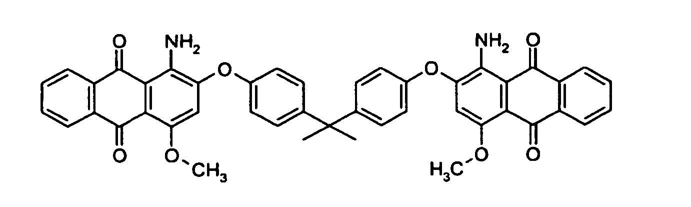 erhalten.
UV/VIS (NMP): λₘₐₓ = 521, 552 nm, ε = 13450 l mol⁻¹ cm⁻¹ (bei 521 nm).

### Beispiel 11

Analog zu Beispiel 1 wurde unter Verwendung von 2,2-Bis(4-hydroxy-phenyl)propan und 1-Amino-3-brom-4-hydroxyanthrachinon der Farbstoff der Formel 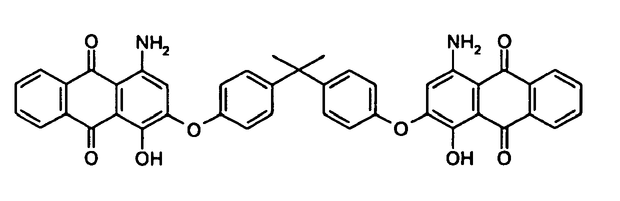 erhalten.

### Beispiel 12

6,70 g Terephthalaldehyd, 24,1 g 1-Amino-4-hydroxy-dihydroanthrachinon, 2,55 g Piperidin und 1,80 g Eisessig wurden unter Stickstoffatmosphäre in 150 ml N-Methylpyrrolidon 8 h bei 150 °C gerührt. Nach dem Abkühlen auf Raumtemperatur wurde die violette Suspension abgesaugt, mit 40 ml N-Methylpyrrolidon, 100 ml Methanol und 300 ml heißem Wasser gewaschen. Nach dem Trocknen bei 80 °C im Vakuum erhielt man 15,8 g (54 % d. Th.) eines violetten Pulvers der Formel 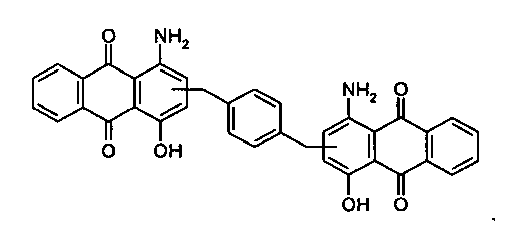 UV/VIS (NMP): λₘₐₓ = 538, 574 nm.

### Färbebeispiele

a) 100 g Polystyrol-Granulat und 0,02 g des Farbstoffs aus Beispiel 4 werden im Trommelmischer während 15 min intensiv vermischt. Das trocken angefärbte Granulat wird bei 240 °C auf einer Schneckenspritzgießmaschine verarbeitet. Man erhält transparente blaustichig rote Platten von sehr guter Lichtechtheit und Migrierechtheit.
   Anstelle von Polystyrol-Polymerisat können auch Mischpolymerisate mit Butadien und Acrylnitril verwendet werden.
   Setzt man zusätzlich 0,5 g Titandioxid zu, so erhält man farbstarke gedeckte Färbungen.
b) 0,025 g des Farbstoffs aus Beispiel 4 werden mit 100 g Polyethylenterephthalat einer transparenten Type vermischt und in einem Zweiwellenextruder bei 280 °C homogenisiert. Man erhält eine transparente blaustichig rote Färbung mit guter Lichtechtheit und Migrierechtheit. Nach anschließender Granulierung kann der eingefärbte Kunststoff nach den üblichen Methoden der thermoplastischen Verformung verarbeitet werden.
   Arbeitet man unter Zusatz von 1 % Titandioxid, so erhält man eine gedeckte Färbung.
c) 100 g eines handelsüblichen Polycarbonats werden in Form von Granulat mit 0,03 g des Farbstoffs aus Beispiel 4 trocken vermischt. Das so bestäubte Granulat wird in einem Zweiwellenextruder bei 290 °C homogenisiert. Man erhält eine transparente blaustichig rote Färbung mit guter Lichtechtheit und Migrierechtheit. Das gefärbte Polycarbonat wird als Strang aus dem Extruder ausgetragen und zu Granulat verarbeitet. Das Granulat kann nach den üblichen Methoden der Konfektionierung thermoplastischer Massen verarbeitet werden.
   Arbeitet man unter Zusatz von 1 % Titandioxid, so erhält man eine gedeckte Färbung.
d) Analog wird verfahren unter Benutzung eines Styrol-Acrylnitril-Copolymerisats, jedoch wird bei 190 °C homogenisiert.
e) In 98,9 g Styrol werden 0,05 g tert.-Dodecylmercaptan sowie 0,05 g des Farbstoffs aus Beispiel 4 gelöst. Diese Lösung dispergiert man in einer Lösung aus 200 g entsalztem Wasser, 0,3 g teilverseiftem Polyvinylacetat (z. B. Mowiol ^{®} 50/88) und 0,05 g Dodecylbenzolsulfonat. Nach Zugabe von 0,1 g Dibenzoylperoxid gelöst in 1 g Styrol wird die Dispersion unter kräftigem Rühren auf 80 °C erhitzt und die Polymerisation gestartet. Bei Anwendung folgender Polymerisationsbedingungen: 4 h bei 80 °C, 2 h bei 90 °C, 3 h bei 110 °C, 2 h bei 130 °C, erhält man das Polymerisat in einer Ausbeute von 98 % der Theorie. Das Polymerisat fällt in Form von Perlen an, die je nach Rührbedingungen einen Durchmesser von 0,1 bis 1,5 mm (D₅₀-Wert) aufweisen. Das Polymerisat wird durch Filtration vom Serum getrennt und bei 110 °C auf eine Restfeuchte von 0,5 % getrocknet. Nach dem Aufschmelzen (Heißwalze) werden 0,5 % Zinkstearat und 0,2 % Ionol zugemischt und das Polymerisat granuliert.

Das blaustichig rot gefärbte Polymerisat kann nach den üblichen Methoden der thermoplastischen Veformung, z. B. im Spritzgussverfahren, zu blaustichig roten transparenten Formteilen verarbeitet werden.

In analoger Weise lassen sich die Farbstoffe der Beispiele 1-3 und 5-11 sowie die Farbstoffe und Mischungen der nachfolgenden Tabelle einsetzen. Man erhält rote oder braune Färbungen mit guten Echtheiten.

### Mischbeispiele

Es wurden folgende Farbstoffe eingesetzt:
(1) = Formel (Ia) mit R¹ = H und -B- = 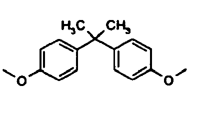
(2) = Formel (Ia) mit R¹ = H und -B- = 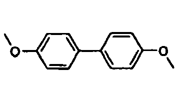
(3) = Beispiel 4 = Formel (Ia) mit R¹ = H und -B- = 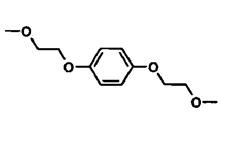
(4) = Formel (L) mit R¹⁰ = COOCH₃ und R¹¹ = H
(5) = Formel (C) mit R²⁰ = COOCH₃, R²¹ = R²³ = CH₃, R²² = R²⁴ = CN, R²⁵ = -CH₂CH₂CH₂OCH₃, R²⁶ = H.
(6) = Formel (LIII)
(7) = 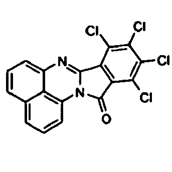 (C. I. Solvent Red 135)
(8) = 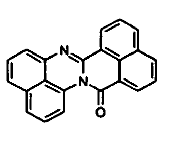
(10) = Formel (CCI)
(11) = Formel (CCII) mit R²⁹ = H
(12) = Formel (CCIII) mit R³⁰ = CH₃, R³¹ = CN, R³² = Ethyl, R³³ = p-ständiges Cyclohexyl
(13) = Formel (CCLI) mit R³⁴ = R³⁵ = H, R³⁶ = R³⁷ = p-ständiges CH₃
(14) = 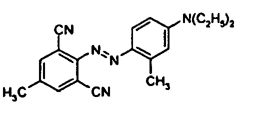
(15) = Beispiel 1 = Formel (Ia) mit R¹ = H und -B- =

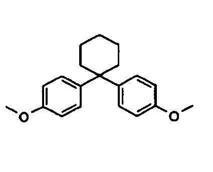

### Färbebeispiele der Mischungen

| | **CIE Farbwerte Transmisslonamessung** | | | | |
|---|---|---|---|---|---|
| **PET transparent 2mm** | **Illum** | **Winkel** | **L*** | **a*** | **b*** |
| (7) (Vergleich) | D65 | 10° | 54,1 | 68,9 | 48,9 |
| (8) (Vergleich) | D65 | 10° | 52,8 | 66,5 | 70,5 |
| (14) (Vergleich) | D65 | 10° | 37,2 | 70,9 | 56,8 |
| (5) (Vergleich) | D65 | 10° | 38,4 | 74,4 | 29,6 |
| Amber 1 a (Vergleich) | D65 | 10° | 61,1 | 14,9 | 59,3 |
| Amber 1b | D65 | 10° | 63,0 | 13,6 | 59,0 |
| Amber 1 c | D65 | 10° | 62,3 | 13,9 | 58,6 |
| Amber 1 d | D65 | 10° | 61,9 | 14,3 | 59,3 |
| | | | | | |
| Amber 2 a (Vergleich) | D65 | 10° | 59,7 | 10,8 | 46,0 |
| Amber 2 b | D65 | 10° | 60,9 | 10,1 | 45,3 |
| Amber 2 c | D85 | 10° | 61,6 | 10,7 | 45,4 |
| Amber 2 d | D65 | 10° | 58,7 | 11,7 | 46,0 |
| | | | | | |
| Amber 3 a (Vergleich) | D65 | 10° | 77,3 | 3,2 | 43,6 |
| Amber 3 b | D65 | 10° | 77,0 | 2,8 | 45,5 |
| Amber 3 c (Vergleich) | D65 | 10° | 62,8 | 13,7 | 58,5 |
| Amber 3 d | D65 | 10° | 62,4 | 14,0 | 59,4 |
| Amber 3 e (Vergleich) | D65 | 10° | 35,5 | 28,3 | 55,4 |
| Amber 3 f | D65 | 10° | 35,7 | 29,4 | 55,1 |
| | | | | | |
| Amber 4 a (Vergleich) | D65 | 10° | 74,6 | 3,7 | 33,6 |
| Amber 4 b | D65 | 10° | 74,9 | 3,5 | 33,6 |
| Amber 4 c (Vergleich) | D65 | 10° | 60.8 | 10.1 | 45,3 |
| Amber 4 e (Vergleich) | D65 | 10° | 30,0 | 21,2 | 44,7 |
| Amber 4 f | D65 | 10° | 30,6 | 22,8 | 44,7 |
| (5) (Vergleich) | D65 | 10° | 43,8 | 80,3 | 3,4 |
| (1) | D65 | 10° | 52,1 | 80,4 | -13,6 |
| (2) | D65 | 10° | 50,2 | 81,0 | -12,1 |
| (3) | D65 | 10° | 55,7 | 77,2 | -4,9 |
| | | | | | |
| Rot 1 a | D65 | 10° | 42,5 | 73,2 | 72,5 |
| Rot 1 b | D65 | 10° | 50,4 | 71,2 | 54,5 |
| Rot 1 c | D65 | 10° | 62,2 | 56,8 | 32,7 |
| | | | | | |
| (7) (Vergleich) | D65 | 10° | 54,0 | 69,0 | 48,9 |
| Rot 2 a | D65 | 10° | 48,2 | 72,1 | 51,3 |
| Rot 2 b | D65 | 10° | 46,6 | 72,4 | 51,1 |
| Rot 2 c | D65 | 10° | 51,9 | 69,8 | 59,9 |
| Rot 2 d | D65 | 10° | 47,5 | 71,3 | 59,1 |
| Rot 2 e | D65 | 10° | 46,1 | 71,7 | 57,9 |
| Rot 2 f | D65 | 10° | 51,1 | 69,6 | 66,0 |
| | | | | | |
| Amber 5 a (Vergleich) | D65 | 10° | 61,7 | 14,5 | 59,2 |
| Amber 5 b | D65 | 10° | 81,0 | 13.1 | 50,9 |
| Amber 5 c | D65 | 10° | 60,8 | 13,2 | 48,8 |
| Amber 5 d | D65 | 10° | 62,6 | 13,3 | 55,2 |
| Amber 5 e | D85 | 10° | 57,0 | 24,9 | 70,3 |
| Amber 5 f | D65 | 10° | 56,8 | 24,2 | 66,7 |
| Amber 5 g | D65 | 10° | 59,0 | 23,3 | 73,1 |

| | **Farbstoff / 100g PET [g]** | | | | |
|---|---|---|---|---|---|
| **PET transparent 2mm** | **(7)** Vergleich | **(8)** Vergleich | **(14)** Vergleich | **(5)** | **Gesamt Farbstoff** |
| **(7)** (Vergleich) | 0,05000 | | | | 0,050000 |
| **(8)** (Vergleich) | | 0,05000 | | | 0,050000 |
| **(14)** (Vergleich) | | | 0,05000 | | 0,050000 |
| **(5)** (Vergleich) | | | | 0,05000 | 0,050000 |

| | **Farbstoff / 100g PET [g]** | | | | | | |
|---|---|---|---|---|---|---|---|
| **PET transparent 2mm** | **(7)** Vergleich | **(5)+(4) 50,8/ 49,2** | **(13)** | **(10)** | **(11)** | **(12)** | **Gesamt Farbstoff** |
| | | | | | | | |
| Amber 1 a | 0,01210 | | 0,00315 | 0,00475 | | | 0,020000 |
| (Vergleich) | | | | | | | |
| Amber 1 b | | 0,00370 | 0,00314 | 0,00492 | | | 0,011760 |
| Amber 1 c | | 0,00350 | 0,00310 | | 0,01320 | | 0,019800 |
| Amber 1 d | | 0,00370 | 0,00310 | | | 0,00470 | 0,011500 |
| | | | | | | | 0,000000 |
| Amber 2 a | 0,01230 | | 0,00460 | 0,00310 | | | 0,020000 |
| (Vergleich) | | | | | | | |
| Amber 2 b | | 0,00370 | 0,00400 | 0,00290 | | | 0,010601 |
| Amber 2 c | | 0,00340 | 0,00360 | | 0,00830 | | 0.015300 |
| Amber 2 d | | 0,00370 | 0,00410 | | | 0,00300 | 0,010800 |
| | | | | | | | |
| Amber 3 a | 0,00605 | | 0,00157 | 0,00238 | | | 0,010000 |
| (Vergleich) | | | | | | | |
| Amber 3 b | | 0,00019 | 0,00016 | 0,00025 | | | 0,000588 |
| Amber 3 c | 0,01210 | | 0,00315 | 0,00475 | | | 0,020000 |
| (Vergleich) | | | | | | | |
| Amber 3 d | | 0,00370 | 0,00314 | 0,00492 | | | 0,011760 |
| Amber 3 e | 0,03024 | | 0,00787 | 0,01189 | | | 0,050000 |
| (Vergleich) | | | | | | | |
| Amber 3 f | | 0,00925 | 0,00785 | 0,01230 | | | 0,029400 |
| | | | | | | | |
| Amber 4 a | 0,00615 | | 0,00229 | 0,00156 | | | 0,010000 |
| (Vergleich) | | | | | | | |
| Amber 4 b | | 0,00185 | 0,00200 | 0,00145 | | | 0,005300 |
| Amber 4 c | 0,01229 | | 0,00459 | 0,00312 | | | 0,020000 |
| (Vergleich) | | | | | | | |
| Amber 4 d | | 0,00370 | 0,00400 | 0,00290 | | | 0,010600 |
| Amber 4 e | 0,03070 | | 0,01150 | 0,00780 | | | 0,050000 |
| (Vergleich) | | | | | | | |
| Amber 4 f | | 0,00925 | 0,01000 | 0,00725 | | | 0,026500 |

| | **Farbstoff / 100g PET [g]** | | | | | | |
|---|---|---|---|---|---|---|---|
| **PET transparent 2mm** | **(7)** Vergleich | **(5)** | **(1)** | **(2)** | **(3)** | **(4)** | **Gesamt Farbstoff** |
| | | 0,02500 | | | | | 0,025000 |
| (5) (Vergleich) | | | | | | | |
| (1) | | | 0,02500 | | | | 0,025000 |
| (2) | | | | 0,02500 | | | 0,025000 |
| (3) | | | | | 0,02500 | | 0,025000 |
| | | | | | | | |
| Rot 1 a | | 0,02540 | | | | 0,02460 | 0,050000 |
| Rot 1 b | | | 0,02700 | | | 0,00550 | 0,032500 |
| Rot 1 c | | | 0,01350 | | | 0,00280 | 0,016300 |
| | | | | | | | |
| | 0,05000 | | | | | | 0,050000 |
| (7) (Vergleich) | | | | | | | |
| Rot 2 a | | | 0,03000 | | | 0,01800 | 0,048000 |
| Rot 2 b | | | | 0,03000 | | 0,01800 | 0,048000 |
| Rot 2 c | | | | | 0,03000 | 0,01800 | 0,048000 |
| Rot 2 d | | | 0,03000 | | | 0,00600 | 0,036000 |
| Rot 2 e | | | | 0,03000 | | 0,00600 | 0,036000 |
| Rot 2 f | | | | | 0,03000 | 0,00600 | 0,036000 |

| | **Farbstoff / 100g PET [g]** | | | | | | | | |
|---|---|---|---|---|---|---|---|---|---|
| **PET transparent 2mm** | **(7)** Vergleich | (**1)** | **(2)** | **(3)** | **(13)** | **(4)** | **(6)** | **(10)** | **Gesamt Farbstoff** |
| | | | | | | | | | |
| Amber 5 a | 0,01210 | | | | 0,00315 | | | 0.00475 | 0,020000 |
| (Vergleich) | | | | | | | | | |
| Amber 5 b | | 0,00570 | | | 0,00320 | | 0,00340 | 0,00480 | 0,017100 |
| Amber 5 c | | | 0,00530 | | 0,00320 | | 0,00310 | 0,00480 | 0,016400 |
| Amber 5 d | | | | 0,00600 | 0,00320 | | 0,00360 | 0,00480 | 0,017600 |
| Amber 5 e | | 0,00530 | | | 0,00320 | 0,00320 | | 0,00480 | 0,016500 |
| Amber 5 f | | | 0,00500 | | 0,00320 | 0,00300 | | 0,00480 | 0,016000 |
| Amber 5 g | | | | 0,00550 | 0,00320 | 0,00330 | | 0,00480 | 0,016800 |

### Lösungen der Mischungen

Die maximale Löslichkeit der Farbstoffe wurde in Benzoesäuremethylester ermittelt. Zu diesen gesättigten Lösungen wurde schließlich die angegebene Menge des orangen Farbstoffs (4) (= Formel (L) mit R¹⁰ = COOCH₃ und R¹¹ = H) hinzugegeben. Dieser Farbstoff ging dabei ebenfalls komplett in Lösung.

### Maximale Löslichkeit der Farbstoffe in Benzoesäuremethylester und spektrale Daten

| Farbstoff | Löslichkeit g/100ml | Absorptionsmaximum | E11 |
|---|---|---|---|
| (7) | 0,107 | 496 nm | 0,17 |
| (5) | 0,37 | 536 nm | 3,2 |
| (15) | 0,57 | 523, 558 nm | 1,8 |
| (1) | 0,55 | 523, 558 nm | 1,75 |
| (2) | 0,05 | 524, 560 nm | 0,06 |
| | | | |
| (4) | 1,64 | 471 nm | 19 |

E11 = Extinktion von 1 g dieser Lösung in 100 ml N-Methylpyrrolidon gemessen in einer 1-cm-Küvette

### Lösungen der Rotmischungen in Benzoesäuremethylester und spektrale Daten

| Farbstoff 1 | Menge g/100ml | Farbstoff 2 | Menge g/100ml | Absorptionsmaximum | E11 |
|---|---|---|---|---|---|
| (7) | 0,107 | - | - | 496 nm | 0,17 |
| (5) | 0,37 | (4) | 0,346 | 490 nm | 5 |
| (15) | 0,57 | (4) | 0,6 | 477, 557 (sh) nm | 7,45 |
| (15) | 0,57 | (4) | 0,383 | 481, 517 (sh), 558 nm | 5 |
| (15) | 0,57 | (4) | 0,15 | 489, 519, 559 nm | 2,6 |
| (1) | 0,55 | (4) | 0,583 | 475, 556 (sh) nm | 6,65 |
| (1) | 0,55 | (4) | 0,138 | 489, 519, 558 nm | 2,8 |
| (2) | 0,05 | (4) | 0,07 | 473, 557 (sh) nm | 0,7 |
| (2) | 0,05 | (4) | 0,035 | 492, 521, 560 nm | 0,66 |

E11 = Extinktion von 1 g dieser Lösung in 100 ml N-Methylpyrrolidon gemessen in einer 1-cm-Küvette
(sh) = Schulter

Die spektralen Daten der Lösungen wurden untersucht. Es zeigt sich, dass bezogen auf die mit einer gesättigten Lösung des Vergleichsfarbstoffs (7) 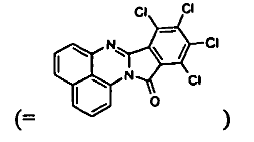 maximal erzielbare Farbstärke (= Extinktion) die Lösungen der erfindungsgemäßen Rotfarbstoffe und Rotmischungen bis über 80 mal farbstärker sind.

### Extrusionsversuche

Die Extrusionsversuche erfolgten mit PET als Kunststoff und
a) mit dem Vergleichs-Farbstoff (7)
b) mit einer 50/50 Mischung aus dem roten Farbstoff (5) und dem orangen Farbstoff (4).

Für die Versuche wurde ein Extruder der Fa Leistritz vom Typ ZSE 18 HP benutzt.
Die Drehzahl wurde konstant auf 600 Upm und die Manteltemperatur des Zylinders auf 245°C gehalten.
Die Dosierung der PET/Farbstoffmischung erfolgte über eine Dosiereinheit vom Typ Brabender DS28 mit Single-Schnecke mit einer Drehzahl von 25 Upm Es wurde der PET-Typ Voridian 9921 W benutzt.

Wie aus der folgenden Tabelle entnommen werden kann, steigt der Massedruck bei Verwendung von Farbstoff (7) schon deutlich von 12 auf 16 bar an, wenn man die Konzentration des Farbstoffes von 20 auf 30 % erhöht. Dagegen ist der Massedruck bei Verwendung einer Farbstoffmischung aus gleiche Teilen Farbstoff (5) und (4) und einem Gesamtfarbstoffgehalt von 30 % mit 4 bar deutlich geringer und steigt auch überraschenderweise bei einer Gesamtfarbstoffkonzentration bis 45% nicht weiter an. Es ist daher eine deutlich höhere Beladung des Batches möglich.

| | Druck (bar) | |
|---|---|---|
| Farbstoffgehalt | Mischung Farbstoff (5) und (4) 1:1 | Farbstoff (7) |
| 20 | 7 | 12 |
| 30 | 4 | 16 |
| 40 | 4 | - |
| 45 | 4 | - |

## Patentansprüche

1. Mischungen, enthaltend wenigstens einen Rotfarbstoff der Formel (I) oder (C) 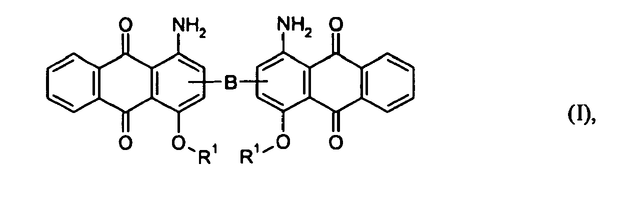 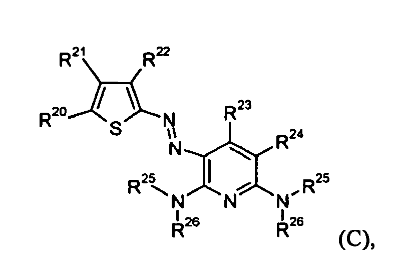 worin
R¹ für Wasserstoff, gegebenenfalls substituiertes C₁-C₈-Alkyl oder gegebenenfalls substituiertes C₃-C₈-Cycloalkyl steht,
B für eine Brücke der Formeln -O-B¹-O- oder -H₂-B²-CH₂- steht,
B¹ für eine Brücke der Formel 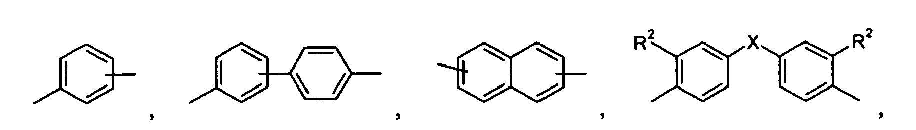 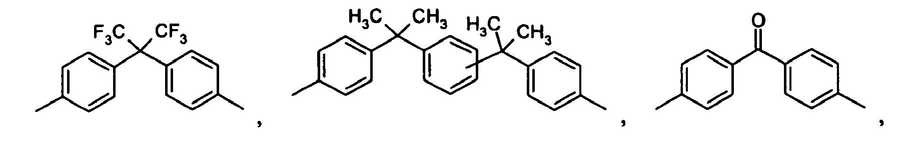 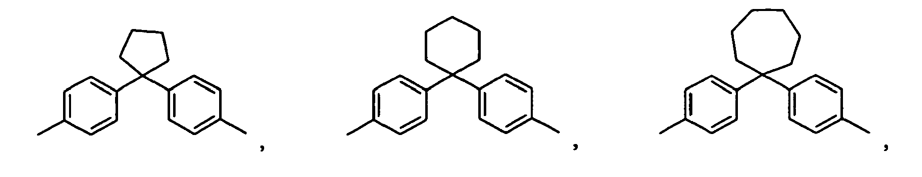 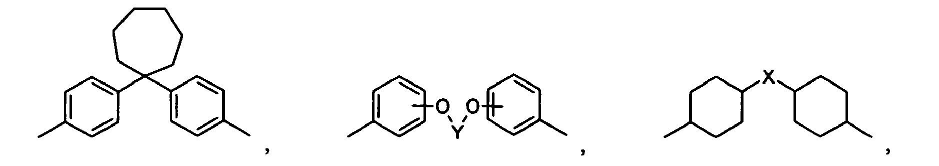 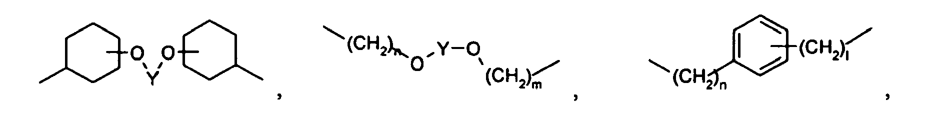 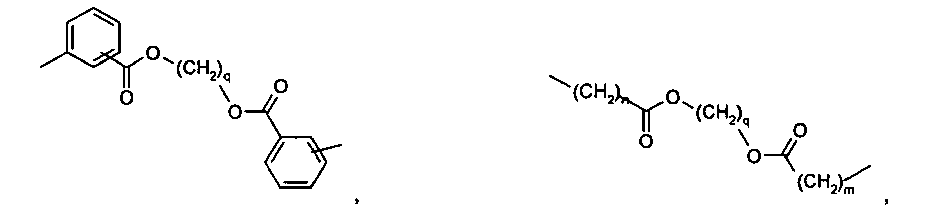 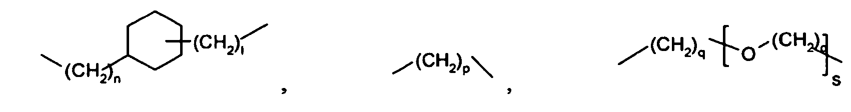 oder 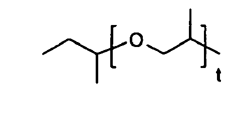 steht,
B² für eine Brücke der Formel 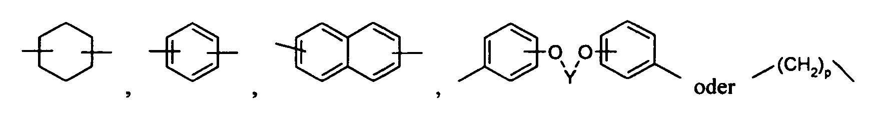 steht,
R² für Wasserstoff, Methyl, Ethyl, Methoxy oder Fluor steht,
X für eine Brücke der Formel 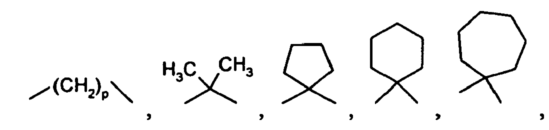 O, S, SO₂ oder CO
steht,
Y für eine Brücke der Formel 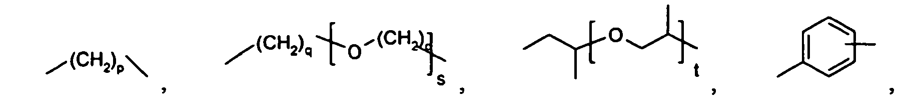 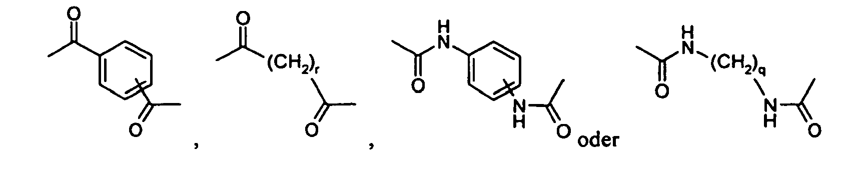 steht,
1 für eine ganze Zahl von 0 bis 8 steht,
m, n, p und r unabhängig voneinander für eine ganze Zahl von 1 bis 8 stehen,
q für eine ganze Zahl von 2 bis 8 steht und
s und t unabhängig voneinander für eine ganze Zahl von 1 bis 4 stehen,
R²⁰ und R²² unabhängig voneinander für Cyano oder gegebenenfalls substituiertes C₁-C₈- Alkoxycarbonyl stehen,
R²¹ für Wasserstoff oder gegebenenfalls substituiertes C₁-C₃-Alkyl steht,
R²³ für Wasserstoff, gegebenenfalls substituiertes C₁-C₈-Alkyl oder gegebenenfalls substituiertes C₁-C₈-Alkoxycarbonyl steht,
R²⁴ für Cyano, gegebenenfalls substituiertes C₁-C₈-Alkylsulfonyl oder gegebenenfalls substituiertes C₁-C₈-Alkoxycarbonyl steht,
R²⁵ für gegebenenfalls substituiertes C₁-C₈-Alkyl, gegebenenfalls substituiertes C₃- C₇-Cycloalkyl, gegebenenfalls substituiertes C₆-C₁₀-Aryl steht,
R²⁶ für Wasserstoff oder unabhängig von R²⁵ die Bedeutung von R²⁵ hat oder
NR^{2s}R²⁶ für Pyrrolidino, Piperidino oder Morpholino steht,
und wenigstens einen Orangefarbstoff der Formel (L), (LI), (LII), (LIII), (LIV) oder (LV) 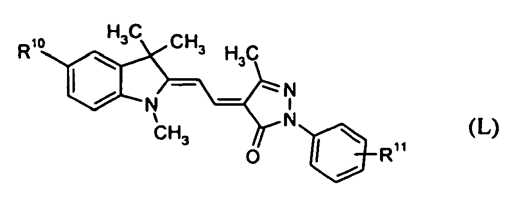 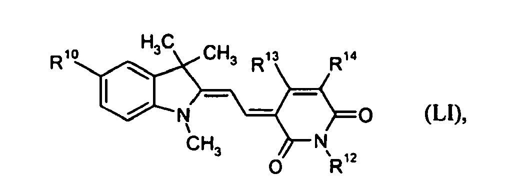 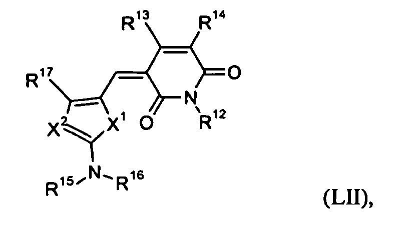 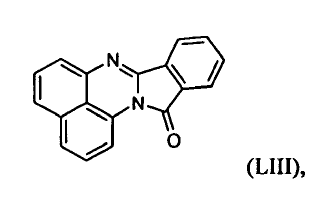 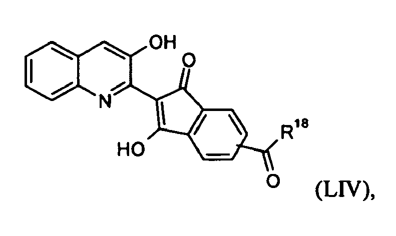 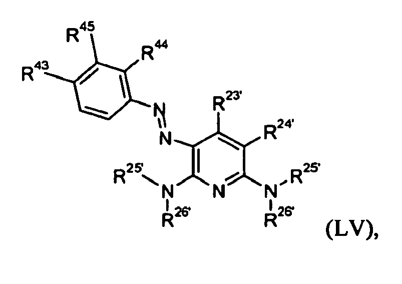 worin
R¹⁰ für Wasserstoff, gegebenenfalls substituiertes C₁-C₈-Alkyl, gegebenenfalls substituiertes C₁-C₈-Alkoxy, gegebenenfalls substituiertes C₁-C₈-Alkylsulfonyl, gegebenenfalls substituiertes C₁-C₈-Alkoxycarbonyl, Nitro, Cyano oder Fluor, steht,
R¹¹ für Wasserstoff, gegebenenfalls substituiertes C₁-C₈-Alkyl, gegebenenfalls substituiertes C₁-C₈-Alkoxy, gegebenenfalls substituiertes C₁-C₈- Alkoxycarbonyl, Nitro, Cyano oder Fluor steht,
R¹² für Wasserstoff, gegebenenfalls substituiertes C₁-C₈-Alkyl oder gegebenenfalls substituiertes C₃-C₈-Cycloalkyl steht,
R¹³ für Wasserstoff, gegebenenfalls substituiertes C₁-C₈-Alkyl oder gegebenenfalls substituiertes C₁-C₈-Alkoxycarbonyl steht,
R¹⁴ für Cyano, gegebenenfalls substituiertes C₁-C₈-Alkylsulfonyl oder gegebenenfalls substituiertes C₁-C₈-Alkoxycarbonyl steht,
X¹ für O oder S steht,
X² für N oder C-CN steht,
R¹⁵ und R¹⁶ unabhängig voneinander für gegebenenfalls substituiertes C₁-C₈-Alkyl, gegebenenfalls substituiertes C₃-C₈-Cycloalkyl oder gegebenenfalls substituiertes C₆- C₁₀-Aryl stehen oder
NR¹⁵R¹⁶ für Pyrrolidino, Piperidino oder Morpholino steht,
R¹⁷ für Wasserstoff oder C₁-C₈-Alkyl steht,
R¹⁸ für gegebenenfalls substituiertes C₆-C₁₀-Aryl steht,
R^{23'} für Wasserstoff, gegebenenfalls substituiertes C₁-C₈-Alkyl oder gegebenenfalls substituiertes C₁-C₈-Alkoxycarbonyl steht,
R^{24'} für Cyano, gegebenenfalls substituiertes C₁-C₈-Alkylsulfonyl oder gegebenenfalls substituiertes C₁-C₈-Alkoxycarbonyl steht,
R^{25'} für gegebenenfalls substituiertes C₁-C₈-Alkyl, gegebenenfalls substituiertes C₃- C₇-Cycloalkyl, gegebenenfalls substituiertes C₆-C₁₀-Aryl steht,
R^{26'} für Wasserstoff oder unabhängig von R^{25'} die Bedeutung von R^{25'} hat oder
NR^{25'}R^{26'} für Pyrrolidino, Piperidino oder Morpholino steht,
R⁴³ und R⁴⁴ unabhängig voneinander für Wasserstoff, gegebenenfalls substituiertes C₁-C₄- Alkyl, insbesondere durch ein oder mehrere Fluor substituiertes C₁-C₄-Alkyl, Nitro, Cyano, C₁-C₄-Alkoxycarbonyl stehen und
R⁴⁵ für Wasserstoff, Methyl, Fluor oder Chlor steht.

2. Mischung gemäß Anspruch 1, enthaltend einen Rotfarbstoff der Formel (I) und wenigstens einen Orangefarbstoff der Formel (L), (LI), (LII), (LIII), (LIV) oder (LV), insbesondere der Formel (L).

3. Mischung gemäß Anspruch 1, enthaltend einen Rotfarbstoff der Formel (C) und wenigstens einen Orangefarbstoff der Formel (L), (LI), (LII), (LIII), (LIV) oder (LV), insbesondere der Formel (L).

4. Mischung gemäß Anspruch 1, enthaltend einen Rotfarbstoff der Formel 1
worin
R¹ für Wasserstoff oder Methyl steht,
B¹ für eine Brücke der Formel 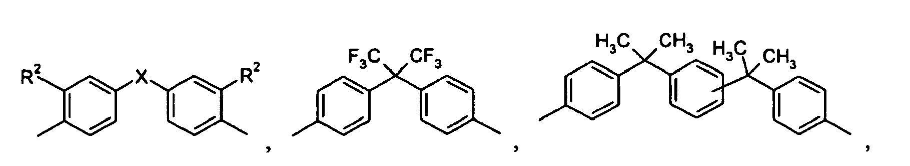 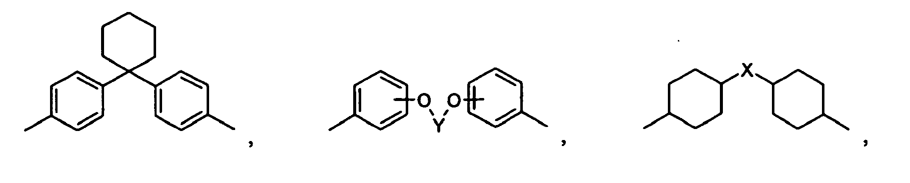 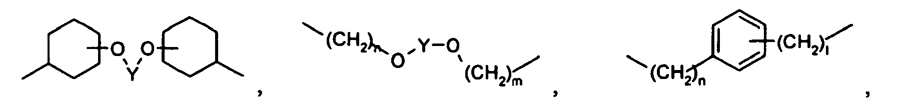 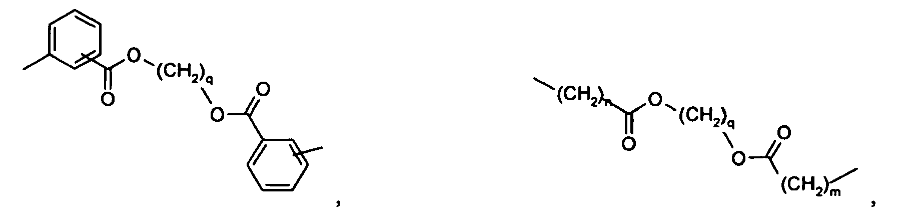 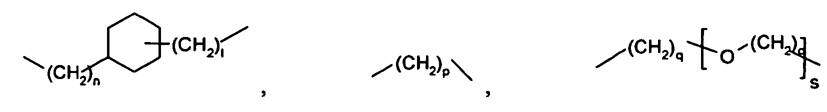 oder 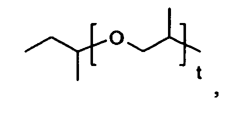 steht,
R² für Methyl oder Chlor steht,
X für eine Brücke der Formel 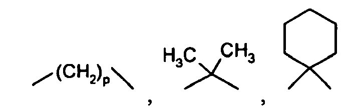 oder O
steht,
Y für eine Brücke der Formel 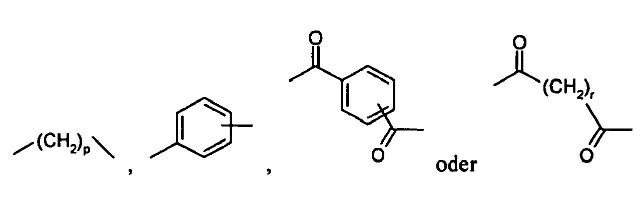 steht,
l für eine ganze Zahl von 0 bis 4 steht,
m, n und p unabhängig voneinander für eine ganze Zahl von 1 bis 4 stehen,
q und r unabhängig voneinander für eine ganze Zahl von 2 bis 6 steht,
s und t unabhängig voneinander für eine ganze Zahl von 1 bis 2 stehen und
die unbestimmten Ringpositionen 1,2-, 1,3- oder 1,4- bedeuten.

5. Mischung gemäß Anspruch 1, enthaltend einen Rotfarbstoff der Formel (I),
worin
R¹ für Wasserstoff oder Methyl steht,
B² für eine Brücke der Formel 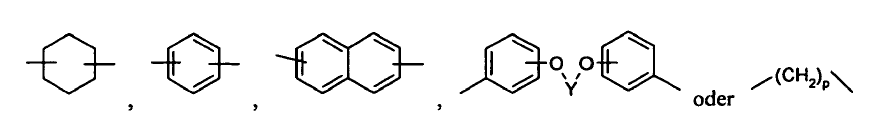 steht,
Y für eine Brücke der Formel 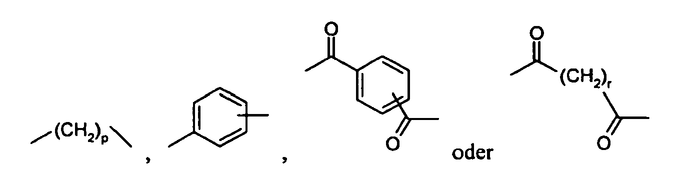 steht,
p für eine ganze Zahl von 1 bis 6 steht und
r für eine ganze Zahl von 2 bis 6 steht und
die unbestimmten Ringpositionen bei Benzol- oder Cyclohexanringen 1,2-, 1,3- oder 1,4-und beim Naphthalinring 2,6- und 2,7- bedeuten.

6. Mischung gemäß Anspruch 1, enthaltend einen Rotfarbstoff der Formel (C),
worin
R²⁰ für Cyano, Methoxycarbonyl oder Ethoxycarbonyl, besonders bevorzugt für Cyano oder Methoxycarbonyl, ganz besonders bevorzugt für Methoxycarbonyl,
R²² für Cyano,
R²¹ für Wasserstoff oder Methyl, besonders bevorzugt für Methyl,
R²³ für Methyl oder Methoxycarbonyl, besonders bevorzugt für Methyl,
R²⁴ für Cyano, Methansulfonyl, Methoxycarbonyl oder Ethoxycarbonyl, besonders bevorzugt für Cyano,
R²⁵ für Methyl, Ethyl, Propyl, Butyl, Methoxyethyl, Methoxypropyl, Cyanethyl, Benzyl, Cyclohexyl, Phenyl oder Tolyl, besonders bevorzugt für Ethyl, Propyl, Butyl, Methoxyethyl, Methoxypropyl, Benzyl, Cyclohexyl oder Phenyl, ganz besonders bevorzugt für Propyl, Butyl oder Methoxypropyl,
R²⁶ für Wasserstoff oder unabhängig von R²⁵ die Bedeutung von R²⁵, oder
NR²⁵R²⁶ für Pyrrolidino, Piperidino oder Morpholino,
steht, insbesondere wenn
R²⁰ für COOCH₃, R²¹ und R²³ für CH₃, R²² und R²⁴ für CN, R²⁵ für -CH₂CH₂CH₂OCH₃ und R²⁶ für H steht
und einen Orangefarbstoff der Formel (L), insbesondere worin
R¹⁰ für COOCH₃ und R¹¹ für H steht.

7. Mischung gemäß Anspruch 1, enthaltend weiterhin wenigstens
a) einen gelben Farbstoff, vorzugsweise mit einem λₘₐₓ von 420 bis 460 nm und wenigstens einen grünen Farbstoff, vorzugsweise mit einem λₘₐₓ von 610 bis 700 nm oder
b) einen gelben Farbstoff, vorzugsweise mit einem λₘₐₓ von 420 bis 460 nm und wenigstens einen blauen Farbstoff, vorzugsweise mit einem λₘₐₓ von 570 bis 640 nm.

8. Mischungen gemäß Anspruch 1, **dadurch gekennzeichnet, dass** sie weiterhin
a) wenigstens einen Gelbfarbstoff der Formel (CCI), (CCII), (CCIII), (CCIV) oder (CCV) 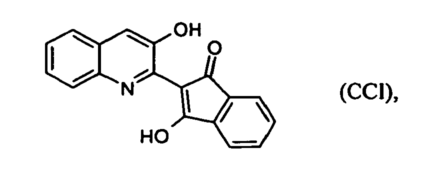 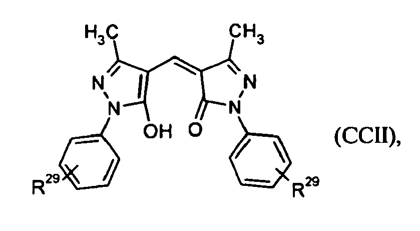 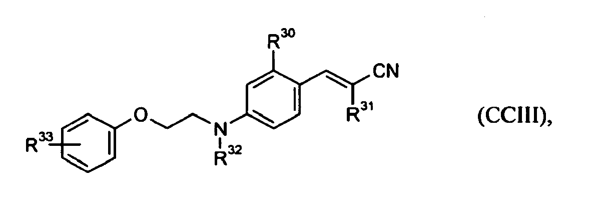 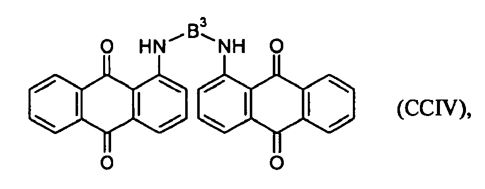 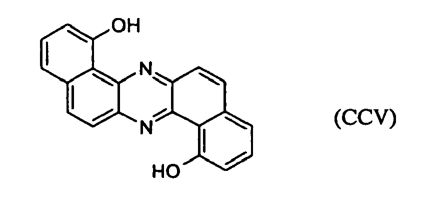 worin
R²⁹ für einen oder mehrere Reste mit der Bedeutung Wasserstoff, gegebenenfalls substituiertes C₁-C₈-Alkyl, gegebenenfalls substituiertes C₁-C₈-Alkoxy, Fluor, Cyano, gegebenenfalls substituiertes C₃-C₇-Cycloalkyl oder gegebenenfalls substituiertes C₆-C₁₀-Aryl steht,
R³⁰ für Wasserstoff, Methyl, Ethyl oder Methoxy steht,
R³¹ für Cyano, C₁-C₈-Alkoxycarbonyl oder C₁-C₈-Alkansulfonyl steht,
R³² für Wasserstoff, gegebenenfalls substituiertes C₁-C₈-Alkyl oder gegebenenfalls substituiertes C₃-C₇-Cycloalkyl steht,
R³³ für Wasserstoff, gegebenenfalls substituiertes C₁-C₈-Alkyl, gegebenenfalls substituiertes C₁-C₈-Alkoxy, gegebenenfalls substituiertes C₃-C₇-Cycloalkyl oder gegebenenfalls substituiertes C₆-C₁₀-Aryl steht, und
B³ für eine Brücke der Formel 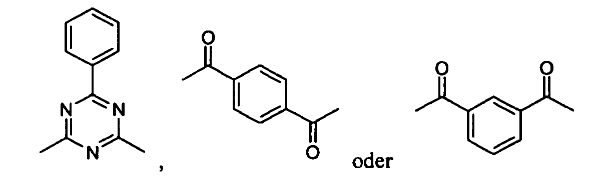 steht
und wenigstens einen Grünfarbstoff der Formel (CCLI) enthält 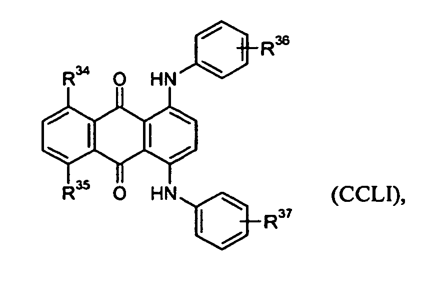 worin
R³⁴ und R³⁵ unabhängig voneinander für Wasserstoff, Hydroxy oder C₁-C₄-Alkoxy stehen,
R³⁶ und R³⁷ unabhängig voneinander für Wasserstoff, gegebenenfalls substituiertes C₁-C₈- Alkyl, gegebenenfalls substituiertes C₁-C₈-Alkoxy, Fluor, gegebenenfalls substituiertes C₃-C₇-Cycloalkyl oder gegebenenfalls substituiertes C₆-C₁₀-Aryl stehen,
oder
b) wenigstens einen Gelbfarbstoff der Formel (CCI), (CCII), (CCIII), (CCIV) oder (CCV) und wenigstens einen Blaufarbstoff der Formel (CCCI) enthält 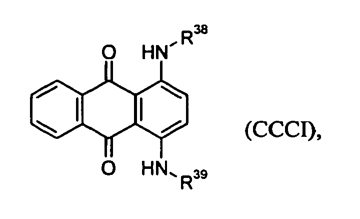 worin
R³⁸ und R³⁹ unabhängig voneinander für gegebenenfalls substituiertes C₁-C₈-Alkyl, gegebenenfalls substituiertes C₃-C₇-Cycloalkyl oder gegebenenfalls substituiertes C₆-C₁₀-Aryl stehen.

9. Masterbatch, enthaltend 15 - 80, insbesondere 15 - 70 Gew.% einer Mischung gemäß Anspruch 1 und einen Carrier.

10. Verwendung einer Mischung gemäß Anspruch 1 zum Massefärben von Kunststoffen.

11. Verfahren zum Massefärben von Kunststoffen, **dadurch gekennzeichnet, dass** man die Mischung gemäß Anspruch 1, vorzugsweise in Form ihres Masterbatchs in die geschmolzene Kunststoffmasse einarbeitet oder bereits den Ausgangskomponenten zur Herstellung des Kunststoffs vor der Polymerisation zusetzt.

12. Kunststoffe enthaltend eine Mischung gemäß Anspruch 1.

13. Masterbatch, enthaltend 15 - 90 Gew.%, insbesondere 15 - 70 Gew.% einer Verbindung der Formel I 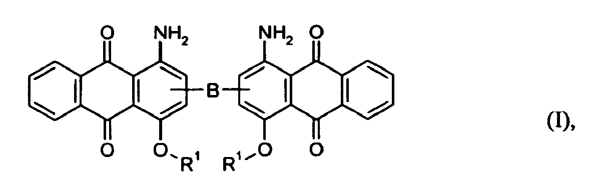 insbesondere der Formel (Ia) 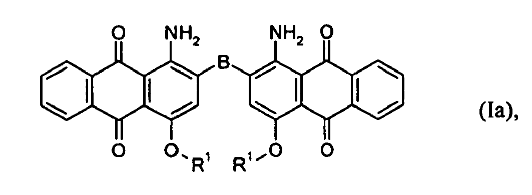 worin
-B- für -O-B¹-O- oder -CH₂-B²-CH₂- steht,
R¹ für Wasserstoff, gegebenenfalls substituiertes C₁-C₈-Alkyl oder gegebenenfalls substituiertes C₃-C₈-Cycloalkyl steht,
B¹ für eine Brücke der Formel 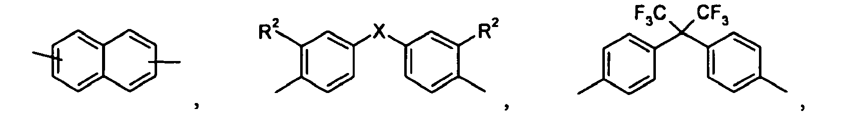 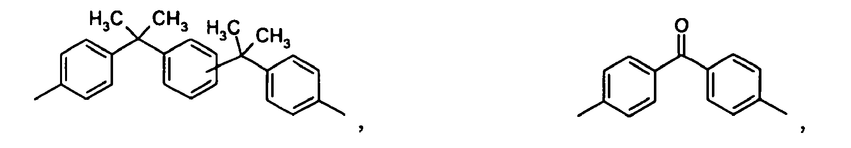 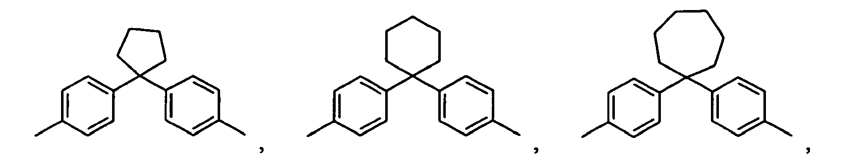 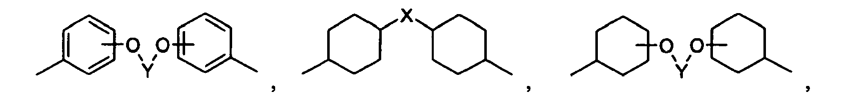 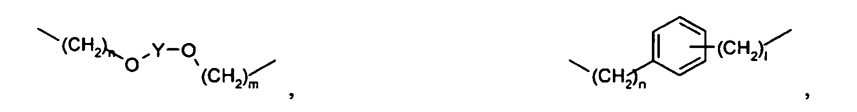 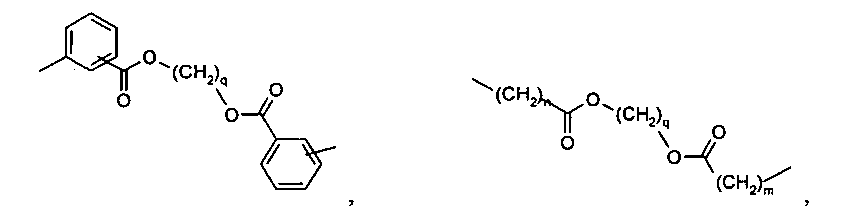 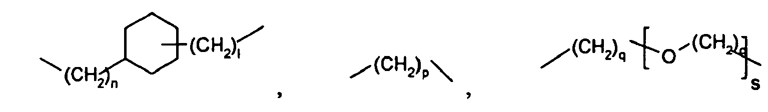 oder 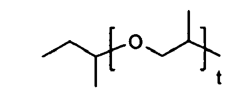 steht,
B² für eine Brücke der Formel 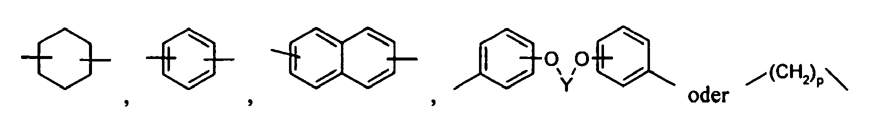 steht,
R² für Methyl, Ethyl, Methoxy oder Fluor steht,
X für eine Brücke der Formel 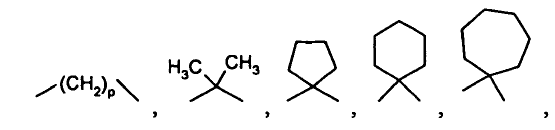 O, S, SO₂ oder CO
steht,
Y für eine Brücke der Formel 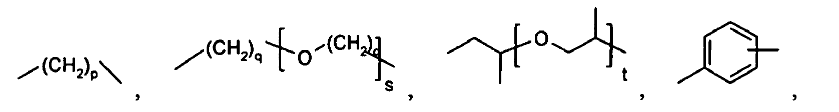 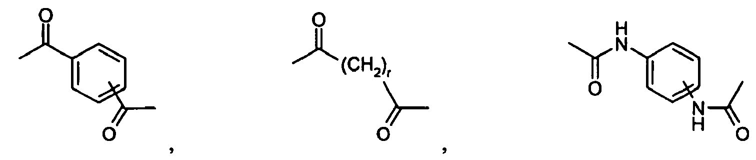 oder 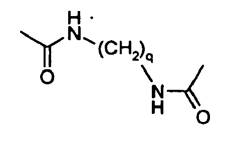 steht,
l für eine ganze Zahl von 0 bis 8 steht,
m, n, p und r unabhängig voneinander für eine ganze Zahl von 1 bis 8 stehen,
q für eine ganze Zahl von 2 bis 8 steht und
s und t unabhängig voneinander für eine ganze Zahl von 1 bis 4 stehen.
und einen Carrier.

14. Verwendung von Verbindungen der Formeln I 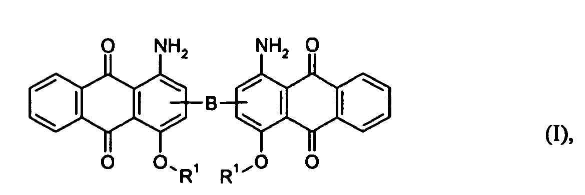 insbesondere der Formel (Ia) 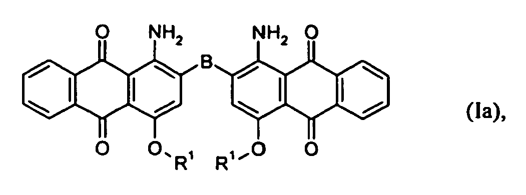 worin
-B- für -O-B¹-O- oder -CH₂-B²-CH₂- steht,
R¹ für Wasserstoff, gegebenenfalls substituiertes C₁-C₈-Alkyl oder gegebenenfalls substituiertes C₃-C₈-Cycloalkyl steht,
B¹ für eine Brücke der Formel 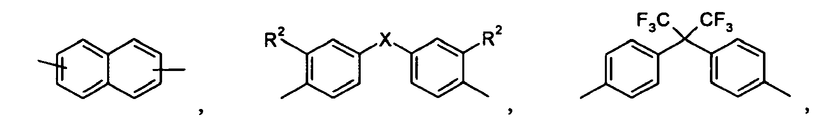 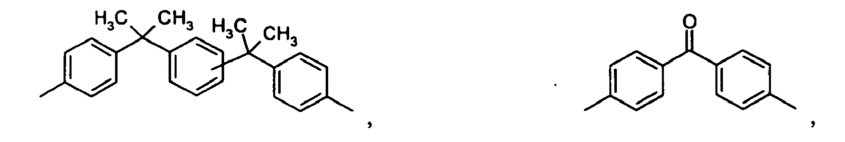 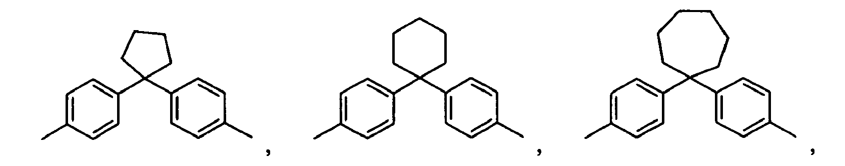 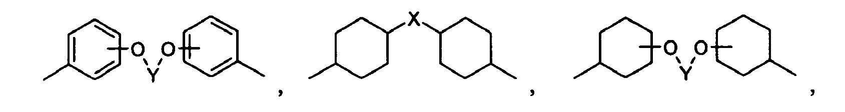 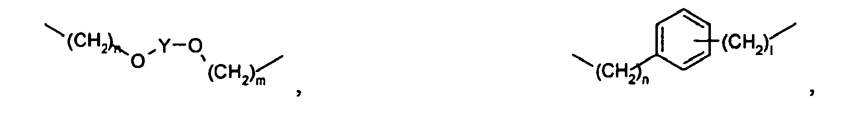 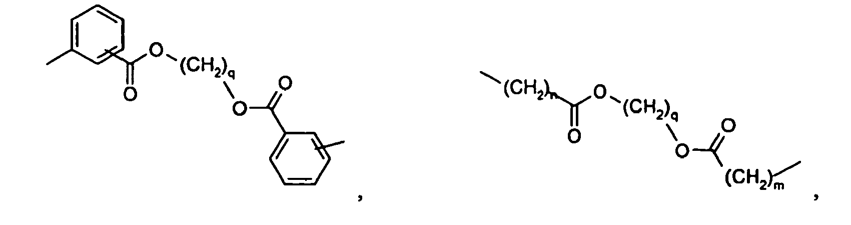 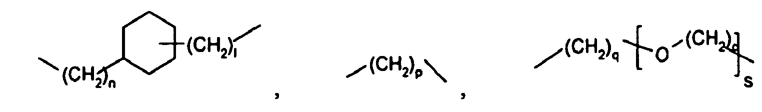 oder 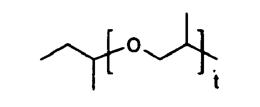 steht,
B² für eine Brücke der Formel 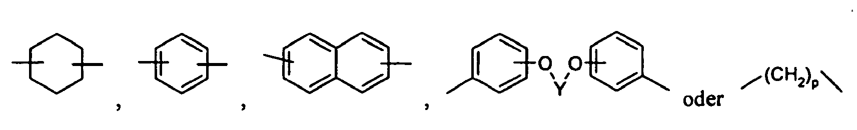 steht,
R² für Methyl, Ethyl, Methoxy oder Fluor steht,
X für eine Brücke der Formel 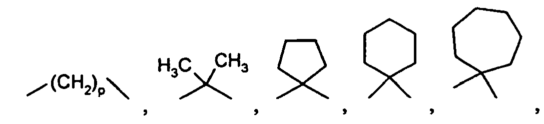 O, S, SO₂ oder CO
steht,
Y für eine Brücke der Formel 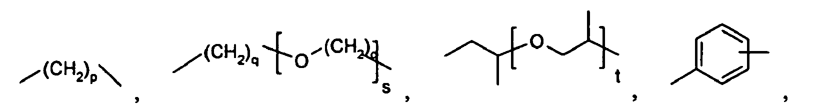 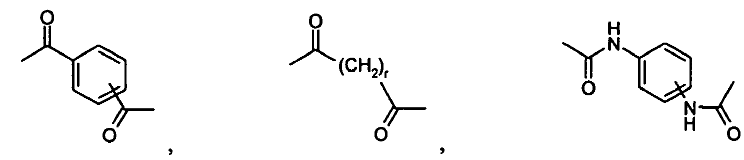 oder 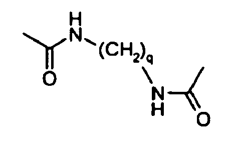 steht,
l für eine ganze Zahl von 0 bis 8 steht,
m, n, p und r unabhängig voneinander für eine ganze Zahl von 1 bis 8 stehen,
q für eine ganze Zahl von 2 bis 8 steht und
s und t unabhängig voneinander für eine ganze Zahl von 1 bis 4 stehen.
zum Massefärben von Kunststoffen

15. Verfahren zum Massefärben von Kunststoffen, **dadurch gekennzeichnet, dass** man eine Verbindung der Formel I 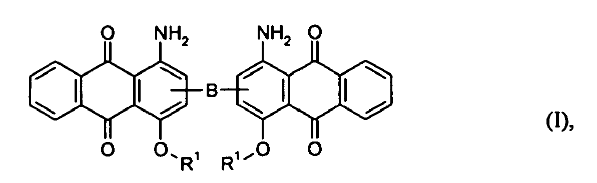 insbesondere der Formel (Ia) 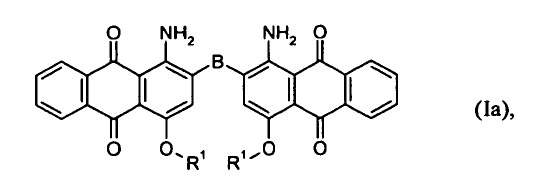 worin
-B- für -O-B¹-O- oder -CH₂-B²-CH₂- steht,
R¹ für Wasserstoff, gegebenenfalls substituiertes C₁-C₈-Alkyl oder gegebenenfalls substituiertes C₃-C₈-Cycloalkyl steht,
B¹ für eine Brücke der Formel 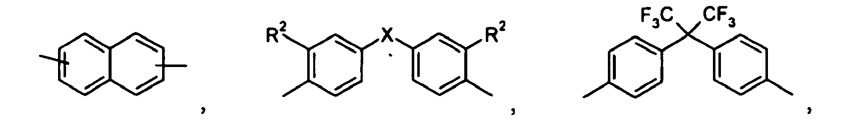 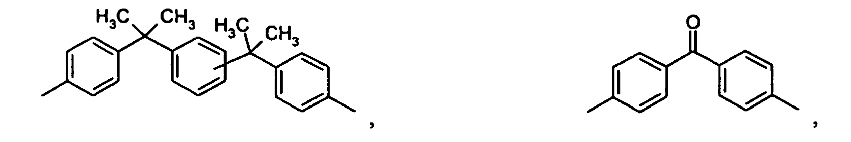 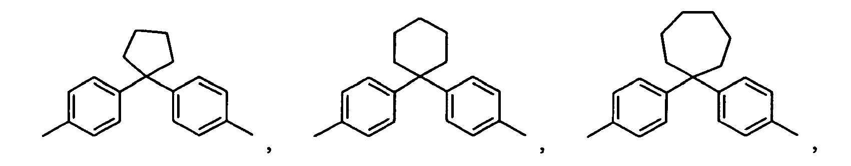 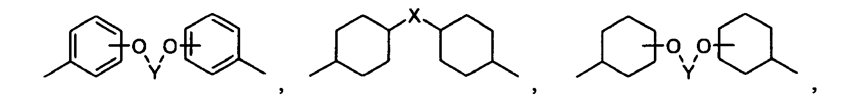 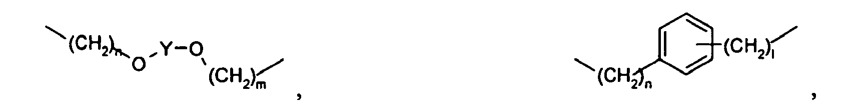 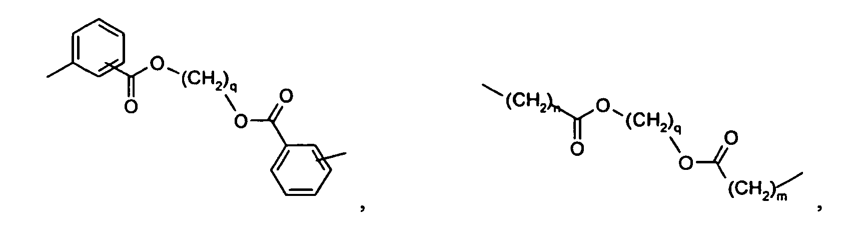 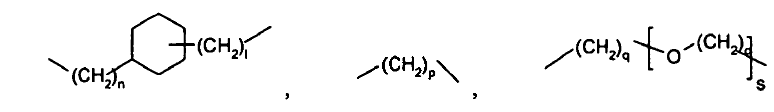 oder 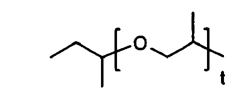 steht,
B² für eine Brücke der Formel 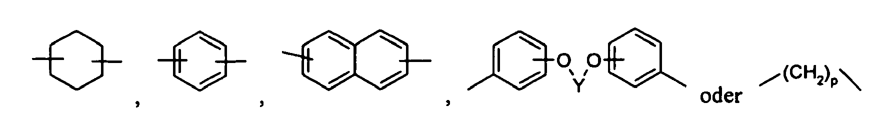 steht,
R² für Methyl, Ethyl, Methoxy oder Fluor steht,
X für eine Brücke der Formel 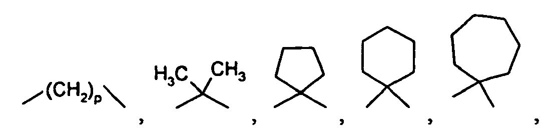 O, S, SO₂ oder CO
steht,
Y für eine Brücke der Formel 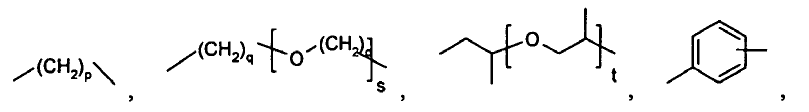 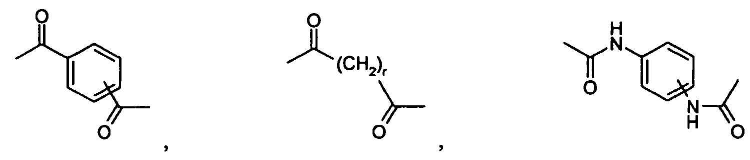 oder 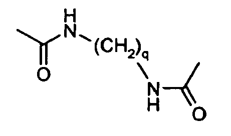 steht,
l für eine ganze Zahl von 0 bis 8 steht,
m, n, p und r unabhängig voneinander für eine ganze Zahl von 1 bis 8 stehen,
q für eine ganze Zahl von 2 bis 8 steht und
s und t unabhängig voneinander für eine ganze Zahl von 1 bis 4 stehen.
, vorzugsweise in Form ihres Masterbatchs in die geschmolzene Kunststoffmasse einarbeitet oder bereits den Ausgangskomponenten zur Herstellung des Kunststoffs vor der Polymerisation zusetzt.

16. Kunststoffe enthaltend eine Verbindung der Formel I 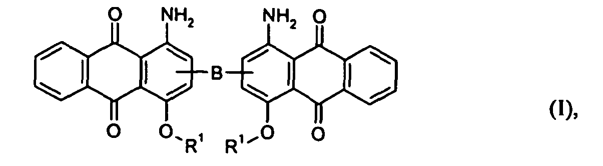 insbesondere der Formel (Ia) 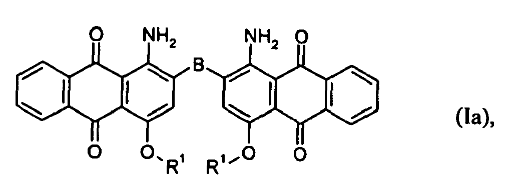 worin
-B- für -O-B¹-O- oder -CH₂-B²-CH₂- steht,
R¹ für Wasserstoff, gegebenenfalls substituiertes C₁-C₈-Alkyl oder gegebenenfalls substituiertes C₃-C₈-Cycloalkyl steht,
B¹ für eine Brücke der Formel 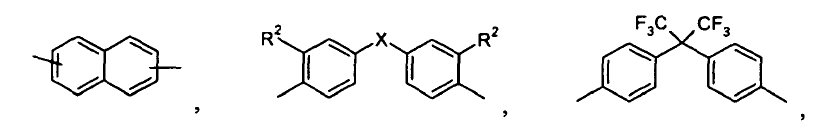 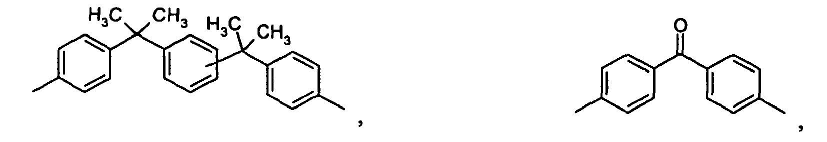 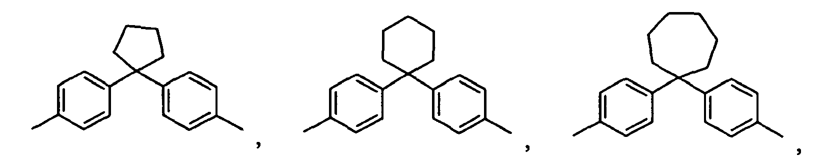 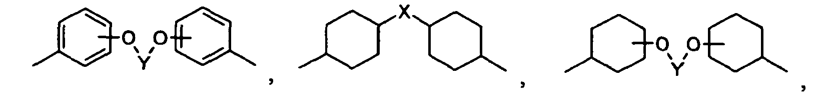 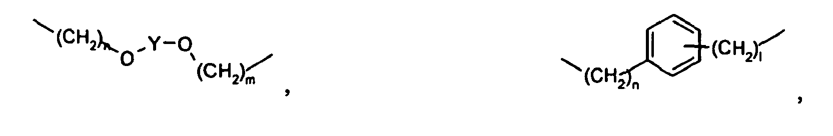 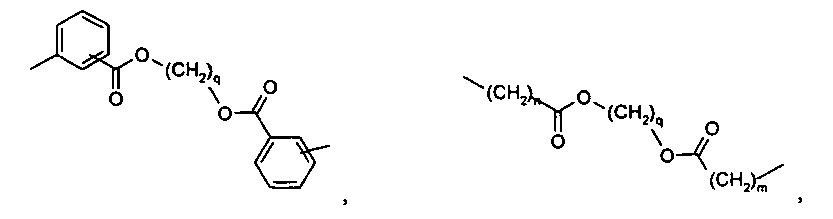 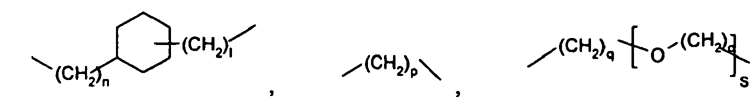 oder 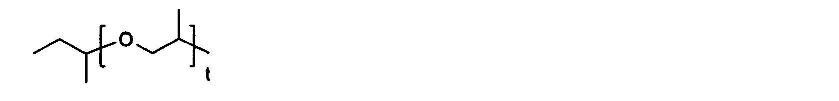 steht,
B² für eine Brücke der Formel 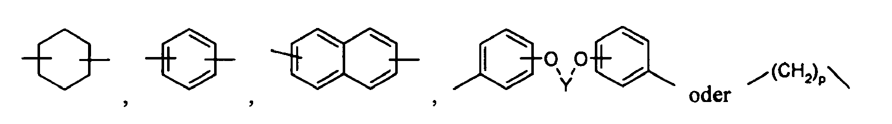 steht,
R² für Methyl, Ethyl, Methoxy oder Fluor steht,
X für eine Brücke der Formel 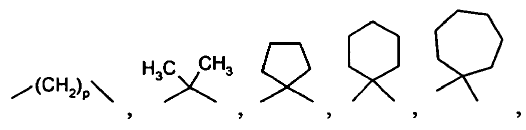 O, S, SO₂ oder CO
steht,
Y für eine Brücke der Formel 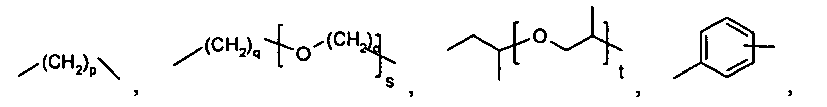 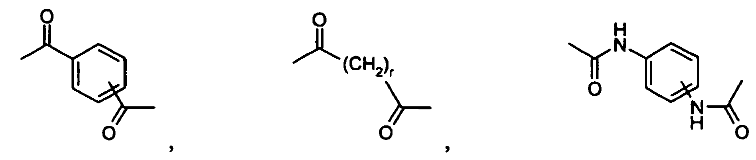 oder 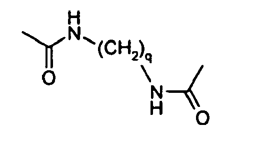 steht,
l für eine ganze Zahl von 0 bis 8 steht,
m, n, p und r unabhängig voneinander für eine ganze Zahl von 1 bis 8 stehen,
q für eine ganze Zahl von 2 bis 8 steht und
s und t unabhängig voneinander für eine ganze Zahl von 1 bis 4 stehen.

17. Verwendung der Mischungen gemäß Anspruch 1 oder der Verbindungen der Formel I 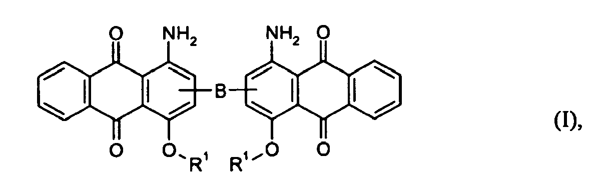 insbesondere der Formel (Ia) 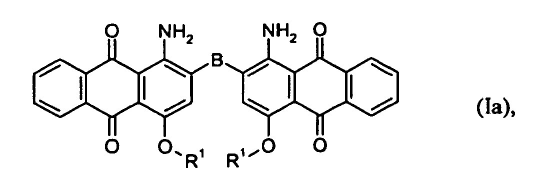 worin
-B- für -O-B¹-O- oder -CH₂-B²-CH₂- steht,
R¹ für Wasserstoff, gegebenenfalls substituiertes C₁-C₈-Alkyl oder gegebenenfalls substituiertes C₃-C₈-Cycloalkyl steht,
B¹ für eine Brücke der Formel 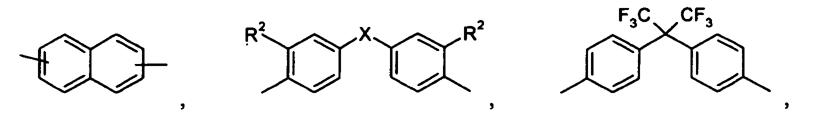 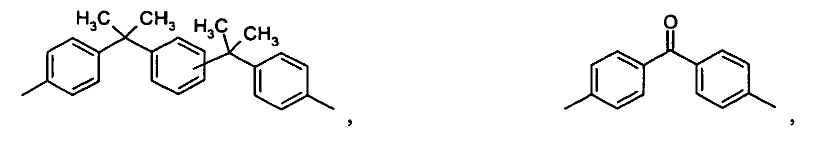 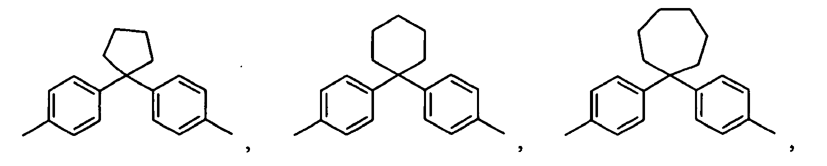 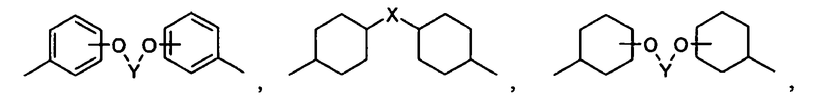 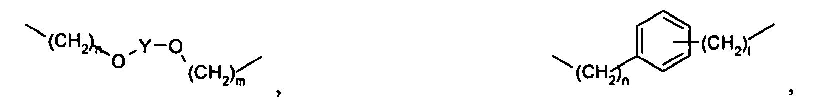 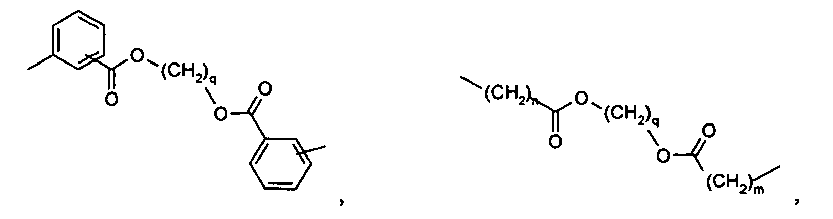 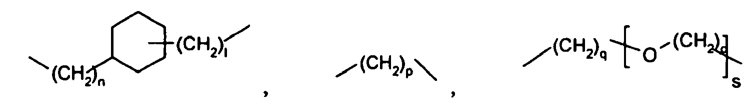 oder 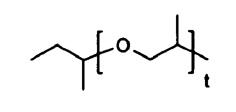 steht,
B² für eine Brücke der Formel 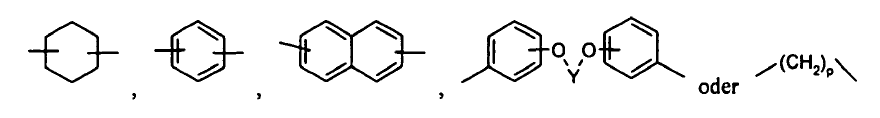 steht,
R² für Methyl, Ethyl, Methoxy oder Fluor steht,
X für eine Brücke der Formel 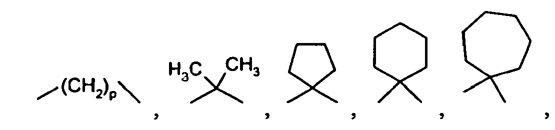 O, S, SO₂ oder CO
steht,
Y für eine Brücke der Formel 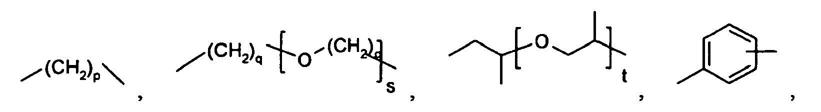 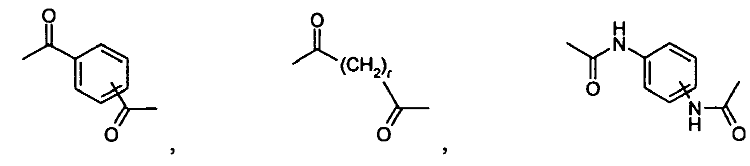 oder 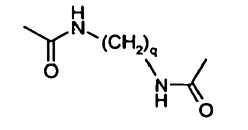 steht,
l für eine ganze Zahl von 0 bis 8 steht,
m, n, p und r unabhängig voneinander für eine ganze Zahl von 1 bis 8 stehen,
q für eine ganze Zahl von 2 bis 8 steht und
s und t unabhängig voneinander für eine ganze Zahl von 1 bis 4 stehen.
zum Färben von Synthesefasern, vorzugsweise in dispergierter Form.

## Claims

1. Mixtures comprising at least one red dye of the formula (I) or (C) 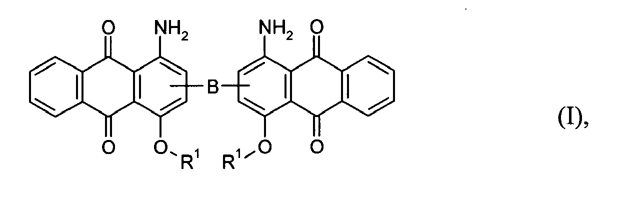 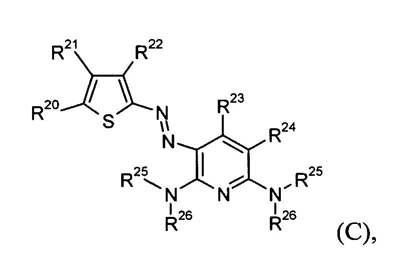 in which
R¹ is hydrogen, optionally substituted C₁-C₈-alkyl or optionally substituted C₃-C₈-cycloalkyl,
B is a bridge of the formulae -O-B¹-O- or -CH₂-B²-CH₂-,
B¹ is a bridge of the formula 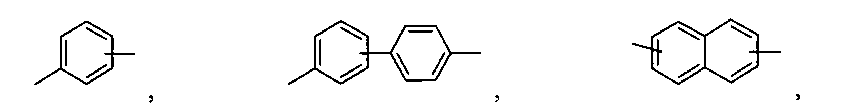 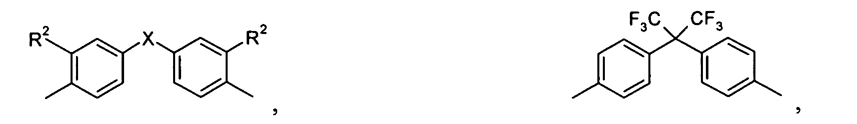 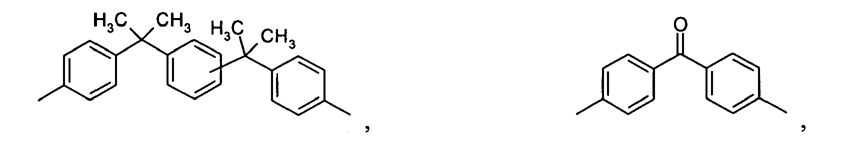 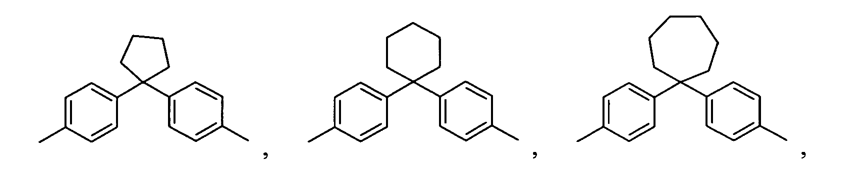 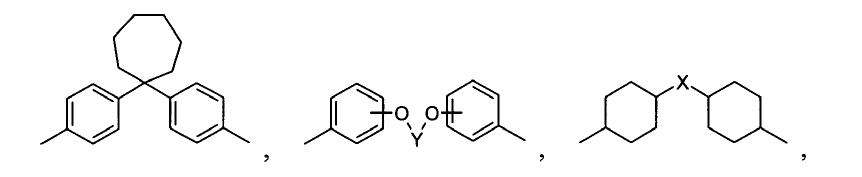 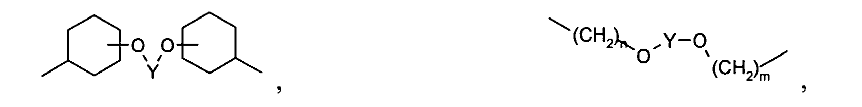 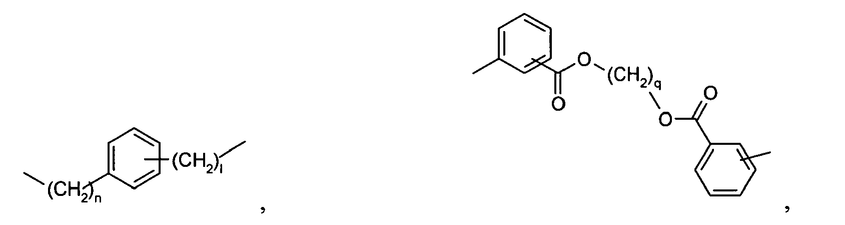 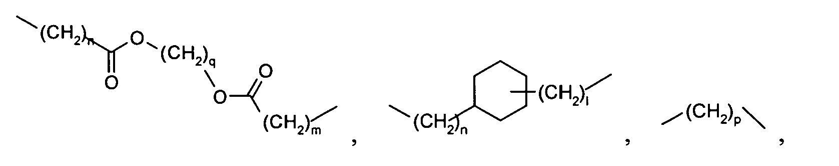 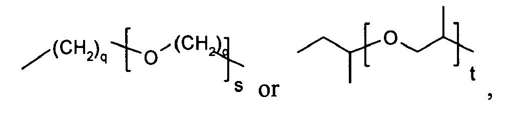
B² is a bridge of the formula 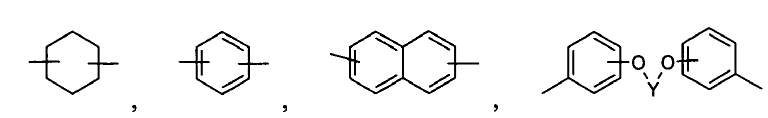 or 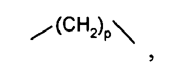
R² is hydrogen, methyl, ethyl, methoxy or fluorine,
X is a bridge of the formula 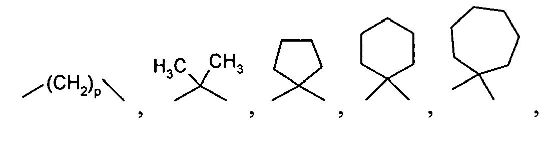 O, S, SO₂ or CO,
Y is a bridge of the formula 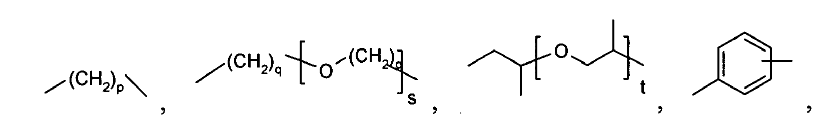 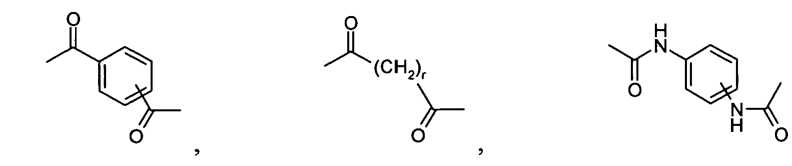 or 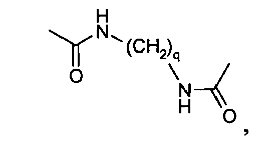
l is an integer from 0 to 8,
m, n, p and r independently of one another are an integer from 1 to 8,
q is an integer from 2 to 8 and
s and t independently of one another are an integer from 1 to 4,
R²⁰ and R²² independently of one another are cyano or optionally substituted C₁-C₈-alkoxycarbonyl,
R²¹ is hydrogen or optionally substituted C₁-C₃-alkyl,
R²³ is hydrogen, optionally substituted C₁-C₈-alkyl or optionally substituted C₁-C₈-alkoxycarbonyl,
R²⁴ is cyano, optionally substituted C₁-C₈-alkylsulphonyl or optionally substituted C₁-C₈-alkoxycarbonyl,
R²⁵ is optionally substituted C₁-C₈-alkyl, optionally substituted C₃-C₇- cycloalkyl, optionally substituted C₆-C₁₀-aryl,
R²⁶ is hydrogen or independently of R²⁵ has the definition of R²⁵, or
NR²⁵R²⁶ is pyrrolidino, piperidino or morpholino,
and at least one orange dye of the formula (L), (LI), (LII), (LIII), (LIV) or (LV) 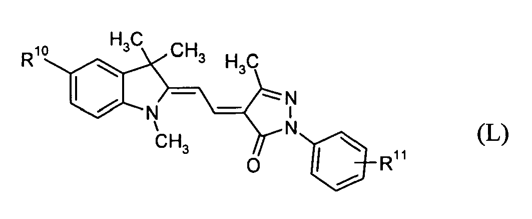 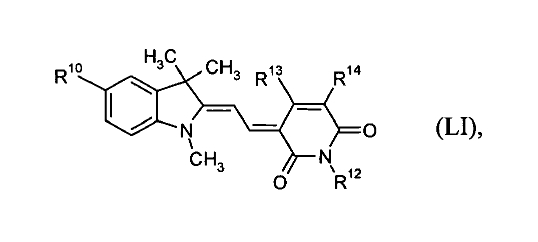 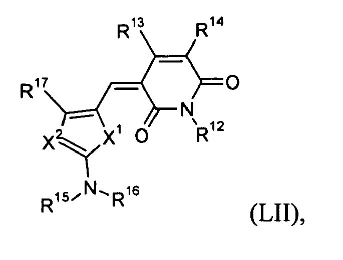 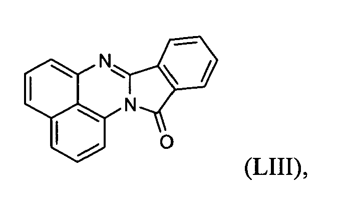 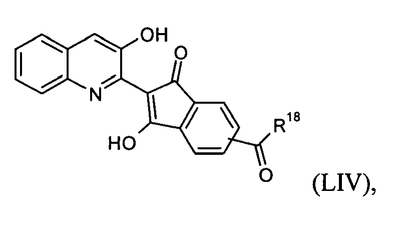 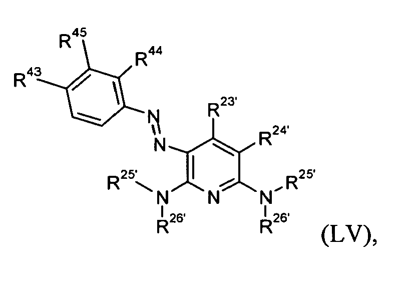 in which
R¹⁰ is hydrogen, optionally substituted C₁-C₈-alkyl, optionally substituted C₁-C₈-alkoxy, optionally substituted C₁-C₈- alkylsulphonyl, optionally substituted C₁-C₈-alkoxycarbonyl, nitro, cyano or fluorine,
R¹¹ is hydrogen, optionally substituted C₁-C₈-alkyl, optionally substituted C₁-C₈-alkoxy, optionally substituted C₁-C₈- alkoxycarbonyl, nitro, cyano or fluorine,
R¹² is hydrogen, optionally substituted C₁-C₈-alkyl or optionally substituted C₃-C₈-cycloalkyl,
R¹³ is hydrogen, optionally substituted C₁-C₈-alkyl or optionally substituted C₁-C₈-alkoxycarbonyl,
R¹⁴ is cyano, optionally substituted C₁-C₈-alkylsulphonyl or optionally substituted C₁-C₈-alkoxycarbonyl,
X¹ is O or S,
X² is N or C-CN,
R¹⁵ and R¹⁶ independently of one another are optionally substituted C₁-C₈- alkyl, optionally substituted C₃-C₈-cycloalkyl or optionally substituted C₆-C₁₀-aryl, or
NR¹⁵R¹⁶ is pyrrolidino, piperidino or morpholino,
R¹⁷ is hydrogen or C₁-C₈-alkyl,
R¹⁸ is optionally substituted C₆-C₁₀-aryl,
R^{23'} is hydrogen, optionally substituted C₁-C₈-alkyl or optionally substituted C₁-C₈-alkoxycarbonyl,
R^{24'} is cyano, optionally substituted C₁-C₈-alkylsulphonyl or optionally substituted C₁-C₈-alkoxycarbonyl,
R^{25'} is optionally substituted C₁-C₈-alkyl, optionally substituted C₃-C₇- cycloalkyl, optionally substituted C₆-C₁₀-aryl,
R^{26'} is hydrogen or independently of R^{25'} has the definition of R^{25'}, or
NR^{25'}R^{26'} is pyrrolidino, piperidino or morpholino,
R⁴³ and R⁴⁴ independently of one another are hydrogen, optionally substituted C₁-C₄-alkyl, more particularly mono- or poly-fluorine- substituted C₁-C₄-alkyl, nitro, cyano, C₁-C₄-alkoxycarbonyl, and
R⁴⁵ is hydrogen, methyl, fluorine or chlorine.

2. Mixture according to Claim 1, comprising a red dye of the formula (I) and at least one orange dye of the formula (L), (LI), (LII), (LIII), (LIV) or (LV), more particularly of the formula (L).

3. Mixture according to Claim 1, comprising a red dye of the formula (C) and at least one orange dye of the formula (L), (LI), (LII), (LIII), (LIV) or (LV), more particularly of the formula (L).

4. Mixture according to Claim 1, comprising a red dye of the formula I in which
R¹ is hydrogen or methyl,
B¹ is a bridge of the formula 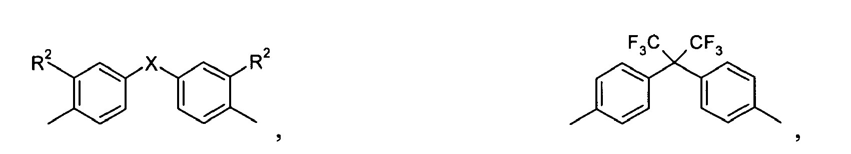 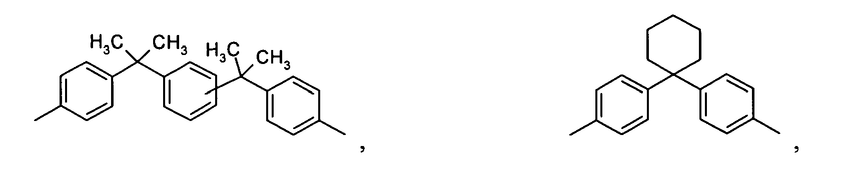 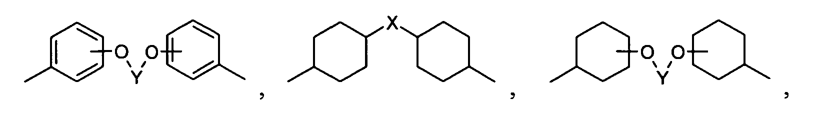 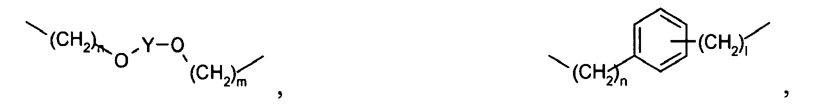 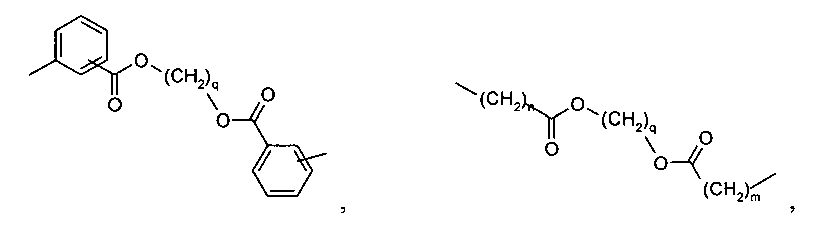 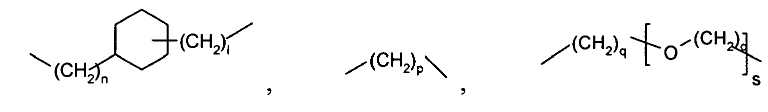 or 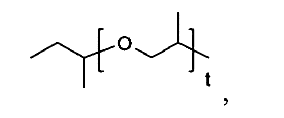
R² is methyl or chlorine,
X is a bridge of the formula 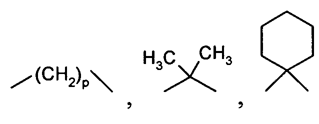 or O,
Y is a bridge of the formula 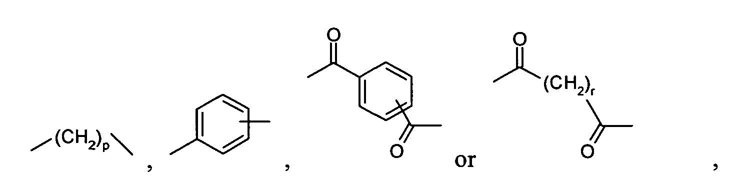
l is an integer from 0 to 4,
m, n and p independently of one another are an integer from 1 to 4,
q and r independently of one another are an integer from 2 to 6,
s and t independently of one another are an integer from 1 to 2 and the undefined ring positions are 1,2-, 1,3- or 1,4-.

5. Mixture according to Claim 1, comprising a red dye of the formula (I),
in which
R¹ is hydrogen or methyl,
B² is a bridge of the formula 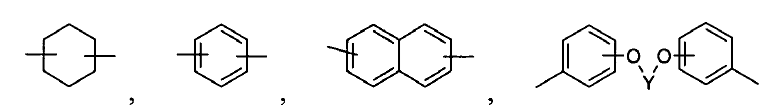 or 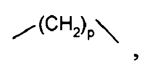
Y is a bridge of the formula 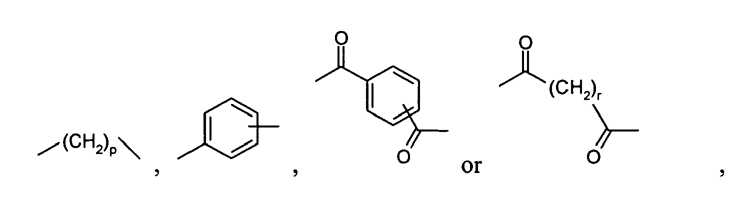
p is an integer from 1 to 6, and
r is an integer from 2 to 6 and
the undefined ring positions in the case of benzene or cyclohexane rings are 1,2-, 1,3- or 1,4- and in the case of the naphthalene ring are 2,6- and 2,7-.

6. Mixture according to Claim 1, comprising a red dye of the formula (C),
in which
R²⁰ is cyano, methoxycarbonyl or ethoxycarbonyl, more preferably cyano or methoxycarbonyl, very preferably methoxycarbonyl,
R²² is cyano,
R²¹ is hydrogen or methyl, more preferably methyl,
R²³ is methyl or methoxycarbonyl, more preferably methyl,
R²⁴ is cyano, methanesulphonyl, methoxycarbonyl or ethoxycarbonyl, more preferably cyano,
R²⁵ is methyl, ethyl, propyl, butyl, methoxyethyl, methoxypropyl, cyanoethyl, benzyl, cyclohexyl, phenyl or tolyl, more preferably ethyl, propyl, butyl, methoxyethyl, methoxypropyl, benzyl, cyclohexyl or phenyl, very preferably propyl, butyl or methoxypropyl,
R²⁶ is hydrogen or independently of R²⁵ has the definition of R²⁵, or
NR²⁵R²⁶ is pyrrolidino, piperidino or morpholino,
more particularly when
R²⁰ is COOCH₃, R²¹ and R²³ is CH₃, R²² and R²⁴ are CN, R²⁵ is -CH₂CH₂CH₂OCH₃ and R²⁶ is H
and an orange dye of the formula (L), more particularly in which
R¹⁰ is COOCH₃ and R¹¹ is H.

7. Mixture according to Claim 1, further comprising at least
a) a yellow dye, preferably with a λₘₐₓ of 420 to 460 nm and at least one green dye, preferably with a λₘₐₓ of 610 to 700 nm, or
b) a yellow dye, preferably with a λₘₐₓ of 420 to 460 nm, and at least one blue dye, preferably with a λₘₐₓ of 570 to 640 nm.

8. Mixtures according to Claim 1, **characterized in that** they further comprise
a) at least one yellow dye of the formula (CCI), (CCII), (CCIII), (CCIV) or (CCV) 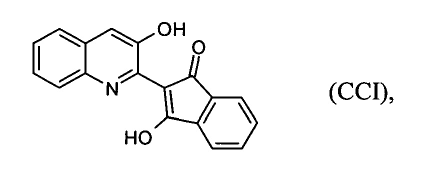 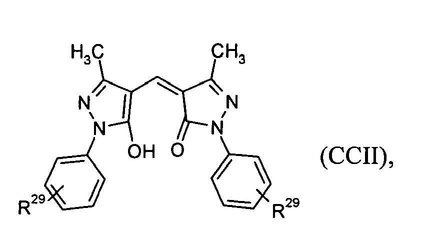 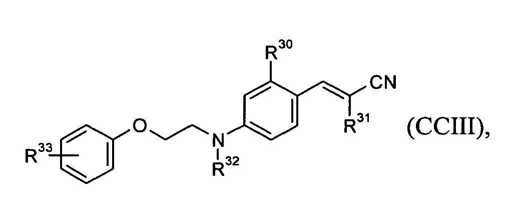 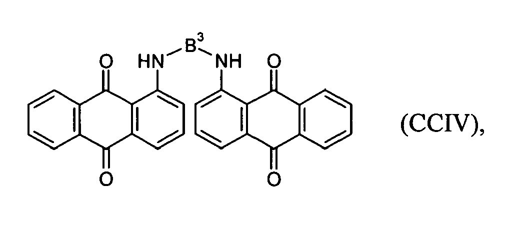 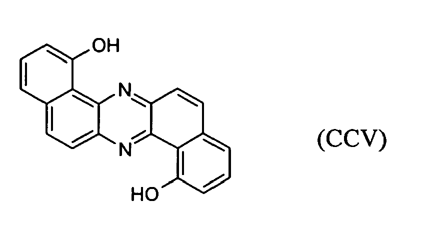 in which
R²⁹ is one or more radicals having the definition hydrogen, optionally substituted C₁-C₈-alkyl, optionally substituted C₁-C₈-alkoxy, fluorine, cyano, optionally substituted C₃-C₇-cycloalkyl or optionally substituted C₆-C₁₀-aryl,
R³⁰ is hydrogen, methyl, ethyl or methoxy,
R³¹ is cyano, C₁-C₈-alkoxycarbonyl or C₁-C₈-alkanesulphonyl,
R³² is hydrogen, optionally substituted C₁-C₈-alkyl or optionally substituted C₃-C₇-cycloalkyl,
R³³ is hydrogen, optionally substituted C₁-C₈-alkyl, optionally substituted C₁-C₈-alkoxy, optionally substituted C₃-C₇-cycloalkyl or optionally substituted C₆-C₁₀-aryl,
and
B³ is a bridge of the formula 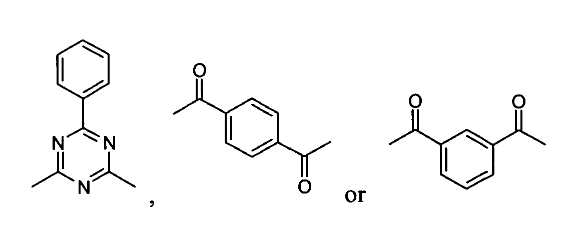 and at least one green dye of the formula (CCLI) 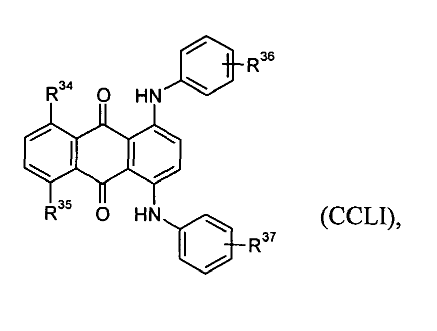
in which
R³⁴ and R³⁵ independently of one another are hydrogen, hydroxyl or C₁-C₄- alkoxy,
R³⁶ and R³⁷ independently of one another are hydrogen, optionally substituted C₁-C₈-alkyl, optionally substituted C₁-C₈-alkoxy, fluorine, optionally substituted C₃-C₇-cycloalkyl or optionally substituted C₆-C₁₀-aryl,
or
b) at least one yellow dye of the formula (CCI), (CCII), (CCIII), (CCIV) or (CCV) and at least one blue dye of the formula (CCCI) 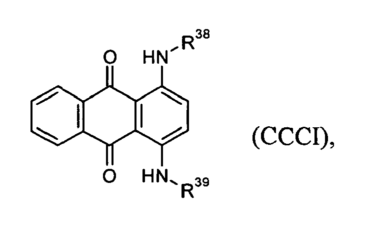 in which
R³⁸ and R³⁹ independently of one another are optionally substituted C₁-C₈-alkyl, optionally substituted C₃-C₇-cycloalkyl or optionally substituted C₆-C₁₀-aryl.

9. Masterbatch containing 15% - 80%, more particularly 15% - 70% by weight of a mixture according to Claim 1 and a carrier.

10. Use of a mixture according to Claim 1 for the mass colouring of plastics.

11. Method of mass-colouring plastics, **characterized in that** the mixture according to Claim 1, preferably in the form of its masterbatch, is incorporated into the melted mass of plastic or actually added to the starting components for producing the plastic, prior to the polymerization.

12. Plastics comprising a mixture according to Claim 1.

13. Masterbatch containing 15% - 90% by weight, more particularly 15% - 70% by weight of a compound of the formula I 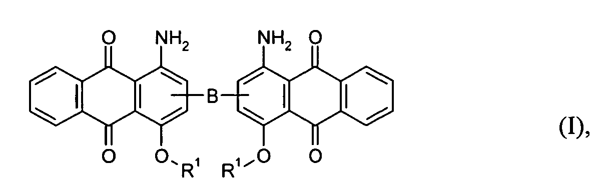 more particularly of the formula (Ia) 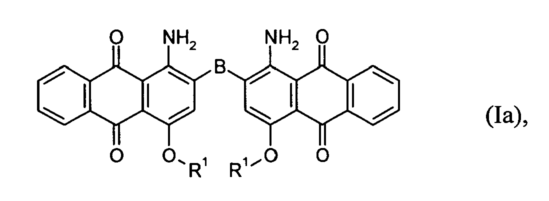 in which
-B- is -O-B¹-O- or -CH₂-B²-CH₂-,
R¹ is hydrogen, optionally substituted C₁-C₈-alkyl or optionally substituted C₃-C₈-cycloalkyl,
B¹ is a bridge of the formula 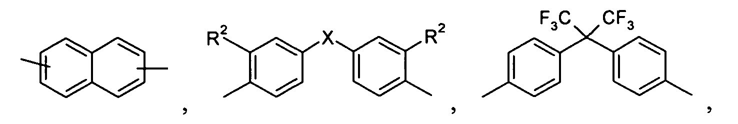 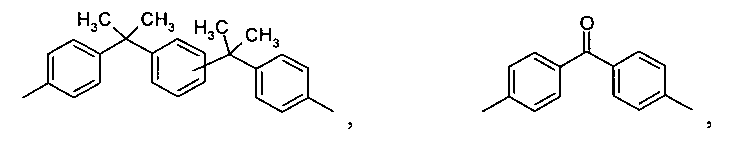 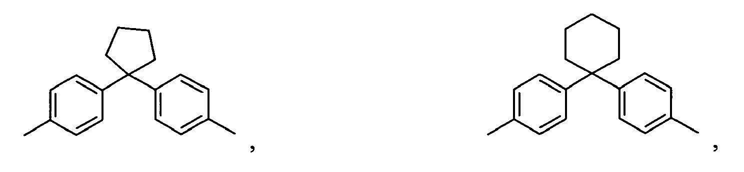 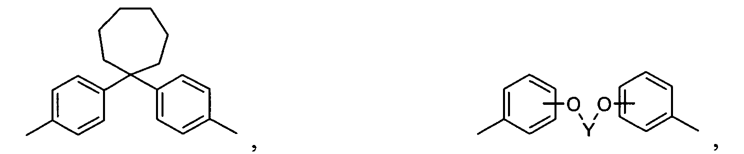 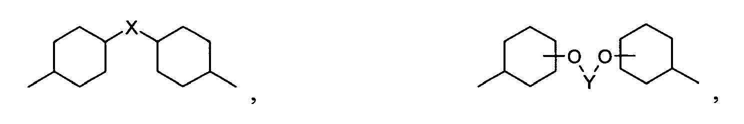 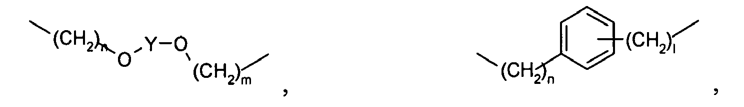 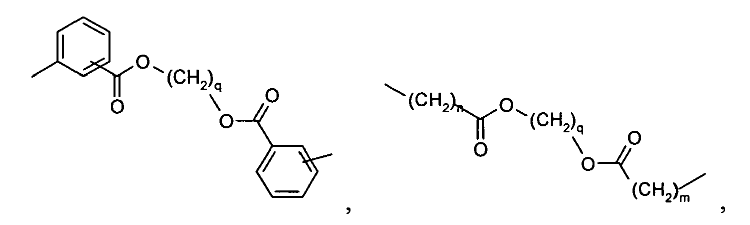 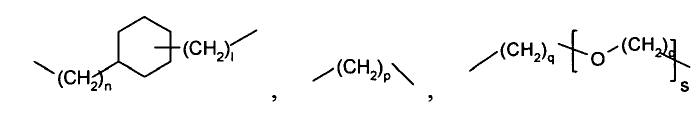 or 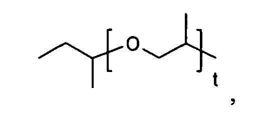
B² is a bridge of the formula 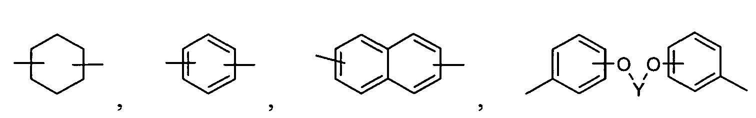 or 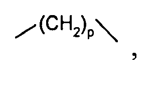
R² is methyl, ethyl, methoxy or fluorine,
X is a bridge of the formula 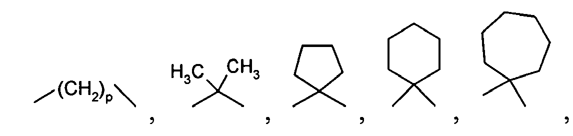 O, S, SO₂ or CO,
Y is a bridge of the formula 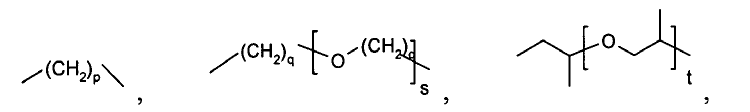 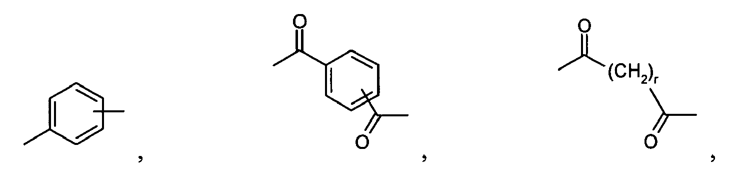 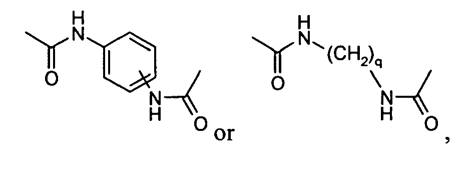
l is an integer from 0 to 8,
m, n, p and r independently of one another are an integer from 1 to 8,
q is an integer from 2 to 8 and
s and t independently of one another are an integer from 1 to 4,
and a carrier.

14. Use of compounds of the formula I 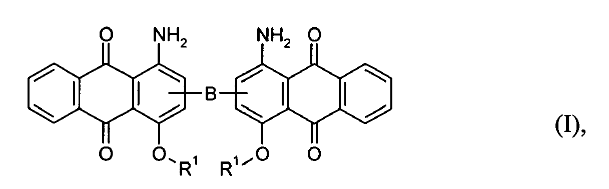 more particularly of the formula (Ia) 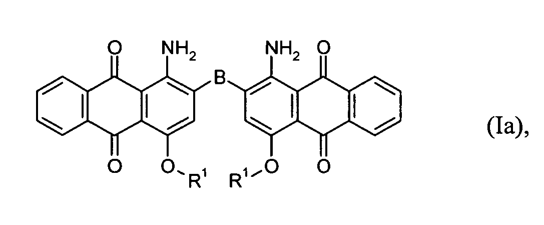 in which
-B- is -O-B¹-O- or -CH₂-B²-CH₂-,
R¹ is hydrogen, optionally substituted C₁-C₈-alkyl or optionally substituted C₃-C₈-cycloalkyl,
B¹ is a bridge of the formula 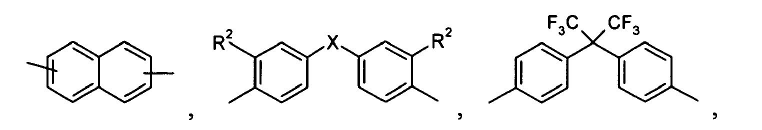 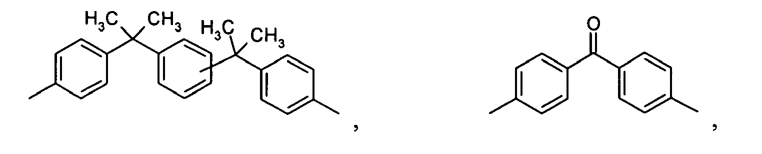 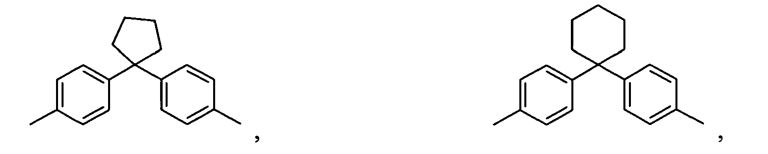 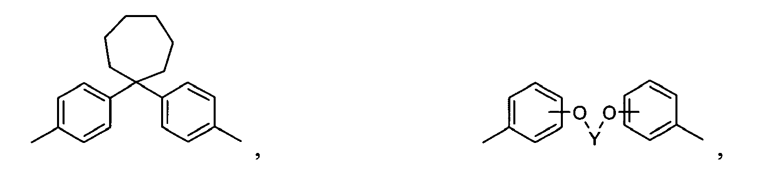 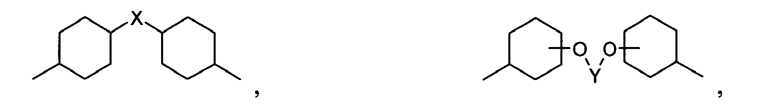 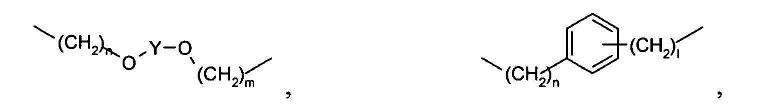 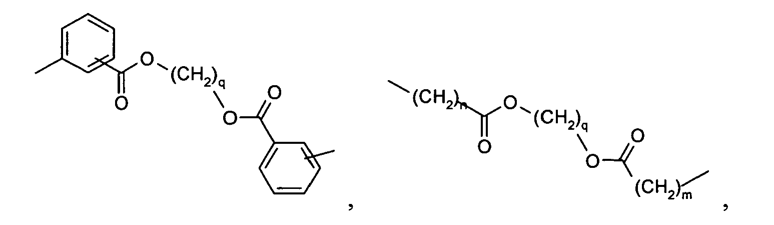 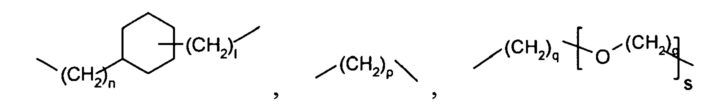 or 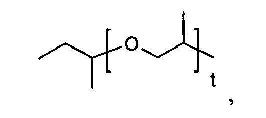
B² is a bridge of the formula 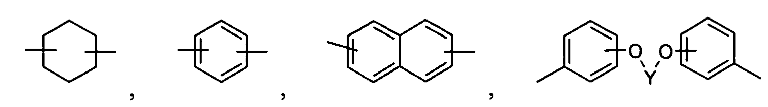 or 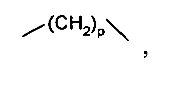
R² is methyl, ethyl, methoxy or fluorine,
X is a bridge of the formula 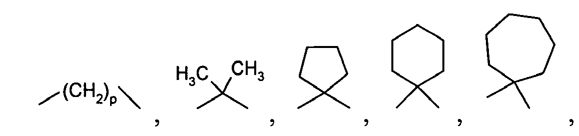 O, S, SO₂ or CO,
Y is a bridge of the formula 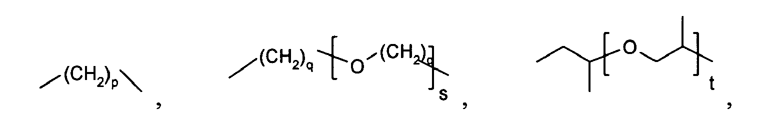 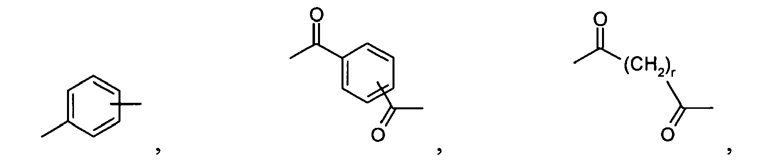 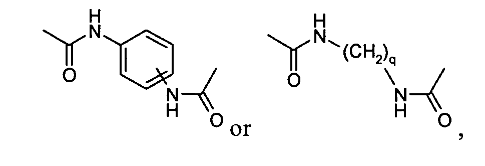
l is an integer from 0 to 8,
m, n, p and r independently of one another are an integer from 1 to 8,
q is an integer from 2 to 8 and
s and t independently of one another are an integer from 1 to 4, for
the mass colouring of plastics.

15. Method of mass-colouring plastics, **characterized in that** a compound of the formula I 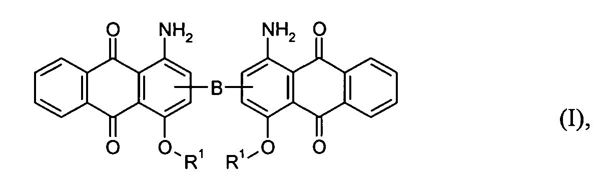 more particularly of the formula (Ia) 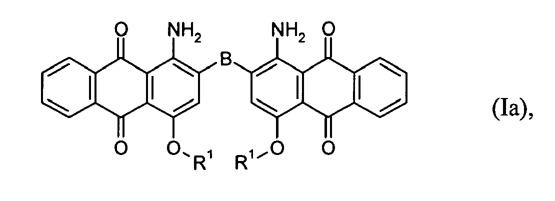 in which
-B- is -O-B¹-O- or -CH₂-B²-CH₂-,
R¹ is hydrogen, optionally substituted C₁-C₈-alkyl or optionally substituted C₃-C₈-cycloalkyl,
B¹ is a bridge of the formula 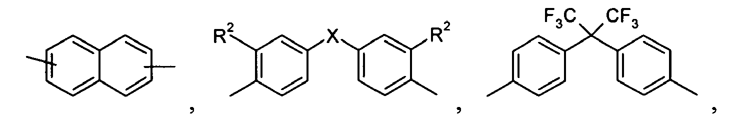 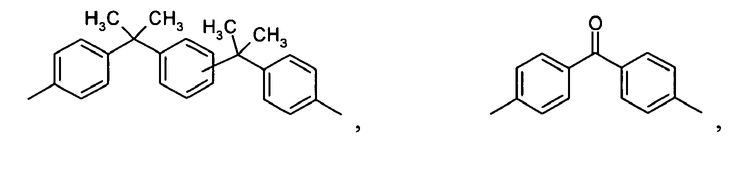 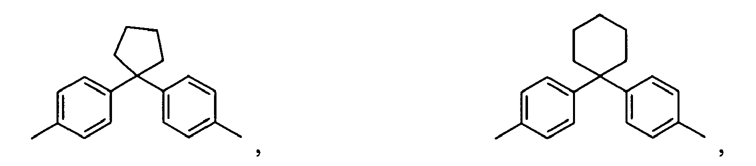 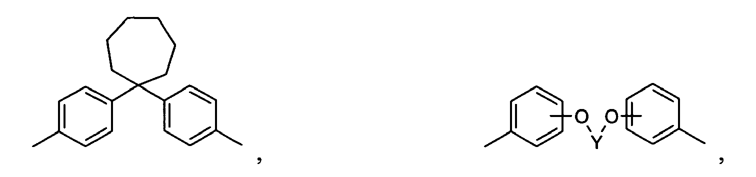 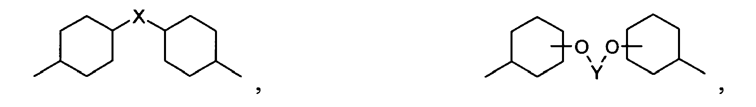 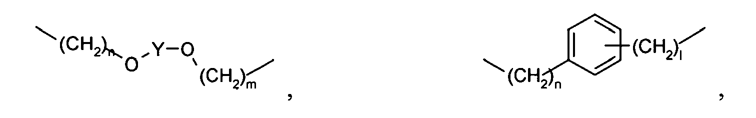 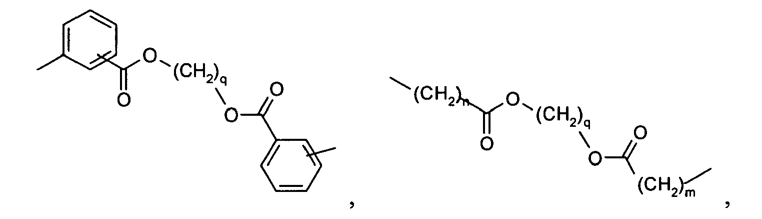 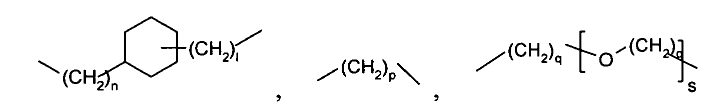 or 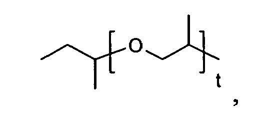
B² is a bridge of the formula 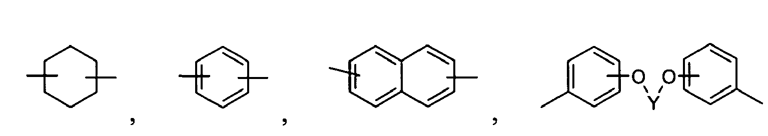 or 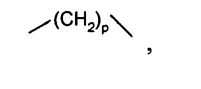
R² is methyl, ethyl, methoxy or fluorine,
X is a bridge of the formula 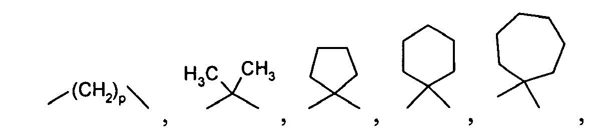 O, S, SO₂ or CO,
Y is a bridge of the formula 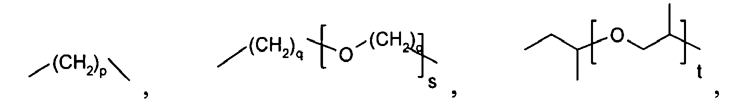 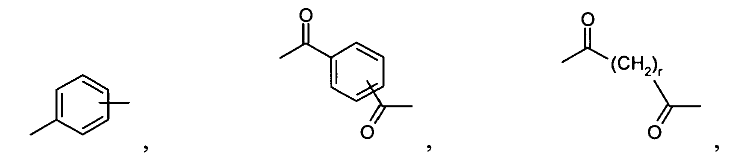 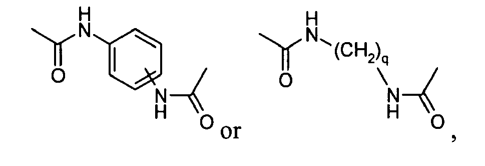
l is an integer from 0 to 8,
m, n, p and r independently of one another are an integer from 1 to 8,
q is an integer from 2 to 8 and
s and t independently of one another are an integer from 1 to 4, preferably in the form of its masterbatch, is incorporated into the melted plastic mass or actually added to the starting components for producing the plastic, prior to the polymerization.

16. Plastics comprising a compound of the formula I 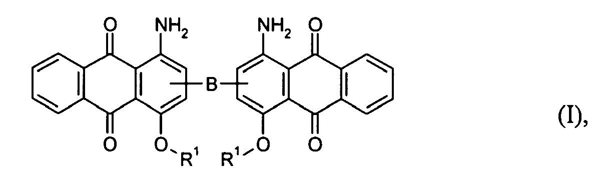 more particularly of the formula (Ia) 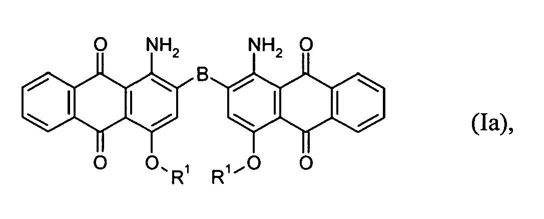 in which
-B- is -O-B¹-O- or -CH₂-B²-CH₂-,
R¹ is hydrogen, optionally substituted C₁-C₈-alkyl or optionally substituted C₃-C₈-cycloalkyl,
B¹ is a bridge of the formula 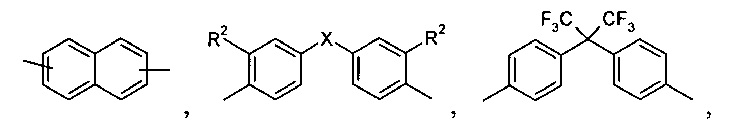 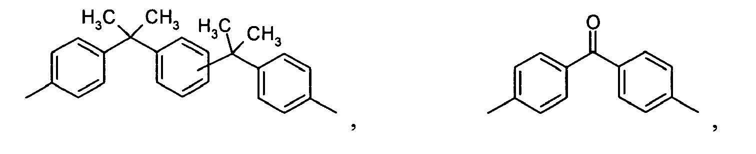 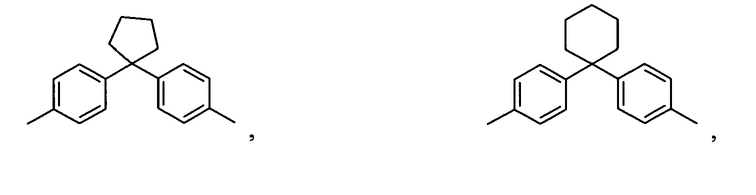 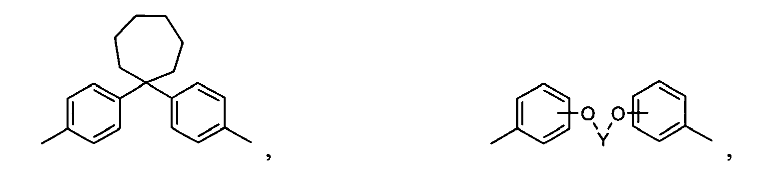 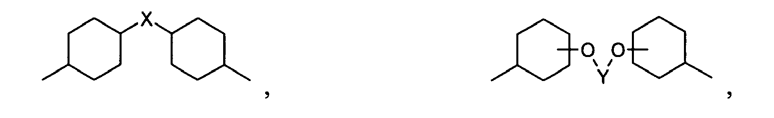 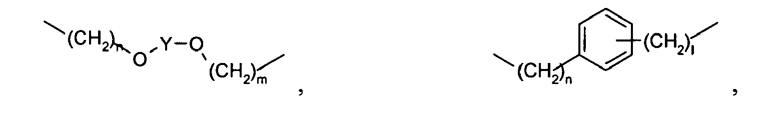 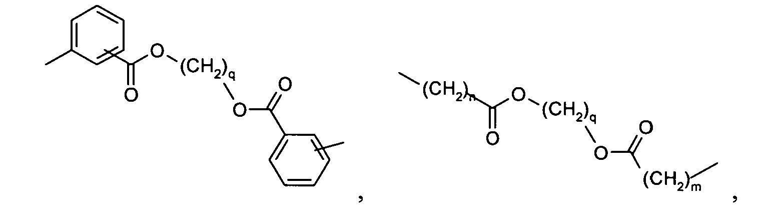 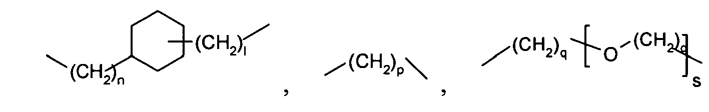 or 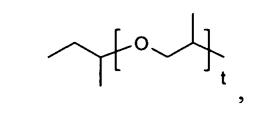
B² is a bridge of the formula 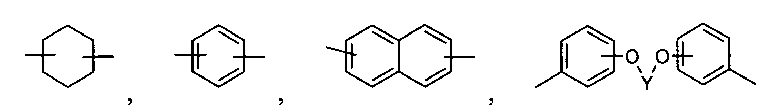 or 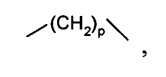
R² is methyl, ethyl, methoxy or fluorine,
X is a bridge of the formula 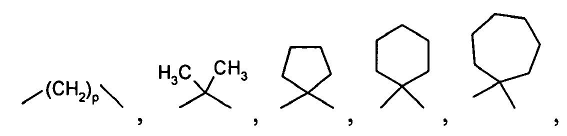 O, S, SO₂ or CO,
Y is a bridge of the formula 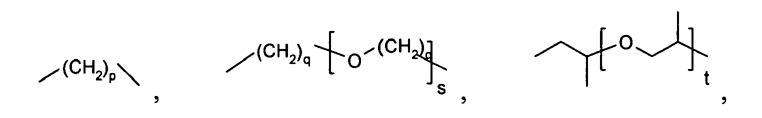 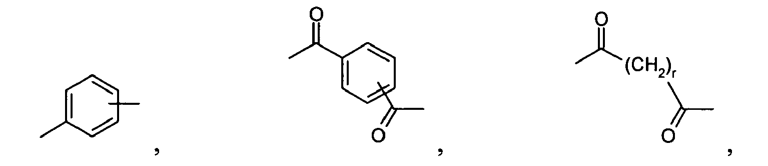 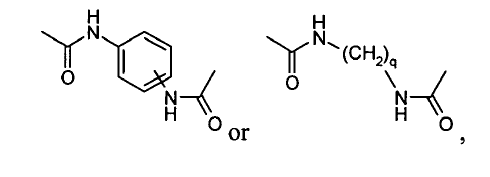
l is an integer from 0 to 8,
m, n, p and r independently of one another are an integer from 1 to 8,
q is an integer from 2 to 8 and
s and t independently of one another are an integer from 1 to 4.

17. Use of the mixtures according to Claim 1 or of the compounds of the formula I 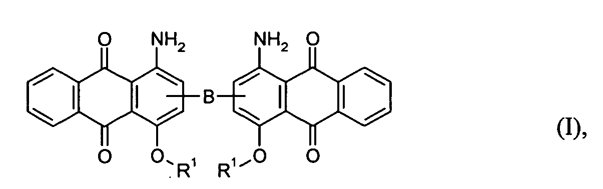 more particularly of the formula (Ia) 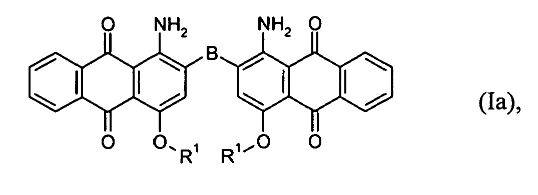 in which
-B- is -O-B¹-O- or -CH₂-B²-CH₂-,
R¹ is hydrogen, optionally substituted C₁-C₈-alkyl or optionally substituted C₃-C₈-cycloalkyl,
B¹ is a bridge of the formula 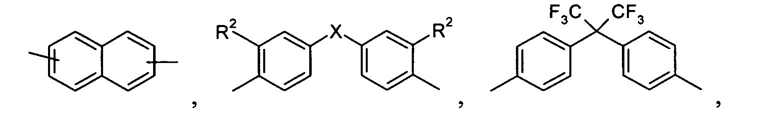 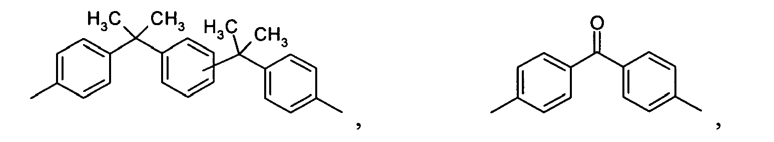 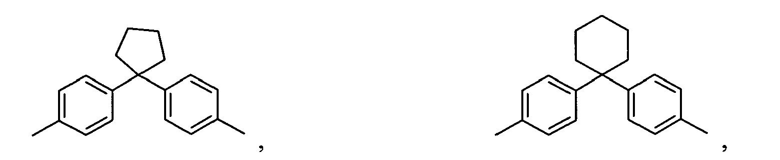 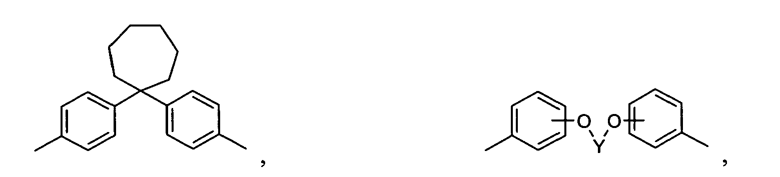 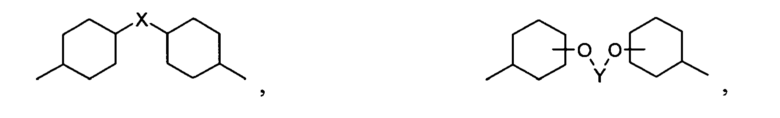 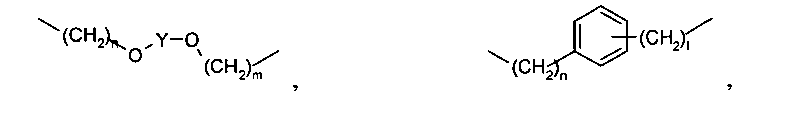 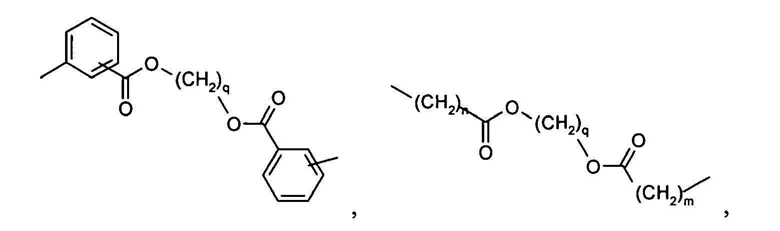 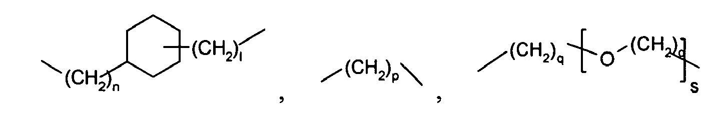 or 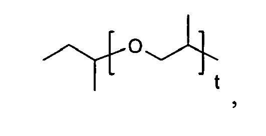
B² is a bridge of the formula 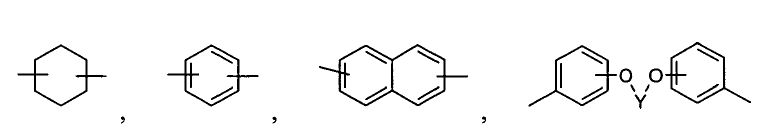 or 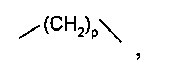
R² is methyl, ethyl, methoxy or fluorine,
X is a bridge of the formula 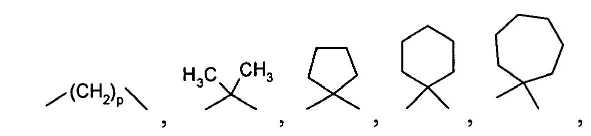 O, S, SO₂ or CO,
Y is a bridge of the formula 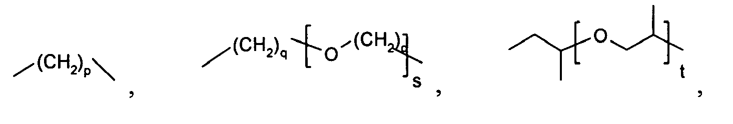 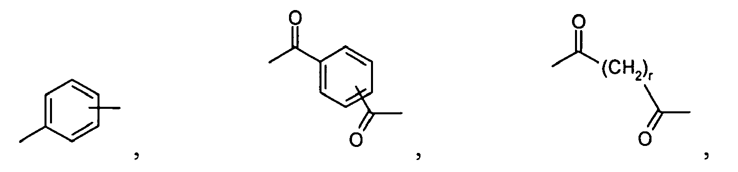 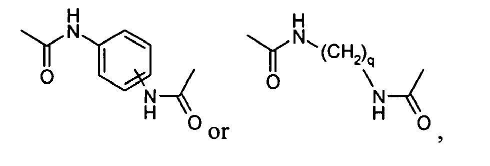
l is an integer from 0 to 8,
m, n, p and r independently of one another are an integer from 1 to 8,
q is an integer from 2 to 8 and
s and t independently of one another are an integer from 1 to 4,
for colouring synthetic fibres, preferably in dispersed form.

## Revendications

1. Mélanges, contenant au moins un colorant rouge de formule (I) ou (C) 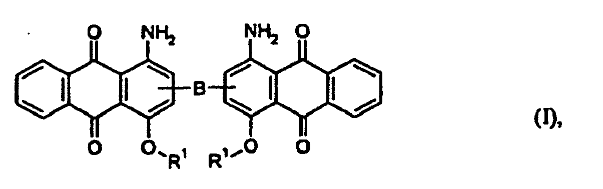 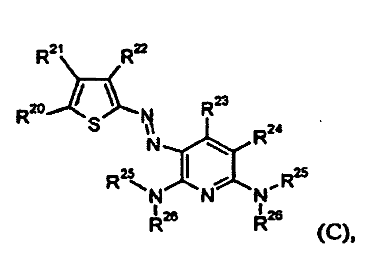 dans lesquelles
R¹ représente hydrogène, alkyle en C₁-C₈ éventuellement substitué ou cycloalkyle en C₃-C₈ éventuellement substitué,
B représente un pont de formule -O-B¹-O- ou -CH₂-B²-CH₂-,
B¹ représente un pont de formule 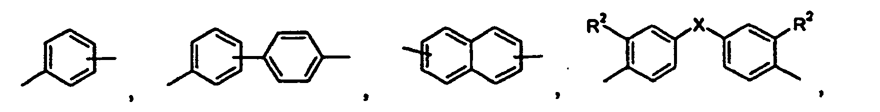 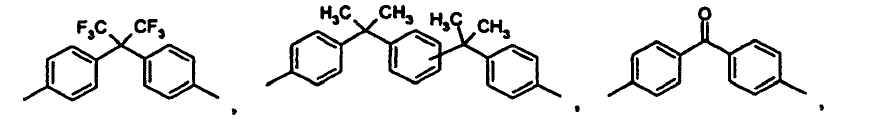 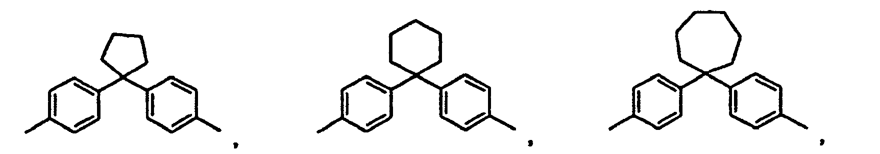 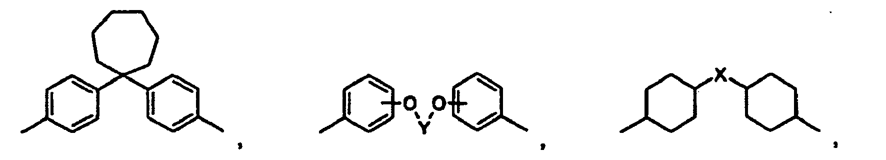 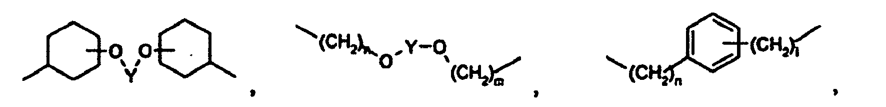 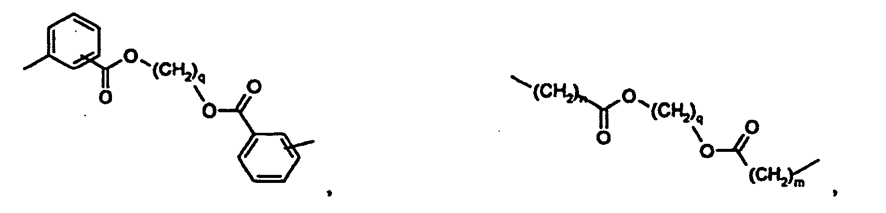 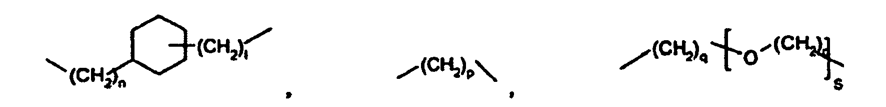 ou 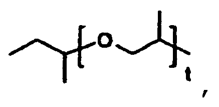 B² représente un pont de formule 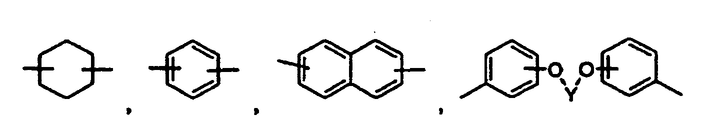 ou 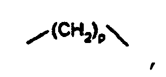 R² représente hydrogène, méthyle, éthyle, méthoxy ou fluor,
X représente un pont de formule 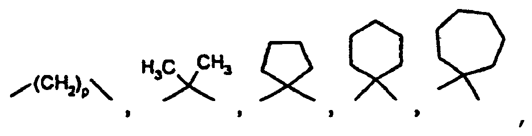 O, S, SO₂ ou CO,
Y représente un pont de formule 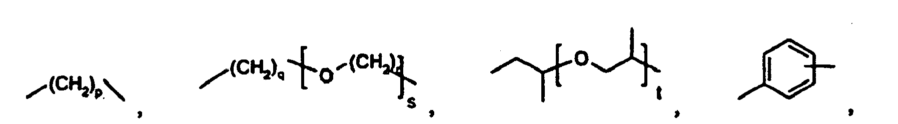 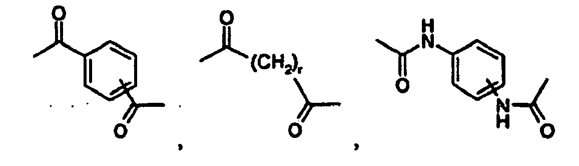 ou 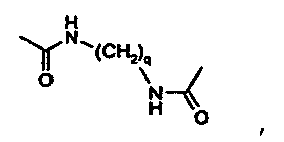 l représente un nombre entier de 0 à 8,
m, n, p et r représentent indépendamment les uns des autres un nombre entier de 1 à 8,
q représente un nombre entier de 2 à 8, et
s et t représentent indépendamment l'un de l'autre un nombre entier de 1 à 4,
R²⁰ et R²² représentent indépendamment l'un de l'autre cyano ou alcoxycarbonyle en C₁-C₈ éventuellement substitué,
R²¹ représente hydrogène ou alkyle en C₁-C₃ éventuellement substitué,
R²³ représente hydrogène, alkyle en C₁-C₈ éventuellement substitué ou alcoxycarbonyle en C₁-C₈ éventuellement substitué,
R²⁴ représente cyano, alkylsulfonyle en C₁-C₈ éventuellement substitué ou alcoxycarbonyle en C₁-C₈ éventuellement substitué,
R²⁵ représente alkyle en C₁-C₈ éventuellement substitué, cycloalkyle en C₃-C₇ éventuellement substitué, aryle en C₆-C₁₀ éventuellement substitué,
R²⁶ représente hydrogène ou a indépendamment de R²⁵ la signification de R²⁵, ou
NR²⁵R²⁶ représente pyrrolidino, pipéridino ou morpholino,
et au moins un colorant orange de formule (L), (LI), (LII), (LIII), (LIV) ou (LV) 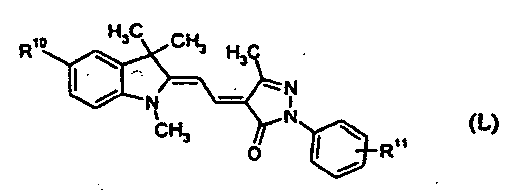 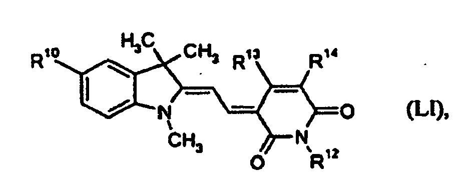 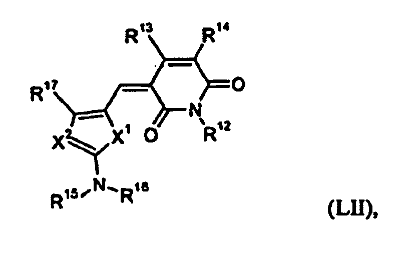 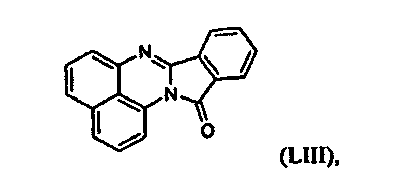 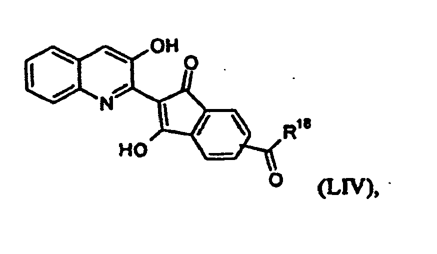 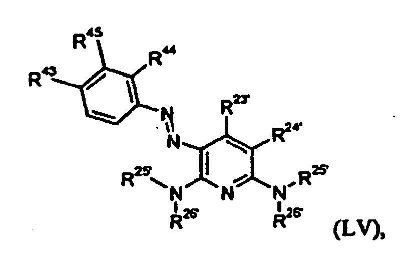 dans lesquelles
R¹⁰ représente hydrogène, alkyle en C₁-C₈ éventuellement substitué, alcoxy en C₁-C₈ éventuellement substitué, alkylsulfonyle en C₁-C₈ éventuellement substitué, alcoxycarbonyle en C₁-C₈ éventuellement substitué, nitro, cyano ou fluor,
R¹¹ représente hydrogène, alkyle en C₁-C₈ éventuellement substitué, alcoxy en C₁-C₈ éventuellement substitué, alcoxycarbonyle en C₁-C₈ éventuellement substitué, nitro, cyano ou fluor,
R¹² représente hydrogène, alkyle en C₁-C₈ éventuellement substitué ou cycloalkyle en C₃-C₈ éventuellement substitué,
R¹³ représente hydrogène, alkyle en C₁-C₈ éventuellement substitué ou alcoxycarbonyle en C₁-C₈ éventuellement substitué,
R¹⁴ représente cyano, alkylsulfonyle en C₁-C₈ éventuellement substitué ou alcoxycarbonyle en C₁-C₈ éventuellement substitué,
X¹ représente O ou S,
X² représente N ou C-CN,
R¹⁵ et R¹⁶ représentent indépendamment l'un de l'autre alkyle en C₁-C₈ éventuellement substitué, cycloalkyle en C₃-C₈ éventuellement substitué ou aryle en C₆-C₁₀ éventuellement substitué, ou
NR¹⁵R¹⁶ représente pyrrolidino, pipéridino ou morpholino,
R¹⁷ représente hydrogène ou alkyle en C₁-C₈,
R¹⁸ représente aryle en C₆-C₁₀ éventuellement substitué,
R²³' représente hydrogène, alkyle en C₁-C₈ éventuellement substitué ou alcoxycarbonyle en C₁-C₈ éventuellement substitué,
R²⁴' représente cyano, alkylsulfonyle en C₁-C₈ éventuellement substitué ou alcoxycarbonyle en C₁-C₈ éventuellement substitué,
R²⁵' représente alkyle en C₁-C₈ éventuellement substitué, cycloalkyle en C₃-C₇ éventuellement substitué, aryle en C₆-C₁₀ éventuellement substitué,
R²⁶' représente hydrogène ou a indépendamment de R²⁵' la signification de R^{25'} ou
NR²⁵' R²⁶' représente pyrrolidino, pipéridino ou morpholino,
R⁴³ et R⁴⁴ représentent indépendamment l'un de l'autre hydrogène, alkyle en C₁-C₄ éventuellement substitué, notamment alkyle en C₁-C₄ substitué par un ou plusieurs fluors, nitro, cyano, alcoxycarbonyle en C₁-C₄, et
R⁴⁵ représente hydrogène, méthyle, fluor ou chlore.

2. Mélange selon la revendication 1, contenant un colorant rouge de formule (I) et au moins un colorant orange de formule (L), (LI), (LII), (LIII), (LIV) ou (LV), notamment de formule (L).

3. Mélange selon la revendication 1, contenant un colorant rouge de formule (C) et au moins un colorant orange de formule (L), (LI), (LII), (LIII), (LIV) ou (LV), notamment de formule (L).

4. Mélange selon la revendication 1, contenant un colorant rouge de formule (I),
dans laquelle
R¹ représente hydrogène ou méthyle,
B¹ représente un pont de formule 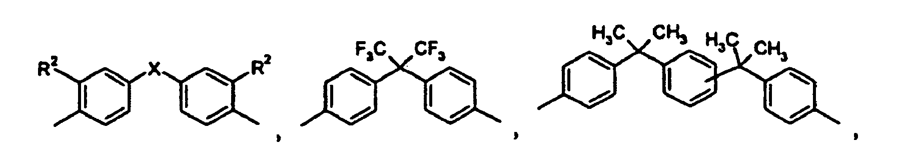 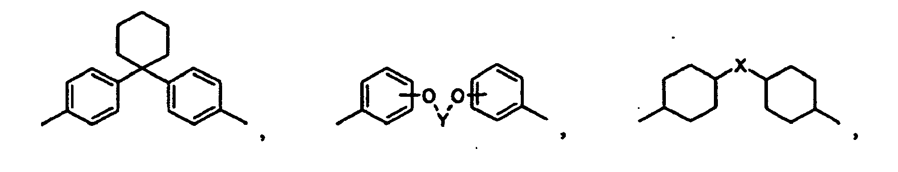 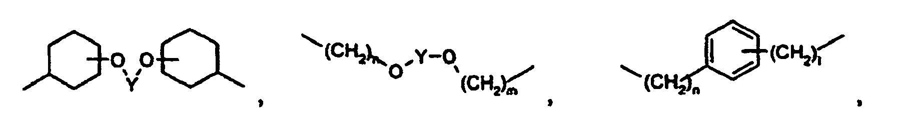 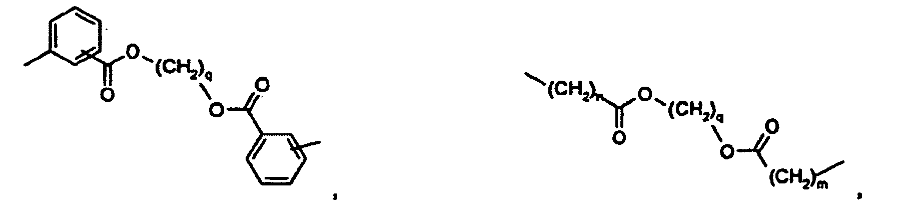 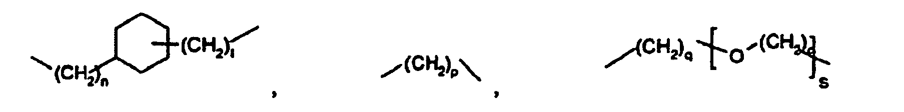 ou 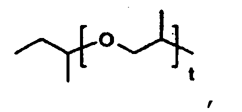
R² représente méthyle ou chlore,
X représente un pont de formule 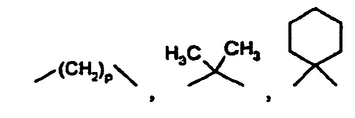 ou O,
Y représente un pont de formule 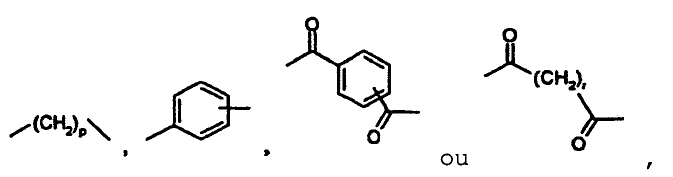
l représente un nombre entier de 0 à 4,
m, n et p représentent indépendamment les uns des autres un nombre entier de 1 à 4,
q et r représentent indépendamment l'un de l'autre un nombre entier de 2 à 6,
s et t représentent indépendamment l'un de l'autre un nombre entier de 1 à 2, et
les positions de cycle non définies signifient 1,2-, 1,3- ou 1,4-.

5. Mélange selon la revendication 1, contenant un colorant rouge de formule (I),
dans laquelle
R¹ représente hydrogène ou méthyle,
B² représente un pont de formule 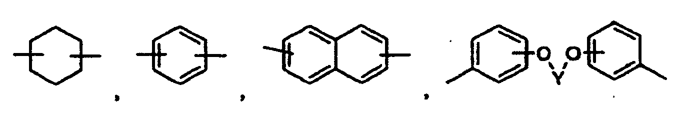 ou 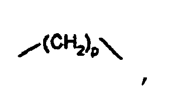
Y représente un pont de formule 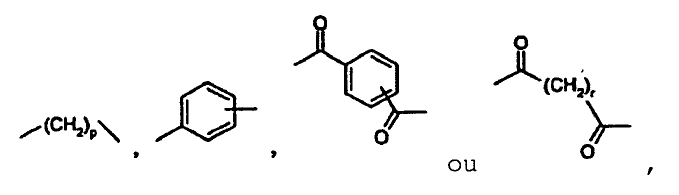
p représente un nombre entier de 1 à 6, et
r représente un nombre entier de 2 à 6, et
les positions de cycle non définies dans les cycles benzène et cyclohexane signifient 1,2-, 1,3- ou 1,4- et dans le cycle naphtaline 2,6- et 2,7-.

6. Mélange selon la revendication 1, contenant un colorant rouge de formule (C),
dans laquelle
R²⁰ représente cyano, méthoxycarbonyle ou éthoxycarbonyle, de manière particulièrement préférée cyano ou méthoxycarbonyle, de manière tout particulièrement préférée méthoxycarbonyle,
R²² représente cyano,
R²¹ représente hydrogène ou méthyle, de manière particulièrement préférée méthyle,
R²³ représente méthyle ou méthoxycarbonyle, de manière particulièrement préférée méthyle,
R²⁴ représente cyano, méthanesulfonyle, méthoxycarbonyle ou éthoxycarbonyle, de manière particulièrement préférée cyano,
R²⁵ représente méthyle, éthyle, propyle, butyle, méthoxyéthyle, méthoxypropyle, cyanéthyle, benzyle, cyclohexyle, phényle ou tolyle, de manière particulièrement préférée éthyle, propyle, butyle, méthoxyéthyle, méthoxypropyle, benzyle, cyclohexyle ou phényle, de manière tout particulièrement préférée propyle, butyle ou méthoxypropyle,
R²⁶ représente hydrogène ou a indépendamment de R²⁵ la signification de R²⁵, ou
NR²⁵R²⁶ représente pyrrolidino, pipéridino ou morpholino,
notamment lorsque
R²⁰ représente COOCH₃, R²¹ et R²³ représentent CH₃, R²² et
R²⁴ représentent CN, R²⁵ représente -CH₂CH₂CH₂OCH₃ et R²⁶ représente H,
et un colorant orange de formule (L), notamment dans laquelle
R¹⁰ représente COOCH₃ et R¹¹ représente H.

7. Mélange selon la revendication 1, contenant également au moins
a) un colorant jaune, de préférence ayant un λₘₐₓ de 420 à 460 nm, et au moins un colorant vert, de préférence ayant un λₘₐₓ de 610 à 700 nm, ou
b) un colorant jaune, de préférence ayant un λₘₐₓ de 420 à 460 nm, et au moins un colorant bleu, de préférence ayant un λₘₐₓ de 570 à 640 nm.

8. Mélanges selon la revendication 1, **caractérisés en ce qu'**ils contiennent également
a) au moins un colorant jaune de formule (CCI), (CCII), (CCIII), (CCIV) ou (CCV) 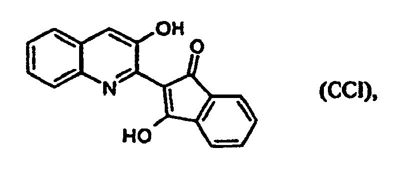 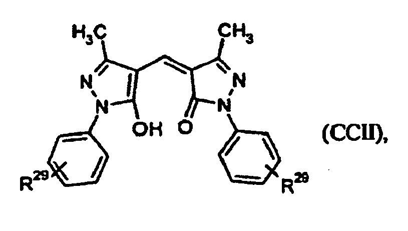 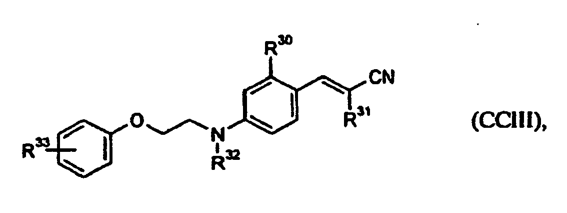 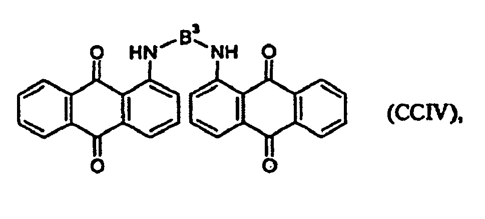 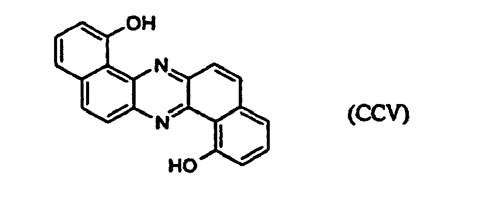 dans lesquelles
R²⁹ représente un ou plusieurs radicaux ayant la signification hydrogène, alkyle en C₁-C₈ éventuellement substitué, alcoxy en C₁-C₈ éventuellement substitué, fluor, cyano, cycloalkyle en C₃-C₇ éventuellement substitué ou aryle en C₆-C₁₀ éventuellement substitué, R³⁰ représente hydrogène, méthyle, éthyle ou méthoxy, R³¹ représente cyano, alcoxycarbonyle en C₁-C₈ ou alcanesulfonyle en C₁-C₈,
R³² représente hydrogène, alkyle en C₁-C₈ éventuellement substitué ou cycloalkyle en C₃-C₇ éventuellement substitué,
R³³ représente hydrogène, alkyle en C₁-C₈ éventuellement substitué, alcoxy en C₁-C₈ éventuellement substitué, cycloalkyle en C₃-C₇ éventuellement substitué ou aryle en C₆-C₁₀ éventuellement substitué,
et
B³ représente un pont de formule 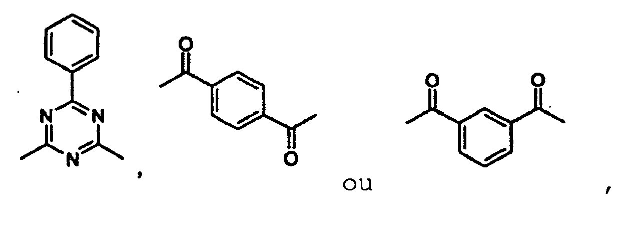 et au moins un colorant vert de formule (CCLI) 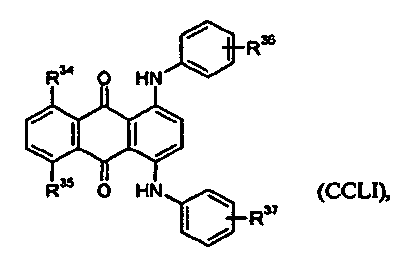 dans laquelle
R³⁴ et R³⁵ représentent indépendamment l'un de l'autre hydrogène, hydroxy ou alcoxy en C₁-C₄,
R³⁶ et R³⁷ représentent indépendamment l'un de l'autre hydrogène, alkyle en C₁-C₈ éventuellement substitué, alcoxy en C₁-C₈ éventuellement substitué, fluor, cycloalkyle en C₃-C₇ éventuellement substitué ou aryle en C₆-C₁₀ éventuellement substitué,
ou
b) au moins un colorant jaune de formule (CCI), (CCII), (CCIII), (CCIV) ou (CCV) et au moins un colorant bleu de formule (CCCI) 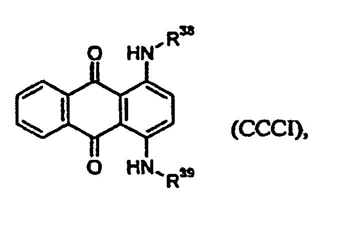 dans laquelle
R³⁸ et R³⁹ représentent indépendamment l'un de l'autre alkyle en C₁-C₈ éventuellement substitué, cycloalkyle en C₃-C₇ éventuellement substitué ou aryle en C₆-C₁₀ éventuellement substitué.

9. Mélange maître, contenant 15 à 80, notamment 15 à 70 % en poids d'un mélange selon la revendication 1 et un support.

10. Utilisation d'un mélange selon la revendication 1 pour la coloration en masse de plastiques.

11. Procédé de coloration en masse de plastiques, **caractérisé en ce que** le mélange selon la revendication 1, de préférence sous la forme de son mélange maître, est incorporé dans la masse plastique fondue ou déjà ajouté aux composants de départ pour la fabrication du plastique avant la polymérisation.

12. Plastiques contenant un mélange selon la revendication 1.

13. Mélange maître, contenant 15 à 90 % en poids, notamment 15 à 70 % en poids, d'un composé de formule I 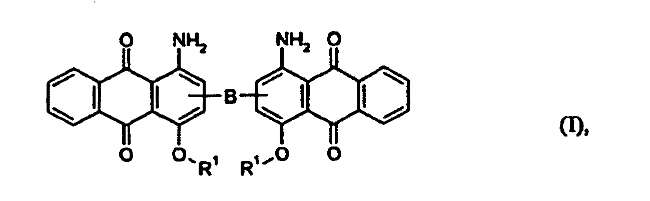 notamment de formule (Ia) 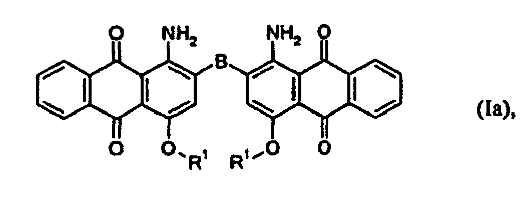 dans lesquelles
-B- représente -O-B¹-O- ou -CH₂-B²-CH₂-,
R¹ représente hydrogène, alkyle en C₁-C₈ éventuellement substitué ou cycloalkyle en C₃-C₈ éventuellement substitué,
B¹ représente un pont de formule 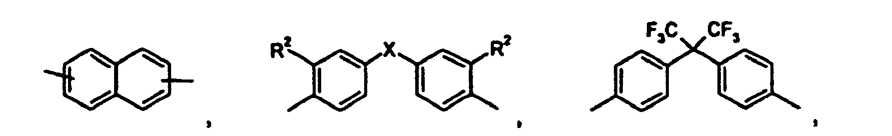 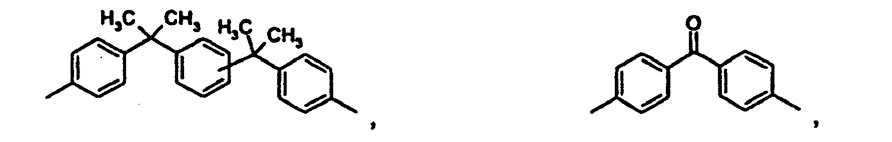 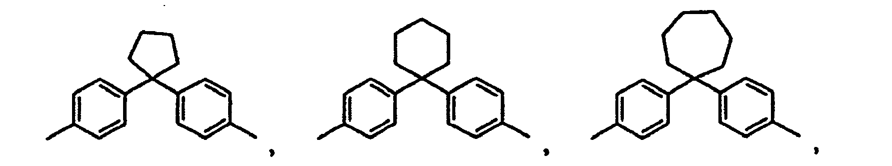 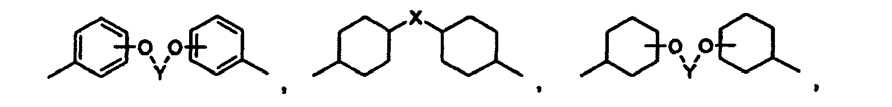 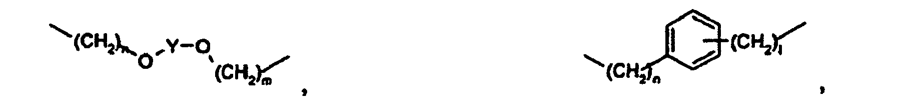 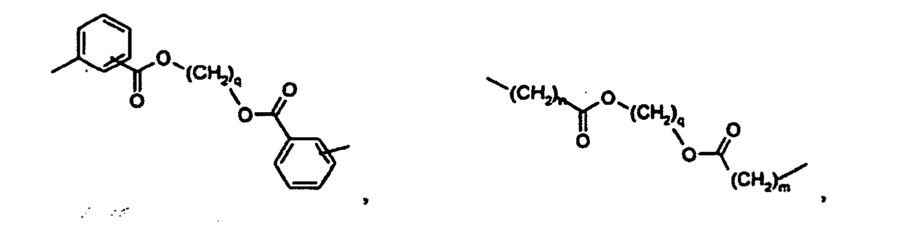 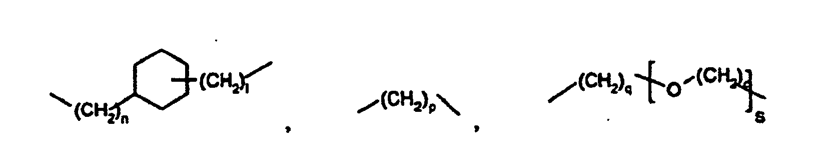 ou 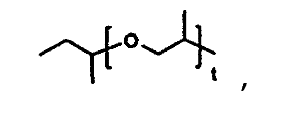 B² représente un pont de formule 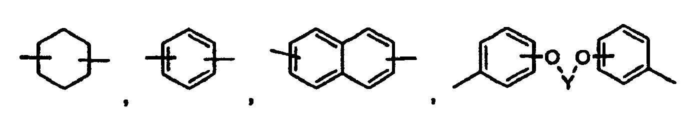 ou 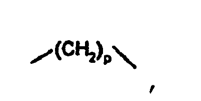 R² représente méthyle, éthyle, méthoxy ou fluor,
X représente un pont de formule 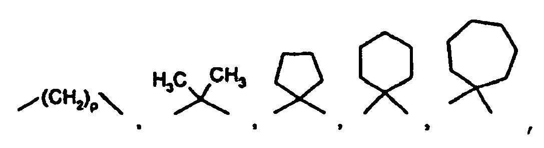 O, S, SO₂ ou CO,
Y représente un pont de formule 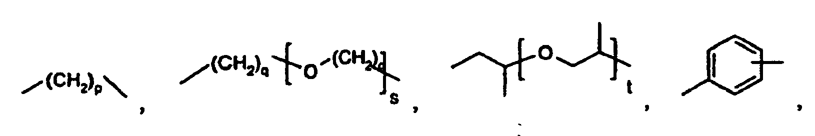 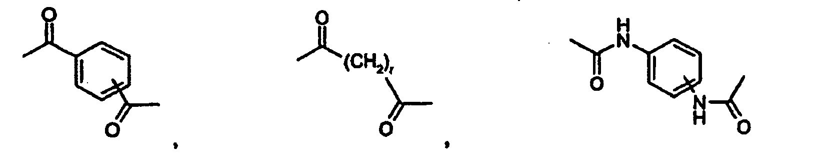 ou 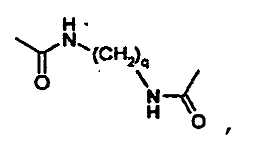 l représente un nombre entier de 0 à 8,
m, n, p et r représentent indépendamment les uns des autres un nombre entier de 1 à 8,
q représente un nombre entier de 2 à 8, et
s et t représentent indépendamment l'un de l'autre un nombre entier de 1 à 4,
et un support.

14. Utilisation de composés de formule I 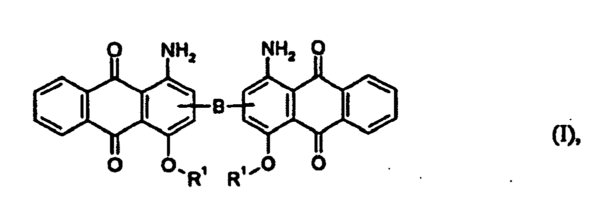 notamment de formule (Ia) 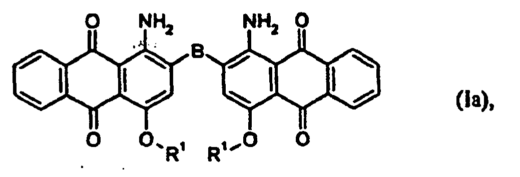 dans lesquelles
-B- représente -O-B¹-O- ou -CH₂-B²-CH₂-,
R¹ représente hydrogène, alkyle en C₁-C₈ éventuellement substitué ou cycloalkyle en C₃-C₈ éventuellement substitué,
B¹ représente un pont de formule 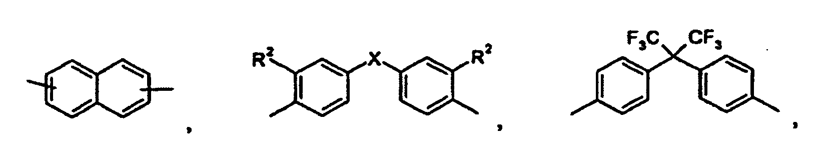 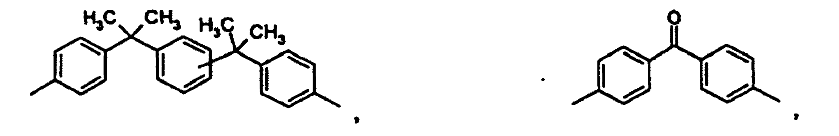 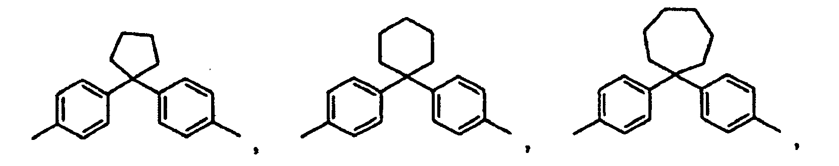 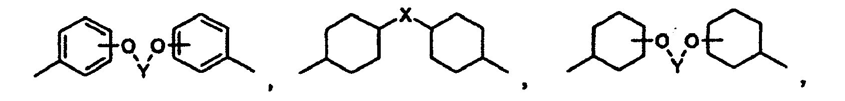 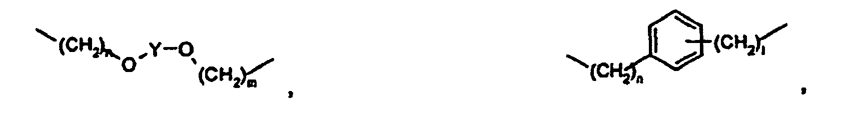 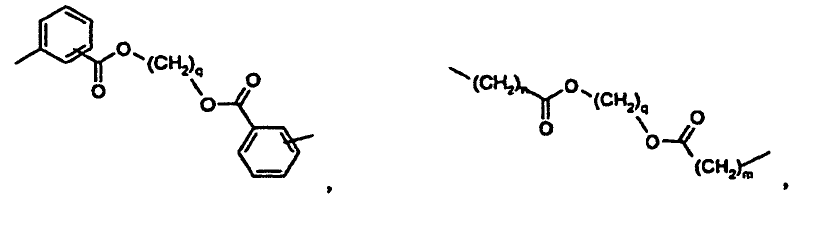 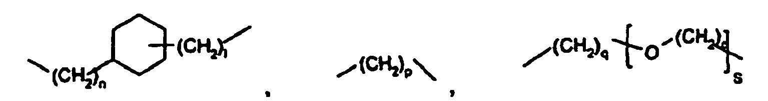 ou 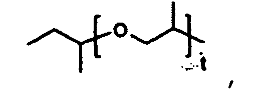 B² représente un pont de formule 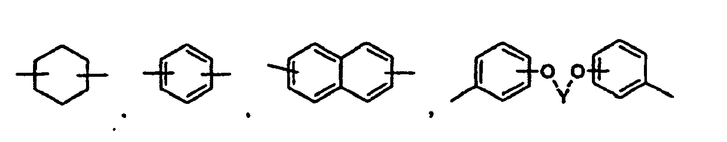 ou 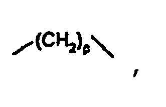 R² représente méthyle, éthyle, méthoxy ou fluor,
X représente un pont de formule 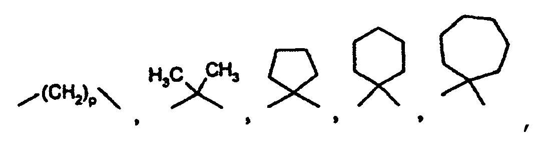 O, S, SO₂ ou CO,
Y représente un pont de formule 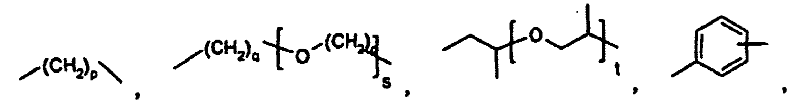 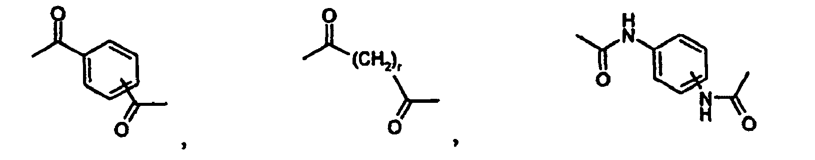 ou 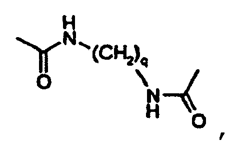 l représente un nombre entier de 0 à 8, m, n, p et r représentent indépendamment les uns des autres un nombre entier de 1 à 8,
q représente un nombre entier de 2 à 8, et
s et t représentent indépendamment l'un de l'autre un nombre entier de 1 à 4,
pour la coloration en masse de plastiques.

15. Procédé de coloration en masse de plastiques, **caractérisé en ce qu'**un composé de formule I 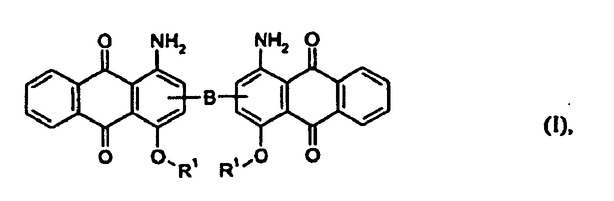 notamment de formule (Ia) 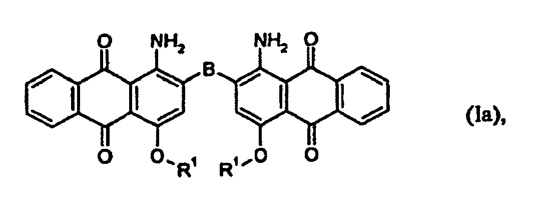 dans lesquelles
-B- représente -O-B¹-O- ou -CH₂-B²-CH₂-,
R¹ représente hydrogène, alkyle en C₁-C₈ éventuellement substitué ou cycloalkyle en C₃-C₈ éventuellement substitué,
B¹ représente un pont de formule 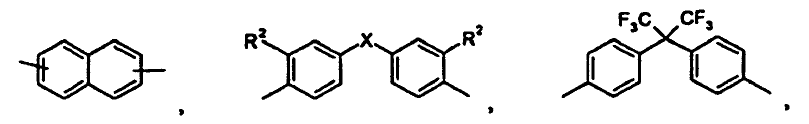 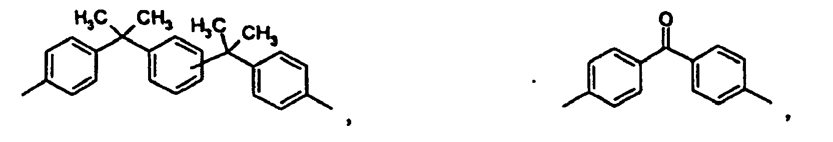 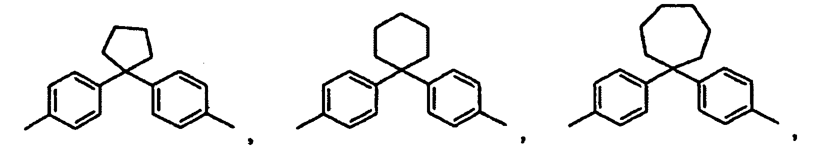 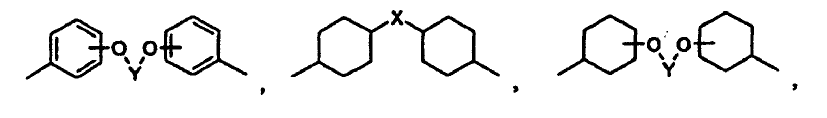 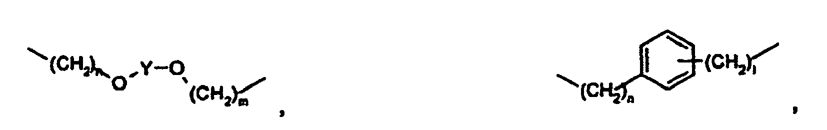 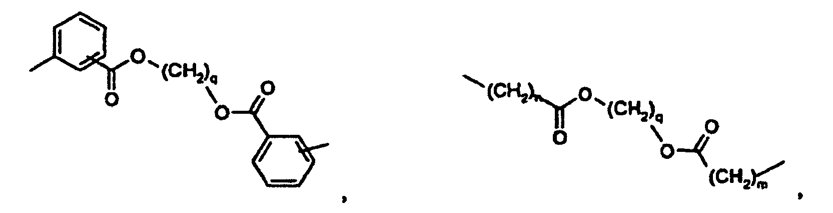 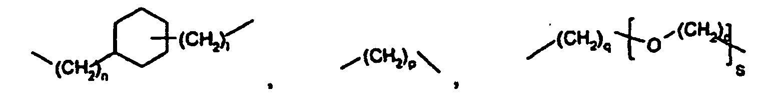 ou 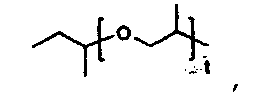 B² représente un pont de formule 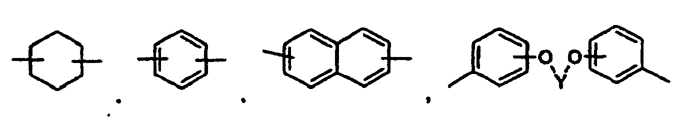 ou 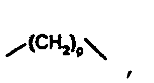 R² représente méthyle, éthyle, méthoxy ou fluor,
X représente un pont de formule 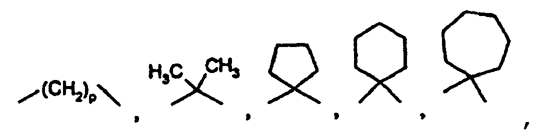 O, S, SO₂ ou CO,
Y représente un pont de formule 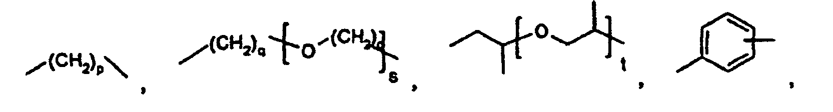 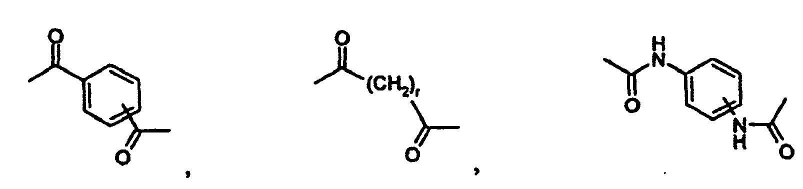 ou 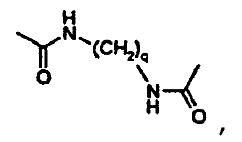 l représente un nombre entier de 0 à 8,
m, n, p et r représentent indépendamment les uns des autres un nombre entier de 1 à 8,
q représente un nombre entier de 2 à 8, et
s et t représentent indépendamment l'un de l'autre un nombre entier de 1 à 4,
de préférence sous la forme de son mélange maître, est incorporé dans la masse plastique fondue ou déjà ajouté aux composants de départ pour la fabrication du plastique avant la polymérisation.

16. Plastiques contenant un composé de formule I 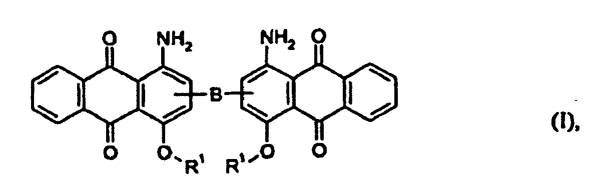 notamment de formule (Ia) 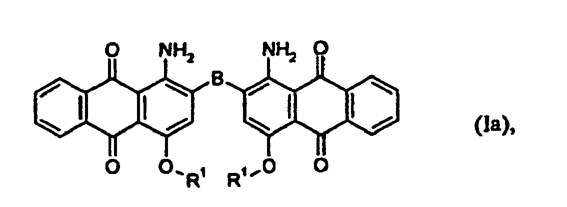 dans lesquelles
-B- représente -O-B¹-O- ou -CH₂-B²-CH₂-,
R¹ représente hydrogène, alkyle en C₁-C₈ éventuellement substitué ou cycloalkyle en C₃-C₈ éventuellement substitué,
B¹ représente un pont de formule 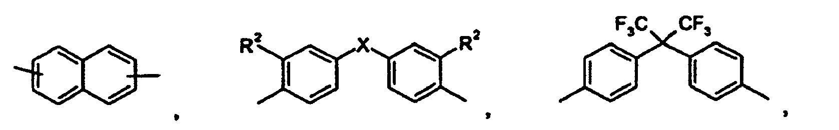 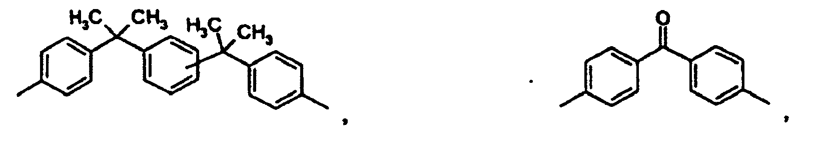 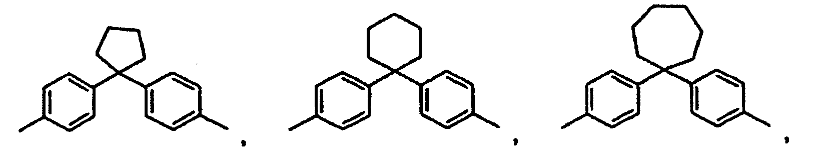 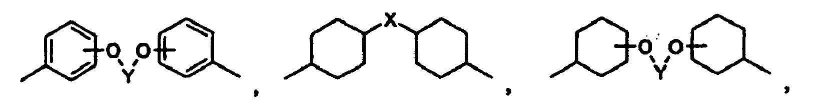 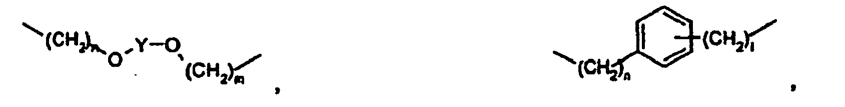 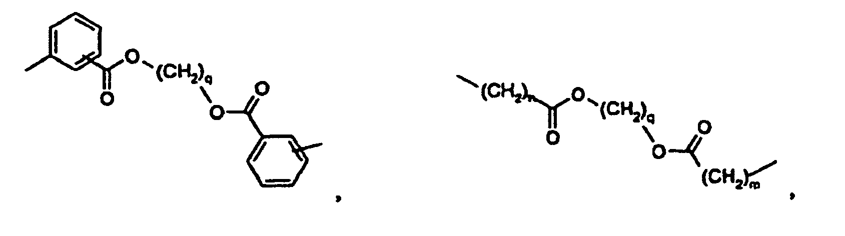 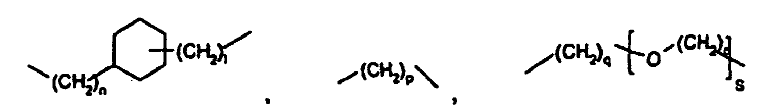 ou 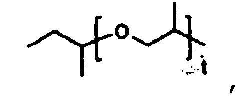 B² représente un pont de formule 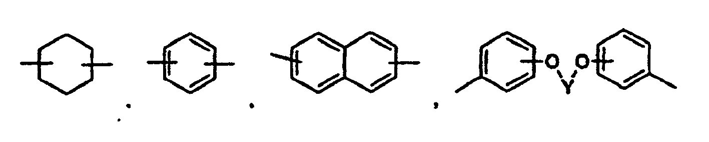 ou 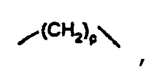 R² représente méthyle, éthyle, méthoxy ou fluor,
X représente un pont de formule 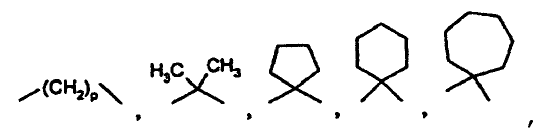 O, S, SO₂ ou CO,
Y représente un pont de formule 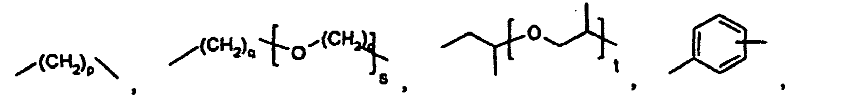 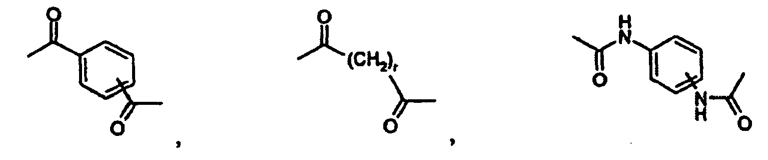 ou 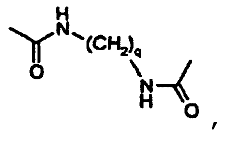 l représente un nombre entier de 0 à 8,
m, n, p et r représentent indépendamment les uns des autres un nombre entier de 1 à 8,
q représente un nombre entier de 2 à 8, et
s et t représentent indépendamment l'un de l'autre un nombre entier de 1 à 4.

17. Utilisation des mélanges selon la revendication 1 ou des composés de formule I 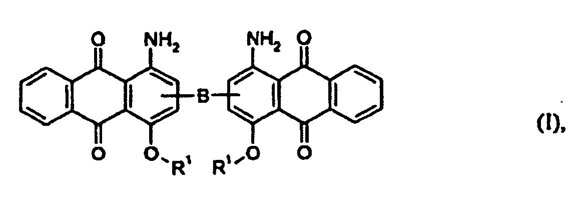 notamment de formule (Ia) 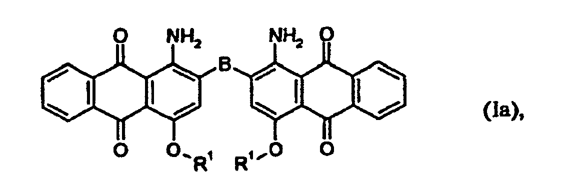 dans lesquelles
-B- représente -O-B¹-O- ou -CH₂-B²-CH₂-,
R¹ représente hydrogène, alkyle en C₁-C₈ éventuellement substitué ou cycloalkyle en C₃-C₈ éventuellement substitué,
B¹ représente un pont de formule 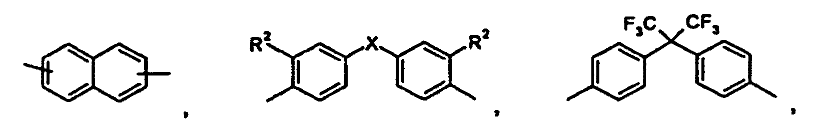 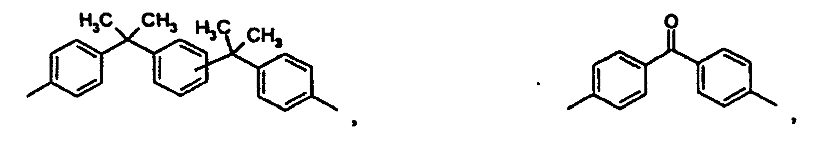 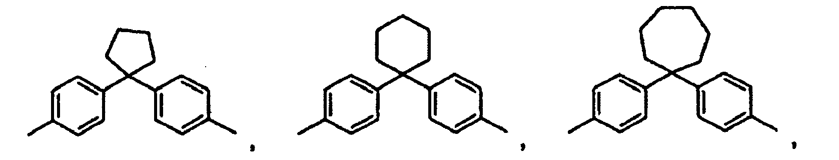 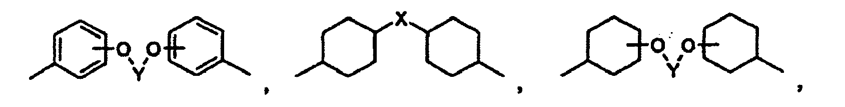 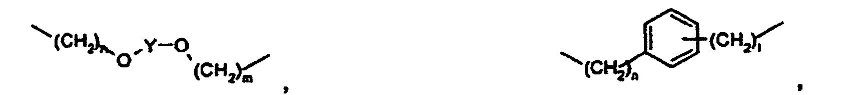 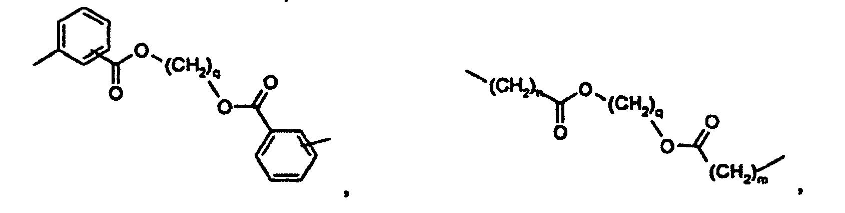 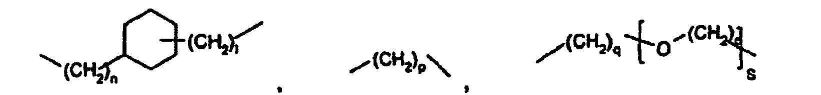 ou 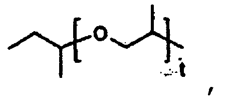 B² représente un pont de formule 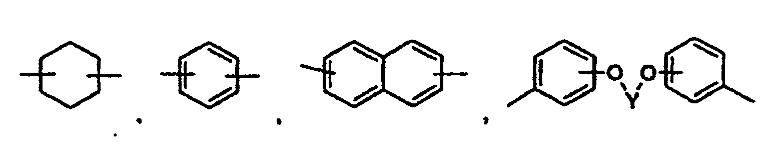 ou 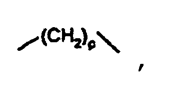 R² représente méthyle, éthyle, méthoxy ou fluor,
X représente un pont de formule 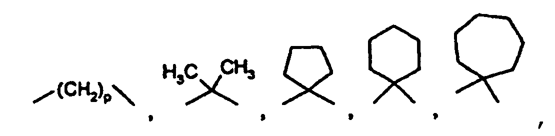 O, S, SO₂ ou CO,
Y représente un pont de formule 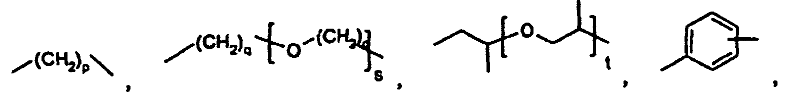 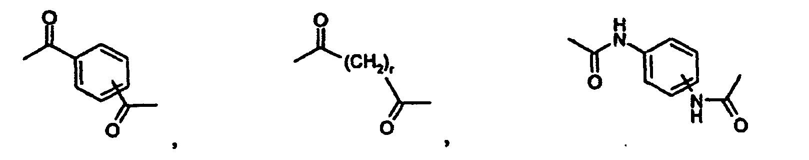 ou 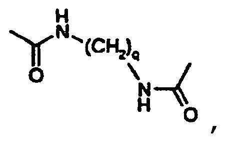 l représente un nombre entier de 0 à 8,
m, n, p et r représentent indépendamment les uns des autres un nombre entier de 1 à 8,
q représente un nombre entier de 2 à 8, et
s et t représentent indépendamment l'un de l'autre un nombre entier de 1 à 4,
pour la coloration de fibres de synthèse, de préférence sous forme dispersée.
